(19) Europäisches Patentamt
European Patent Office
Office européen des brevets

(11) Numéro de publication : **0 564 356 A1**

(12) **DEMANDE DE BREVET EUROPEEN**

(21) Numéro de dépôt : **93400833.5**

(22) Date de dépôt : **31.03.93**

(51) Int. Cl.[5] : **C07D 233/54,** C07D 233/64, C07D 233/68, C07D 233/84, C07D 401/12, C07D 401/14, C07D 403/06, C07D 403/10, C07D 405/12, C07D 405/14, A61K 31/415

(30) Priorité : **01.04.92 FR 9203944**

(43) Date de publication de la demande : **06.10.93 Bulletin 93/40**

(84) Etats contractants désignés : **AT BE CH DE DK ES FR GB GR IE IT LI LU MC NL PT SE**

(71) Demandeur : **FOURNIER INDUSTRIE ET SANTE**
**38, avenue Hoche**
**F-75008 Paris (FR)**

(72) Inventeur : **Dodey, Pierre**
**10, rue des Champs d'Aloux**
**F-21121 Fontaine-lès-Dijon (FR)**
Inventeur : **Bondoux, Michel**
**7, Allée des Monterey**
**F-21121 Fontaine-lès-Dijon (FR)**
Inventeur : **Renaut, Patrice**
**Route Changey**
**F-21121 Hauteville-lès-Dijon (FR)**
Inventeur : **Pruneau, Didier**
**Rue du Dessus**
**F-21370 Pasques (FR)**

(74) Mandataire : **Clisci, Serge et al**
**S.A. FEDIT-LORIOT & AUTRES, CONSEILS EN PROPRIETE INDUSTRIELLE, 38, Avenue Hoche**
**F-75008 Paris (FR)**

(54) **Dérivés de 4-phénylaminométhylimidazole, procédé de préparation, antagonistes des récepteurs angiotensin II et leur application en thérapeutique.**

(57) La présente invention concerne les phényl-amino-méthyl-imidazoles de formule :

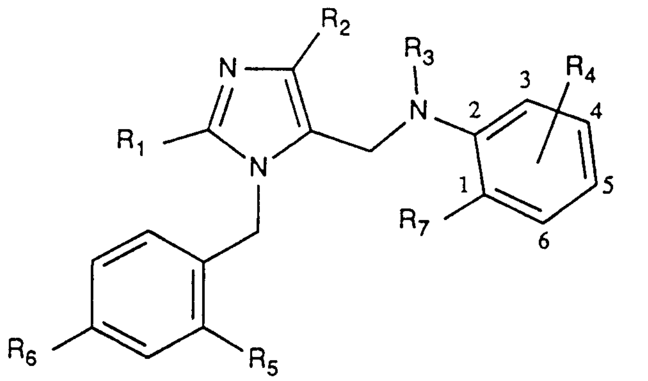

(I)

(où les groupes $R_1$ à $R_7$ sont définis comme indiqué dans la description).

Elle concerne également leur procédé de préparation et leur application en thérapeutique en tant qu'agents antagonistes de l'angiotensine II, utiles dans le traitement de l'hypertension, des désordres circulatoires et du glaucome.

EP 0 564 356 A1

La présente invention concerne des dérivés de l'imidazole, leur procédé de préparation et leur application en thérapeutique en tant qu'agents utiles dans le traitement de l'hypertension, des désordres circulatoires et du glaucome.

On connaît déjà un certain nombre de dérivés de l'imidazole inhibiteurs de l'angiotensine II utiles en tant qu'agents antihypertenseurs.

Les demandes de brevet EP-A-403 158 et EF-A-403 159 décrivent des acides imidazolyl-alkènoïques portant une chaîne insaturée en position 5 du cycle imidazole. La demande de brevet EP-A-465 368 décrit des dérivés soufrés de l'imidazole. La demande WO-A-91/00277 décrit des imidazoles substitués portant une fonction aldéhyde en position 5 du cycle imidazole. La demande EP-A-427 463 décrit des dérivés substitués N-(imidazolyl) alkylalanine portant un amino-acide en position 5 du cycle imidazole. La demande EP-A-324 377 décrit des dérivés de l'imidazole préconisés comme diurétiques, antiinflammatoires et antihypertenseurs. La demande EP-A-253 310 décrit des dérivés de l'imidazole portant un substituant hydroxyméthyl en position 5 du cycle imidazole.

Aucun de ces documents antérieurs ne décrit ni ne suggère des dérivés de l'imidazole portant un substituant phényl-amino-méthyle en position 5 du cycle imidazole.

La présente invention propose donc des dérivés de l'imidazole portant un substituant phényl-amino-méthyle en position 5 du cycle imidazole.

Les composés selon l'invention sont caractérisés en ce qu'ils sont choisis parmi l'ensemble constitué par :

(i) les phényl-amino-méthyl-imidazoles de formule:

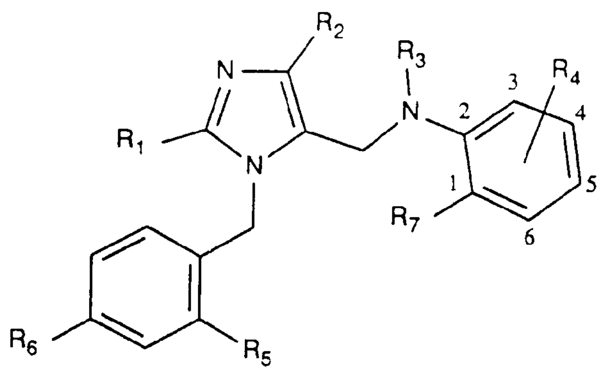

(I)

dans laquelle :

- $R_1$ représente un groupe alkyle en $C_1$-$C_4$,
- $R_2$ représente l'atome d'hydrogène, un halogène, un groupe alkylthio en $C_1$-$C_4$ ou un groupe perfluoroalkyle en $C_1$-$C_3$,
- $R_3$ représente l'atome d'hydrogène, un groupe alkyle en $C_1$-$C_4$ ou un groupe $COR_8$ où $R_8$ représente un groupe alkyle en $C_1$-$C_4$,
- $R_4$ est en position 3, 4, 5 ou 6 et représente l'atome d'hydrogène, un ou plusieurs groupes alkyles en $C_1$-$C_4$, alkoxy en $C_1$-$C_4$, azido, nitro ou un ou plusieurs halogènes, ou forme un groupe amino-2-naphtyle avec le groupe amino-phényle auquel il est lié,
- $R_8$ représente un atome d'hydrogène ou un halogène,
- $R_8$ et $R_7$, identiques ou différents, représentent chacun un groupe tétrazol-5-yle ou un groupe $COR_9$ où $R_9$ représente :
  - un groupe hydroxy,
  - un groupe alkoxy en $C_1$-$C_{16}$,
  - un groupe cyclopropylméthoxy,
  - un groupe phénoxy,
  - un groupe benzyloxy,
  - un groupe 2-phényléthoxy,
  - un groupe glycéryle,
  - un groupe isopropylidène glycéryle,
  - un groupe 2-méthoxyéthoxy,
  - un groupe 2-oxo-butyl oxy,
  - un groupe 1-méthyl-2-oxo-butyl oxy,
  - un groupe 2-(N, N-diéthylamino)-éthoxy,
  - un groupe morpholino-éthoxy,

- un groupe N-(éthyloxy)nicotinamide,
- un groupe O-$CHR_{15}$-O(CO)-$R_{12}$ dans lequel $R_{15}$ représente l'atome d'hydrogène ou un groupe alkyle en $C_1$-$C_3$, et $R_{12}$ représente un groupe alkyle en $C_1$-$C_7$ un groupe cyclopentyle, un groupe cyclohexyle, un groupe cyclopentylméthyle ou un groupe cyclohexylméthyle,
- un groupe oxyacétate de formule O-$CHR_{17}$-$CO_2$-$R_{16}$, dans lequel $R_{16}$ et $R_{17}$ représentent chacun indépendamment l'atome d'hydrogène ou un groupe alkyle en $C_1$-$C_5$,
- un groupe oxyacétamide de formule -O-$CH_2$-CO-$NR_{10}R_{11}$, dans lequel $R_{10}$ et $R_{11}$, identiques ou différents représentent chacun un groupe alkyle en $C_1$-$C_4$, un groupe hydroxyéthyle, ou forment avec l'atome d'azote auquel ils sont liés un groupe 4-méthylpipérazine-1-yle,
- un groupe amino de formule -$NR_{18}R_{19}$ dans lequel $R_{18}$ et $R_{19}$ représentent chacun, indépendamment, l'atome d'hydrogène, un groupe alkyle en $C_1$-$C_4$, un groupe méthoxy, un groupe 2-(N,N-diméthylamino) propyle ou, -$NR_{18}R_{19}$ représente un reste acide aminé de structure glycine ou valine, dont la fonction acide est éventuellement protégée sous forme d'ester ou d'amide,

- $R_6$ pouvant également représenter :

  - un groupe $COR_{13}$ où $R_{13}$ représente un groupe méthylsulfonylamino de formule -NH-$SO_2$-$CH_3$ ou un groupe arylsulfonylamino de formule :

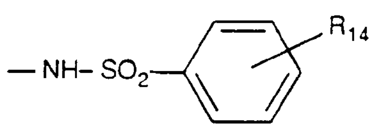

dans laquelle $R_{14}$ représente l'atome d'hydrogène, un halogène, un groupe azido, un groupe alkyle en $C_1$-$C_4$ ou un groupe méthoxy et peut se trouver en position ortho, méta ou para.

- $R_3$ et $R_7$, considérés ensemble, pouvant former, avec l'atome d'azote et le groupe phényle auxquels ils sont respectivement liés, un hétérocycle azoté ortho-condensé carboxy-2-indol-1-yle ou éthoxycarbonyl-2-indol-1-yle; et,

(ii) les sels d'addition avec les acides minéraux et organiques ou avec les bases minérales et organiques des composés de formule I.

Par groupe alkyle en $C_1$-$C_7$, on entend ici un groupe alkyle linéaire, ramifié ou cyclique comportant jusqu'à 7 atomes de carbone.

Les groupes alkyle préférés sont les groupes méthyle, éthyle, n-propyle, isopropyle, n-butyle, tertiobutyle et pentyle.

Par groupe alkoxy en $C_1$-$C_{16}$, on entend ici un groupe où le reste alkyle est linéaire, ramifié ou cyclique et comporte jusqu'à 16 atomes de carbone.

Les groupes alkoxy préférés sont les groupes méthoxy, éthoxy, pentoxy et cyclopropylméthoxy.

Par groupe alkylthio en $C_1$-$C_4$, on entend ici un groupe où le reste alkyle est linéaire ou ramifié et comporte jusqu'à 4 atomes de carbone.

Les groupes alkylthio préférés sont les groupes méthylthio et éthylthio.

Par halogène on entend ici l'atome de fluor, l'atome de chlore, l'atome de brome ou l'atome d'iode.

Parmi les groupes perfluoroalkyle en $C_1$-$C_3$ on préfèrera les groupes trifluorométhyle et perfluoroéthyle.

Parmi les sels d'addition avec les acides minéraux et organiques on préfèrera les sels d'addition formés avec les acides chlorhydrique, bromhydrique, sulfurique, nitrique, phosphorique, maléique, malique, acétique, glutamique, tartrique, lactique, citrique, aspartique, oléique, gluconique, ascorbique, valérique, succinique, éthylsuccinique, fumarique, oxalique, gallique, pivalique, caprique, décanoïque, heptanoïque, propanoïque, caproïque, stéarique, iséthionique, éthanedisulfonique, méthanesulfonique, naphtalène-sulfonique et métasulfobenzoïque.

Parmi les sels d'addition avec les bases minérales ou organiques on préfèrera les sels d'addition formés avec l'hydroxyde de sodium, l'hydroxyde de potassium, l'hydroxyde de magnésium, l'hydroxyde de calcium, l'hydroxyde de manganèse, l'hydroxyde de lithium, la lysine, la cystéine, l'arginine, la monoéthanolamine, la méglumine, la bétaïne, la diéthylamine et la dicyclohexylamine.

Les composés préférés selon l'invention sont les composés de formule I dans laquelle $R_6$ et $R_7$ représentent un groupe sulfonylaminocarbonyl ou un groupe COOH ainsi que leurs sels obtenus par réaction avec une base organique ou minérale.

On préférera encore les composés de formule I salifiés par réaction avec un acide organique ou minéral.

Les composés de formule I selon l'invention peuvent être préparés selon un procédé caractérisé en ce qu'il comprend les étapes consistant à

(a)soumettre à une substitution nucléophile un composé de formule :

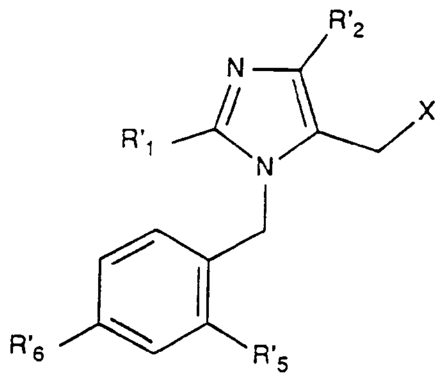

(II')

dans laquelle :

- $R'_1$ représente un groupe alkyle en $C_1$-$C_4$,
- $R'_2$ représente l'atome d'hydrogène, un halogène, un groupe alkylthio en $C_1$-$C_4$ ou un groupe perfluoroalkyle en $C_1$-$C_3$,
- $R'_5$ représente un atome d'hydrogène ou un halogène,
- $R'_6$ représente un groupe cyano, un groupe $COR'_9$ où $R'_9$ représente un groupe alkoxy en $C_1$-$C_{16}$, un groupe benzyloxy ou un groupe isopropylidène glycéryle,
- X représente un halogène, notamment l'atome de chlore, ou un groupe paratoluènesulfonyle,

  par réaction avec un composé de formule :

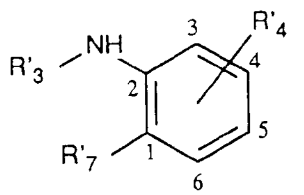

(III')

dans laquelle :

- $R'_3$ représente l'atome d'hydrogène ou un groupe alkyle en $C_1$-$C_4$,
- $R'_4$ est en position 3, 4, 5 ou 6 et représente l'atome d'hydrogène, un ou plusieurs groupes alkyles en $C_1$-$C_4$, alkoxy en $C_1$-$C_4$, azido, nitro ou un ou plusieurs halogènes ou forme un groupe amino-2 naphtyle avec le groupe amino-phényle auquel il est lié,
- $R'_7$ représente un groupe cyano, ou un groupe $COR'_9$ où $R'_9$ représente
  - un groupe alkoxy en $C_1$-$C_{16}$, un groupe benzyloxy, un groupe isopropylidène glycéryle, un groupe phénoxy, un groupe 2-phényléthoxy, un groupe 2-méthoxyéthoxy, un groupe 2-oxo-butyl oxy, un groupe 1-méthyl-2-oxo-butyl oxy, un groupe 2-(N, N-diéthylamino)-éthoxy,
  - un groupe $O$-$CHR_{15}$-$O(CO)$-$R_{12}$ dans lequel $R_{15}$ représente l'atome d'hydrogène ou un groupe alkyle en $C_1$-$C_3$ et $R_{12}$ représente un groupe alkyle en $C_1$-$C_7$, un groupe cyclopentyle, un groupe cyclohexyle, un groupe cyclopentylméthyle ou un groupe cyclohexylméthyle,
  - un groupe oxyacétate de formule $O$-$CHR_{17}$-$CO_2$-$R_{16}$, dans lequel $R_{16}$ et $R_{17}$ représentent chacun indépendamment l'atome d'hydrogène ou un groupe alkyle en $C_1$-$C_5$,
  - un groupe oxyacétamide de formule -$O$-$CH_2$-$CO$-$NR_{10}R_{11}$, dans lequel $R_{10}$ et $R_{11}$, identiques ou différents, représentent chacun un groupe alkyle en $C_1$-$C_4$ ou un groupe hydroxyéthyle,
  - un groupe amino de formule -$NR_{18}R_{19}$ dans lequel $R_{18}$ et $R_{19}$ représentent chacun indépendamment l'atome d'hydrogène, un groupe alkyle en $C_1$-$C_4$, un groupe méthoxy, un groupe 2-(N,N-diméthylamino) propyle ou, -$NR_{18}R_{19}$ représente un reste acide aminé de structure glycine ou valine, dont la fonction acide est éventuellement protégée sous forme d'ester où d'amide,
- $R'_3$ et $R'_7$, considérés ensemble, pouvant former, avec l'atome d'azote et le groupe phényle auxquels ils sont respectivement liés, un hétérocycle azoté ortho-condensé carboxy-2-indol-1-yle ou éthoxycarbonyle-2-indol-1-yle,

en milieu anhydre, en présence ou non d'un solvant polaire ou non polaire et aprotique, comme par exemple

le toluène, les xylènes, le tétrahydrofuranne, le diméthylformamide, les hydrocarbures chlorés, les éthers, le dioxanne, la N-méthylpyrrolidin-2-one, la N,N'-diméthylpropylèneurée ou le diméthylsulfoxyde et en présence ou non d'une base forte commme par exemple la triéthylamine, la 2,6-lutidine, les hydrures de sodium ou potassium, l'hexaméthyldisilylamidure de potassium ou de lithium ou le diisopropylamidure de lithium, à raison de 1 mole de composé II' pour 1 à 20 moles de composé III', à une température comprise entre la température ambiante (15-25 °C) et environ 200°C, pendant 0,1 à 12 heures, pour obtenir un composé de formule :

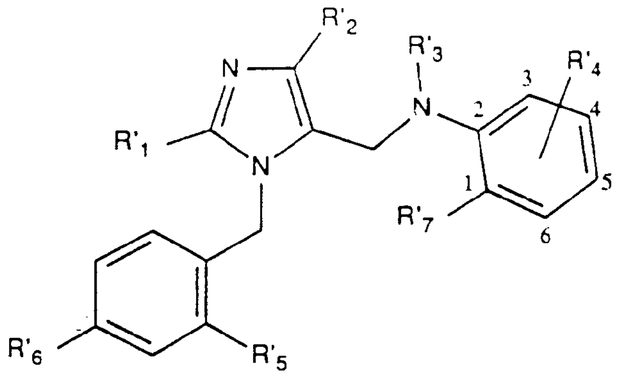

(I')

dans laquelle $R'_1$, $R'_2$, $R'_3$, $R'_4$, $R'_5$, $R'_6$ et $R'_7$ ont les significations indiquées ci-dessus;

(b) si nécessaire, soumettre le composé de formule I' ainsi obtenu aux traitements suivants :

(i) saponification d'un composé de formule I' dans laquelle l'un au moins des groupes $R'_6$ et $R'_7$ représente un groupe $COR'_9$ où $R'_9$ représente un groupe alcoxy en $C_1$-$C_{16}$, selon les méthodes connues de l'homme de l'art, notamment en présence d'une base forte comme par exemple une solution aqueuse d'hydroxyde de sodium ou de potassium, dans le diméthoxyéthane ou un alcool comme par exemple le méthanol, pour obtenir un composé de formule I dans laquelle $R_6$ et $R_7$ représentent un groupe COOH, ou $R_6$ représente un groupe COOH et $R_7$ représente un groupe $COR_9$ où $R_9$ représente un groupe alkoxy en $C_1$-$C_{16}$;

(ii)estérification du composé ainsi obtenu au stade (i) selon les méthodes connues de l'homme de l'art, notamment par réaction avec un alcool approprié ou par réaction avec un dérivé halogéné approprié, pour obtenir un composé de formule I dans laquelle $R_6$ et $R_7$ représentent un groupe $COR_9$ où $R_9$ a les significations des groupes $R'_9$ indiqués ci-dessus;

(iii) acylation du méthylsulfonamide ou d'un arysulfonamide de formule :

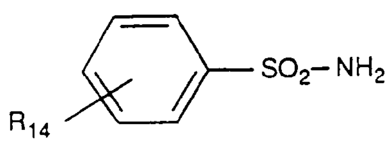

dans laquelle $R_{14}$ représente l'atome d'hydrogène, un halogène, un groupe azido, un groupe alkyle en $C_1$-$C_4$ ou un groupe méthoxy, par un mono-acide obtenu au stade (i) selon les méthodes connues de l'homme de l'art, notamment en présence d'un réactif de couplage comme par exemple le chlorhydrate de 1-(3-diméthylaminopropyl)-3-éthylcarbodiimide ou la N,N-dicyclohexylcarbodiimide, pour obtenir un composé de formule I dans laquelle $R_6$ représente un groupe $COR_{13}$ où $R_{13}$ représente un groupe méthylsulfonylamino de formule $-NH-SO_2-CH_3$, ou un groupe arylsulfonylamino de formule :

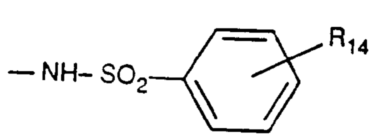

dans laquelle $R_{14}$ a la signification indiquée ci-dessus, $R_7$ représente un groupe $COR_9$ où $R_9$ représente un groupe alkoxy en $C_1$-$C_{16}$ et $R_1$, $R_2$, $R_3$, $R_4$ $R_6$ ont les significations indiquées ci-dessus respective-

ment $R'_1$, $R'_2$, $R'_3$, $R'_4$ et $R'_5$;

(iv) acylation d'un composé de formule I' dans laquelle $R'_3$ représente l'atome d'hydrogène et $R'_1$, $R'_2$, $R'_4$, $R'_5$ $R'_6$ et $R'_7$ ont les significations indiquées ci-dessus, selon les méthodes connues de l'homme de l'art, notamment par réaction avec un anhydrique d'acide, comme par exemple l'anhydrique acétique, pour obtenir un composé de formule I dans laquelle $R_3$ représente un groupe $COR_8$ où $R_8$ représente un groupe alkyle en $C_1$-$C_4$, et $R_1$, $R_2$, $R_4$, $R_5$, $R_6$ et $R_7$ ont les significations indiquées ci-dessus pour respectivement $R'_1$, $R'_2$, $R'_4$, $R'_5$, $R'_6$ et $R'_7$;

(v) si nécessaire, déprotection d'un composé de formule I' dans laquelle l'un au moins des groupes $R'_6$ et $R'_7$ représente un groupe $COR'_9$ où $R'_9$ représente un groupe alkoxy en $C_1$-$C_4$, un groupe benzyloxy ou un groupe isopropylidène glycéryde, selon les méthodes connues de l'homme de l'art, notamment par traitement en milieu acide ou par hydrogénation catalytique, pour obtenir un composé de formule I dans laquelle au moins l'un des groupes $R_8$ ou $R_7$ représente un groupe COOH ou CO-glycéryle et l'autre groupe représente un groupe $COR_9$ où $R_9$ a la signification indiquée ci-dessus pour $R'_9$;

(vi) conversion d'un composé de formule I' dans laquelle $R_8$ ou $R_7$ représente un groupe cyano,en composé de formule I dans laquelle $R_8$ ou $R_7$ représente un groupe tétrazol-5-yle selon les méthodes connues de l'homme de l'art, notamment par cycloaddition 1,3-dipolaire d'azotures de trialkylétain ou triarylétain.

Pour accéder aux composés de formule II' on préconise de réduire un aldéhyde de formule :

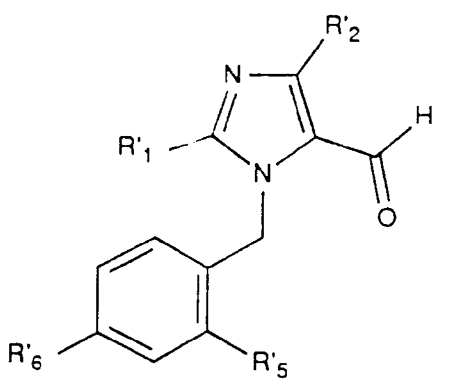

(IV)

dans laquelle $R'_1$, $R'_2$, $R'_5$ et $R'_6$ ont les significations indiquées ci-dessus, selon les méthodes connues de l'homme de l'art, notamment par action de $NaBH_4$ ou $KBH_4$ dans un alcool, pour obtenir un alcool de formule :

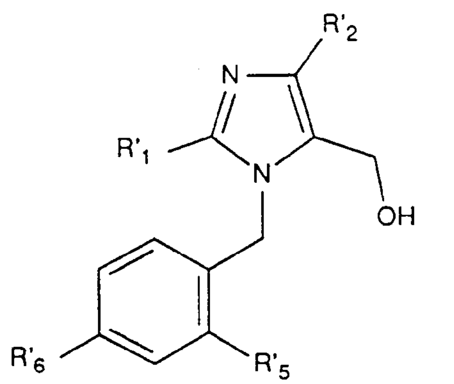

(V)

dans laquelle $R'_1$, $R'_2$, $R'_5$ et $R'_6$ ont les significations indiquées ci-dessus, puis convertir l'alcool ainsi obtenu en dérivé de formule II', notamment en dérivé chloré, selon les méthodes connues de l'homme de l'art, notamment par action du chlorure de thionyle dans un solvant inerte comme par exemple un solvant halogéné.

Les composés intermédiaires de formule IV dans laquelle :

(i) $R'_1$ représente un groupe n-propyle, $R'_2$ représente un atome d'hydrogène ou un halogène, $R'_5$ représente l'atome d'hydrogène et $R'_6$ représente un groupe cyano ou un groupe $COR'_9$ où $R'_9$ représente un groupe alkoxy en $C_1$-$C_{16}$ ou un groupe benzyloxy, ou

(ii) $R'_1$ représente un groupe n-butyle, $R'_2$ et $R'_5$ représentent l'atome d'hydrogène et $R'_6$ représente un

groupe COR'$_9$ dans lequel R'$_9$ représente un groupe t-butoxy ou benzyloxy sont des composés nouveaux et constituent un des objets de l'invention.

Les composés intermédiaires de formule V dans laquelle R'$_1$ représente un groupe alkyle en $C_1$-$C_4$, R'$_2$ représente l'atome d'hydrogène ou un halogène, R'$_5$ représente l'atome d'hydrogène et R'$_6$ représente un groupe COR'$_9$ où R'$_9$ représente un groupe alkoxy en $C_1$-$C_{16}$ ou un groupe benzyloxy sont des composés nouveaux et constituent un des objets de l'invention.

Les composés intermédiaires de formule II' dans laquelle R'$_1$ représente un groupe n-butyle, R'$_2$ et R'$_5$ représentent l'atome d'hydrogène et R'$_6$ représente un groupe COR'$_9$ dans lequel R'$_9$ représente un groupe t-butoxy ou benzyloxy sont des composés nouveaux et constituent un des objets de l'invention.

L'invention sera mieux comprise à la lecture des exemples de préparation qui suivent où les préparations se réfèrent aux produits intermédiaires et les exemples se réfèrent aux produits selon l'invention. Ces exemples sont destinés à illustrer l'invention sans en limiter la portée.

## PREPARATION 1

### 2-butyl-4-iodo-1H-imidazol-5-carboxaldéhyde

A une suspension à 15°C de 16 g ($57.10^{-3}$ mole) de 2-butyl-4-iodo-1H-imidazol-5-méthanol dans 48 ml d'acide acétique, on additionne goutte à goutte une solution de 70,5 g ($128.5.10^{-3}$mole) de nitrate de cérium ammoniacal dans 58 ml d'eau. On agite à température ambiante pendant 24 heures. Le mélange réactionnel est alors additionné de solution d'hydroxyde de sodium 10 N jusqu'à pH 6. Le précipité formé est extrait à l'acétate d'éthyle, lavé à l'eau puis séché sur sulfate de magnésium. On obtient ainsi 14,7 g (rendement : 93 %) de solide beige.

**F = 90°C**

## PREPARATION 2

### acide 4-[(4-chloro-5-formyl-2-propyl-1H-imidazol-1-yl)méthyl]-benzoïque, méthyl ester

A une solution de 18,15 g ($105.10^{-3}$ mole) de 4-chloro-2-propyl-1H-imidazol-5-carboxaldéhyde et de 28,9 g ($126.10^{-3}$ mole) de 4-bromométhyl-benzoate de méthyle dans 275 ml de diméthylformamide, on ajoute 17,4 g ($126.10^{-3}$ mole) de carbonate de potassium. Le mélange réactionnel est chauffé sous agitation à 40°C pendant 2 heures, puis après refroidissement, versé dans l'eau et extrait à l'acétate d'éthyle. Les phases organiques sont lavées à l'eau jusqu'à neutralité, séchées sur sulfate de magnésium et concentrées. Le solide brut obtenu est recristallisé dans l'éthanol pour donner 28 g (rendement : 83 %) de solide blanc.

**F = 89°C**

En opérant de façon analogue, on obtient les produits des préparations 9, 10, 11 et 31.

## PREPARATION 3

### acide 4-[(5-formyl-2-propyl-1H-imidazol-1-yl)méthyl]-benzoïque, méthyl ester

A une solution de 23,2 g ($72.10^{-3}$ mole) d'acide 4-[(4-chloro-5-formyl-2-propyl-1H-imidazol-1-yl)méthyl]-benzoïque, méthyl ester dans 230 ml de méthanol on ajoute 7,1 g ($72.10^{-3}$ mole) d'acétate de potassium puis, sous azote, 3,48 g de charbon palladié à 5%. La suspension obtenue est agitée sous atmosphère d'hydrogène à température ambiante pendant 3 jours. Le catalyseur est éliminé par filtration, le méthanol est évaporé, le résidu repris par l'acétate d'éthyle et la solution résultante est lavée à l'eau jusqu'à neutralité. Après séchage sur sulfate de magnésium la solution est concentrée et le produit brut huileux obtenu chromatographié sur silice en éluant par le mélange cyclohexane/acétone (7/3 ; v/v). L'évaporation de l'éluant conduit à 19 g (rendement à 91,6 %) de solide blanc.

**F = 72°C**

En opérant de façon analogue, on a obtenu les produits suivants :

### acide 4-[(2-butyl-5-formyl-1H-imidazol-1-yl)méthyl]-benzoïque, 1,1-diméthyléthyl ester (préparation 12)

RMN $^1$H (300 MHz; $CDCl_3$ ; ppm)

0,89 (t,3H) ; 1,31 (m,2H) ; 1,57 (s,9H) ; 1,69 (m,2H) ; 2,66 (t,2H) ; 5,82 (s,2H) ; 7,03 (d,2H); 7,81 (s,1H);

7,93(d,2H) ; 9,66 (s,1H).

**acide 4-[(2-butyl-5-formyl-1H-imidazol-1-yl)méthyl]-benzoïque, éthyl ester (préparation 32)**

RMN $^1H$ (300 MHz; $CDCl_3$ ; ppm)
0,89 (t, 3H) ; 1,23- 1,40 (m, 5H) ; 1,63 - 1,74 (m, 2H) ; 2,64 (t, 2H) ; 4,35 (q, 2H) ; 5,60 (s, 2H) ; 7,04 (d, 2H) ; 7,82 (s, 1H) ; 7,97 (d, 2H) ; 9,67 (s, 1H).

**PREPARATION 4**

**acide 4-[(2-butyl-5-formyl-4-trifluorométhyl-1H-imidazol-1-yl)méthyl]-benzoïque, méthyl ester**

A une suspension de 82 g de cadmium en poudre dans 183 ml de diméthylformamide à température ambiante on ajoute, en 2 heures, 32 ml de dibromodifluorométhane en solution dans 32 ml de diméthylformamide. Le mélange réactionnel est agité 2 heures à température ambiante puis on laisse au repos 30 minutes. A un mélange de 70 ml de cette solution dans 75 ml d'hexaméthylphosphorotriamide à 0°C on additionne 8,34 g ($58.10^{-3}$ mole) de bromure cuivreux, puis 7,08 g ($16,6.10^{-3}$ mole) d'acide 4-[(2-butyl-5-formyl-4-iodo-1H-imidazol- 1-yl)méthyl]-benzoïque, méthyl ester en solution dans 50 ml de diméthylformamide. Le mélange réactionnel est chauffé à 70°C pendant 4 heures. Après refroidissement du mélange on additionne 600 ml d'eau et on extrait à l'acétate d'éthyle. Les phases organiques sont lavées à l'eau, séchées sur sulfate de magnésium, filtrées et évaporées sous pression réduite. L'huile résultante est purifiée par chromatographie sur silice en éluant avec un mélange toluène/acétate d'éthyle (9/1 ; v/v). On obtient ainsi 5,71 g (rendement : 93 %) de solide ocre.

**F = 59°C**

**PREPARATION 5**

**acide 4-[(2-butyl-5-formyl-1H-imidazol-1-yl)méthyl]-benzoïque**

A une solution de 16 g ($53,3.10^{-3}$ mole) d'acide 4-[(2-butyl-5-formyl-1H-imidazol-1-yl)méthyl]-benzoïque, méthyl ester dans 100 ml de méthanol on additionne 25 ml d'eau et 3 g ($75.10^{-3}$ mole) d'hydroxyde de sodium. Le mélange réactionnel est chauffé à reflux pendant 5 heures. Le méthanol est évaporé sous pression réduite et le résidu est additionné de 150 ml d'eau. On lave par deux fois 50 ml d'acétate d'éthyle. La phase aqueuse est acidifiée par une solution d'acide chlorhydrique 1N jusqu'à pH=6 et extraite par 2 fois 100 ml d'un mélange acétate d'éthyle/n-butanol (80/20 ; v/v). Les phases organiques sont lavées à l'eau, séchées sur sulfate de magnésium, filtrées et évaporées sous pression réduite. On obtient ainsi 14 g (rendement: 94 %) de solide jaune.

**F = 148°C**

En opérant de façon analogue à la préparation 5, on obtient le produit de la préparation 33.

**PREPARATION 6**

**acide 4-[(2-butyl-5-formyl-1H-imidazol-1-yl)méthyl]-benzoïque, phénylméthyl ester**

A une solution de 13,5 g ($47,2.10^{-3}$ mole) d'acide 4-[(2-butyl-5-formyl-1H-imidazol-1-yl)méthyl]-benzoïque, dissous dans un mélange de 5 ml de diméthylformamide et 200 ml de dichlorométhane, on additionne 5,4 g ($50.10^{-3}$ mole) d'alcool benzylique, 5,85 g ($48.10^{-3}$ mole) de 4-diméthylaminopyridine et 9,17 g ($48.10^{-3}$ mole) de chlorhydrate de 1-(3-diméthyl-aminopropyl)-3-éthylcarbodiimide. Le mélange réactionnel est agité à température ambiante pendant 20 heures puis lavé par deux fois 60 ml d'eau. La phase organique est séchée sur sulfate de magnésium, filtrée et évaporée sous pression réduite. Le résidu est purifié par chromatographie en éluant avec un mélange hexane/acétone (70/30 ; v/v). On obtient 17 g (rendement : 95 %) d'huile jaune.

RMN $^1H$ (300MHz; $CDCl_3$ ; ppm)
0,88 (t,3H) ; 1,35 (m,2H) ; 1,72 (m,2H) ; 2,65 (t,2H) ; 5,34 (s,2H) ; 5,62 (s,2H) ; 7,04 (d,2H); 7,4 (m,5H); 7,80 (s,1H); 8,03(d,2H) ; 9,66 (s,1H).

En opérant de façon analogue, on obtient le produit de la préparation 34 et les produits suivants :

**acide 4-[(2-butyl-5-formyl-1H-imidazol-1-yl)méthyl]-benzoïque, pentyl ester (Préparation 35)**

RMN $^1H$ (300 MHz ; $CDCl_3$ ; ppm)
0,89 (m, 6H) ; 1,18 - 1,40 (m, 5H) ; 1,62 - 1,77 (m, 5H) ; 2,63 (t, 2H) ; 4,29 (t, 2H) ; 5,63 (s, 2H) ; 7,05 (d, 2H) ; 7,81 (s, 1H) ; 7,97 (d, 2H) ; 9,67 (s, 1H).

**acide 4-[(2-butyl-5-formyl-1H-imidazol-1-yl)méthyl]-benzoïque, butyl ester (Préparation 36)**

RMN $^1H$ (300 MHz; $CDCl_3$; ppm)
0,86 (t, 3H) ; 0,96 (t, 3H) ; 1,31 - 1,49 (m, 4H) ; 1,64 - 1,78 (m, 4H) ; 2,63 (t, 2H) ; 4,30 (t, 2H) ; 5,62 (s, 2H) ; 7,04 (d, 2H) ; 7,81 (s, 1H) ; 7,97 (d, 2H) ; 9,67 (s, 1H).

**acid 4-[(2-butyl-5-formyl-1H-imidazol-1-yl)méthyl]-benzoïque, 2-méthyl-propyl ester (Préparation 37)**

RMN $^1H$ (300 MHz; $CDCl_3$; ppm)
0,88 (t, 3H) ; 0,99 (d, 6H) ; 1,31 - 1,38 (m, 2H) ; 1,64 - 1,75 (m, 2H) ; 2,06 (m, 1H) ; 2,63 (t, 2H) ; 4,08 (d, 2H) ; 5,63 (s, 2H) ; 7,05 (d, 2H) ; 7,81 (s, 1H) ; 7,98 (d, 2H) ; 9,67 (s, 1H).

**acide 4-[(2-butyl-5-formyl-1H-imidazol-1-yl)méthyl]-benzoïque, cyclopropyl-méthyl ester (Préparation 38)**

RMN $^1H$ (300 MHz; $CDCl_3$ ; ppm)
0,35 (m, 2H) ; 0,59 (m, 2H) ; 0,88 (t, 3H) ; 1,21 - 1,25 (m, 1H) ; 1,29 - 1,40 (m, 2H) ; 1,60 - 1,75 (mm, 2H) ; 2,63 (t, 2H) ; 4,12 (d, 2H) ; 5,63 (s, 2H) ; 7,05 (d, 2H) ; 7,81 (s, 1H) ; 7,97 (d, 2H) ; 9,67 (s, 1H).

**acide 4-[(2-butyl-5-formyl-1H-imidazol-1-yl)méthyl]-benzoïque, 3-méthyl-butyl ester (Préparation 39)**

RMN $^1H$ (300 MHz; $CDCl_3$ ; ppm)
0,88 (t, 3H) ; 0,97 (d, 6H) ; 1,23 - 1,41 (m, 3H) ; 1,61 - 1,82 (m, 4H) ; 2,63 (t, 2H) ; 4,35 (t, 2H) ; 5,63 (s, 2H) ; 7,05 (d, 2H) ; 7,81 (s, 1H) ; 7,97 (d, 2H) ; 9,67 (s, 1H).

**acide 4-[(2-butyl-4-chloro-5-formyl-1H-imidazol-1-yl)méthyl]-benzoïque, phénylméthyl ester (Préparation 40)**

RMN $^1H$ (300 MHz; $CDCl_3$; ppm)
0,87 (t, 3H) ; 1,29 - 1,37 (m, 2H) ; 1,61 - 1,72 (m, 2H) ; 2,60 (t, 2H) ; 5,35 (s, 2H) ; 5,59 (s, 2H) ; 7,07 (d, 2H) ; 7,34 - 7,44 (m, 5H) ; 8,02 (d, 2H).

**PREPARATION 62**

**acide 4-[[2-butyl-5-formyl-4-méthylthio-1H-imidazol-1-yl]méthyl]-benzoïque,méthyl ester**

A une solution de 3,87 g (1,15 $10^{-2}$ mole) de l'ester méthylique de l'acide 4-[(4-chloro-5-formyl-2-butyl-1H-imidazol-1-yl)méthyl]-benzoïque dans 40 ml de méthanol sont ajoutés 3,24 g (4,62 $10^{-2}$ mole) de thiométhylate de sodium. Le mélange réactionnel est chauffé à reflux sous agitation pendant 4 heures puis après refroidissement versé sur une solution aqueuse à 0°C d'acide citrique à 10 % et extrait à l'acétate d'éthyle. Les phases organiques sont lavées à l'eau jusqu'à neutralité, sèchées sur sulfate de magnésium et concentrées. Le résidu huileux obtenu est chromatographié sur silice en éluant par le mélange toluène/acétate d'éthyle (8/2 v/v). L'évaporation de l'éluant conduit à 2,8 g (rendement : 70 % ) de solide jaune.
**F = 72 - 74°C**

**PREPARATION 7**

**acide 4-[(2-butyl-5-hydroxyméthyl-1H-imidazol-1-yl)méthyl]-benzoïque, méthyl ester**

5,92 g (19,7.$10^{-3}$ mole) de l'ester méthylique de l'acide 4-[(2-butyl-5-formyl-1H-imidazol-1-yl)méthyl]-benzoïque, sont dissous dans 60 ml de méthanol et la solution obtenue est refroidie par un bain de glace. 895 mg (23,6.$10^{-3}$ mole) de borohydrure de sodium sont alors ajoutés par fractions pendant 30 minutes tout en agitant.

10 minutes après, le méthanol est évaporé, le résidu est étendu d'eau et extrait par le chlorure de méthylène. Les phases organiques rassemblées sont lavées à l'eau, séchées sur sulfate de magnésium et concentrées. On obtient 5,22 g (rendement : 88 %) d'un solide blanc.

**F = 144°C**

En opérant de façon analogue, on a préparé les produits des préparations 13 à 21 et 41 à 49 ainsi que le produit suivant :

**4-[(2-butyl-5-hydroxyméthyl-1H-imidazol-1-yl)méthyl]-benzonitrile (Préparation 50)**

**F = 109°C**

**PREPARATION 8**

**Chlorhydrate de l'acide 4-[(2-butyl-5-chlorométhyl-1H-imidazol-1-yl)méthyl]-benzoïque, méthyl ester**

On dissout 5,2 g ($17,2.10^{-3}$ mole) de l'ester méthylique de l'acide 4-[(2-butyl-5-hydroxyméthyl-1H-imidazol-1-yl)méthyl]-benzoïque dans 50 ml de chloroforme et on refroidit par un bain de glace. On ajoute alors goutte à goutte et en agitant 10,23 g ($86.10^{-3}$ mole) de chlorure de thionyle. On maintient l'agitation à cette température pendant 15 minutes après la fin de l'ajout. On évapore le chloroforme puis on ajoute du toluène et on l'évapore. On obtient 6,1 g (rendement : 99,3 %) de solide beige.

**F = 158°C**

En opérant de façon analogue, on a obtenu les produits des préparations 22 à 29, 51 et 53 à 59 ainsi que les produits suivants :

**acide 4-[(2-butyl-5-chlorométhyl-4-trifluorométhyl-1H-imidazol-1-yl)méthyl]-benzoïque, méthyl ester (Préparation 30)**

**huile jaune**

RMN $^1H$ (300 MHz; $CDCl_3$; ppm)

0,87 (t,3H) ; 1,33 (m,2H) ; 1,68 (m,2H) ; 2,60 (t,2H) ; 3,92 (s,3H) ; 4,52 (s,2H) ; 5,29 (s,2H) ; 7,06 (d,2H) ; 8,03 (d,2H).

**Chlorhydrate de 4-[(2-butyl-5-chlorométhyl-1H-imidazol-1-yl)méthyl]-benzonitrile (Préparation 60)**

**F = 95°C**

**acide 4-[(2-butyl-4-chloro-5-chlorométhyl-1H-imidazol-1-yl)méthyl]-benzoïque, phénylméthyl ester (Préparation 61)**

RMN $^1H$ (300 MHz; $CDCl_3$ ; ppm)

0,87 (t, 3H) ; 1,27 - 1,39 (m, 2H) ; 1,62 - 1,72 (m, 2H) ; 2,55 (t, 2H) ; 4,44 (s, 2H) ; 5,24 (s, 2H) ; 5,36 (s, 2H) ; 7,05 (d, 2H) ; 7,16 - 7,45 (m, 5H) ; 8,05 (d, 2H).

**PREPARATION 63**

**acide 2-méthyl-6-nitrobenzoïque, pentyl ester**

A une suspension de 5 g (0,0276 mole) d'acide 2-nitro-6-méthyl benzoïque dans 90 ml de toluène on additionne 6 ml (0,082 mole) de chlorure de thionyle. Le mélange réactionnel est chauffé à reflux sous agitation pendant 3,5h et évaporé sous pression réduite. Le résidu huileux est ensuite additionné de 45 ml de n-pentanol, chauffé à reflux sous agitation pendant 2 heures. Après refroidissement on ajoute 100 ml d'eau et une solution aqueuse saturée de bicarbonate de sodium jusqu'à pH basique et on extrait au toluène. Les phases organiques sont lavées à l'eau, sèchées sur sulfate de magnésium et concentrées. Après distillation sous pression réduite du n-pentanol on obtient 6,74 g d'une huile jaune (rendement : 97 %).

RMN $^1H$ (300 MHz; $CDCl_3$ ; ppm)

0,89 (t, 3H) ; 1,36 (m, 4H) ; 1,77 (m, 2H) ; 2,45 (s, 3H) ; 4,37 (t, 2H) ; 7,43 (t, 1H) ; 7,53(d, 1H) ; 8,00 (d, 1H).

**PREPARATION 64**

**2-[(2-aminobenzoyl)oxy]-N,N-dipropyl-acétamide**

A une solution de 15 g (0,1 mole) d'acide anthranilique dans 150 ml de diméthylformamide on ajoute 15 g (0,084 mole) de N,N dipropylchloroacétamide, 1,26 g ($8,44.10^{-3}$ mole) d'iodure de sodium et 11,1 g (0,1 mole) de triéthylamine. Le mélange est agité pendant une nuit à température ambiante. On ajoute une solution saturée de bicarbonate de sodium et on extrait à l'acétate d'ethyle. Les phases organiques sont lavées à l'eau jusqu'à pH neutre, séchées sur sulfate de magnésium et concentrées. L'huile obtenue est cristallisée en agitant dans l'éther pour donner 7,86 g (rendement : 33,4 %) du produit attendu.

**F = 64°C**

En opérant de façon analogue à la préparation 64, on obtient les produits suivants :

**2-[(2-aminobenzoyl)oxy]-3-pentanone (Préparation 65)**

RMN $^1H$ (300 MHz; $CDCl_3$ ; ppm)
1,09 (t, 3H) ; 1,49 (d, 3H) ; 2,58 (m, 2H) ; 5,28 (q, 1H) ; 5,70 (s, 2H) ; 6,60 (m, 2H) ; 7,28 (m, 1H); 7,91 (m, 1H).

**acide 2-[(2-aminobenzoyl)oxy]-acétique, éthyl ester (Préparation 66)**

RMN $^1H$ (300 MHz; $CDCl_3$; ppm)
1,30 (t, 3H) ; 4,25 (q, 2H) ; 4,80 (s, 2H) ; 5,69 (s, 2H) ; 6,66 (m, 2H) ; 7,29 (m, 1H) ; 7,93(m, 1H).

**acide 2-[(2-aminobenzoyl)oxy]-acétique, pentyl ester (Préparation 67)**

RMN $^1H$ (300 MHz; $CDCl_3$ ; ppm)
0,88 (m, 3H); 1,28 (m, 4H); 1,63 (m, 2H) ; 4,16 (t, 2H); 4,80 (s, 2H); 5,68 (s, 2H) ; 6,65 (m, 2H) ; 7,28 (m, 1H) ; 7,92 (m, 1H) .

**PREPARATION 68**

**acide 2-aminobenzoïque, cyclopropyl méthyl ester**

A une suspension de 9 g (0,0552 mole) d'anhydride isatoïque dans 14,3 g (0,198 mole) de cyclopropylméthanol, on additionne 1,77 g (0,044 mole) d'hydroxyde de sodium. Le mélange réactionnel est chauffé à 80°C sous agitation pendant 3 heures puis versé sur de l'eau et extrait à l'acétate d'éthyle. Les phases organiques lavées à l'eau jusqu'à pH neutre, séchées sur sulfate de magnésium sont concentrées sous pression réduite. On obtient ainsi 7,51g (rendement : 64 %) d'huile ocre.

RMN $^1H$ (300 MHz; $CDCl_3$; ppm)
0,34 (m, 2H) ; 0,61 (m, 2H) ; 1,25 (m, 1H) ; 4,10 (d, 2H) ; 5,70 (s, 2H) ; 6,65 (m, 2H); 7,25 (m, 1H); 7,92 (m, 1H).

En opérant de façon analogue à la préparation 68, on obtient le produit de la préparation 69 et les produits suivants :

**acide 2-aminobenzoïque, 1-méthylpentyl ester (Préparation 70)**

RMN $^1H$ (300 MHz; $CDCl_3$ ; ppm)
0,86 (m, 3H) ; 1,31 - 1,73 (m, 2H) ; 5,10 (m, 1H); 5,71 (s, 2H) ; 6,61 (m, 2H) ; 7,25 (m, 1H) ; 7,88 (m, 1H).

**acide 2-aminobenzoïque, 2-[N,N-diéthyl amino] éthyl ester (Préparation 71)**

RMN $^1H$ (300 MHz; $CDCl_3$ ; ppm)
1,06 (t, 6H) ; 2,63 (q, 4H) ; 2,83 (t, 2H) ; 4,36 (t, 2H) ; 5,70 (s, 2H) ; 6,63 (m, 2H) ; 7,25 (s, 1H) ; 7,86 (d, 1H).

**PREPARATION 72**

**2-[(2-aminobenzoyl)oxy]-N,N-diéthyl-propanamide**

A une solution de 8,31 g (6,06 $10^{-2}$ mole) d'acide anthranilique dans 45 ml de 1,3-diméthyl-3,4,5,6-tétrahydro-2-(1H)-pyrimidinone (DMPU) on ajoute 2 g (6,66 $10^{-2}$ mole) de NaH en suspension à 80 % dans l'huile. On agite à température ambiante pendant 0,5h puis on ajoute goutte à goutte 10,91 g (6,66 $10^{-2}$ mole) de N,N-diéthyl-2-chloropropanamide en solution dans 10 ml de DMPU. Le mélange réactionnel est alors agité à 100°C pendant 1,5h. Après refroidissement on ajoute une solution saturée de bicarbonate de sodium et on filtre le précipité obtenu. Après lavage à l'eau et séchage on obtient 14,31 g (rendement 89 %) du produit attendu.

**F = 134°C**

En opérant de façon analogue à la préparation 72, on obtient les produits des préparations 73, 74 et 75 ainsi que les produits suivants :

**acide 2-éthylbutanoïque, 1-[(2-aminobenzoyl)oxy] éthyl ester (Préparation 76)**

RMN $^1H$ (300 MHz; $CDCl_3$ ; ppm)
0,89 (t, 6H) ; 1,47 - 1,66 (m, 7H) ; 2,22 (m, 1H) ; 5,73 (s, 2H) ; 6,63 (m, 2H) ; 7,13 (q, 1H); 7,26 (m, 1H) ; 7,84 (m, 1H).

**acide cyclopentylcarboxylique, 1-[(2-aminobenzoyl) oxy] éthyl ester (Préparation 77)**

RMN $^1H$ (300 MHz; $CDCl_3$ ; ppm)
1,53 - 1,87 (m, 11H) ; 2,75 (m, 1H) ; 5,73 (s, 2H) ; 6,61 (t, 2H) ; 7,10 (q, 1H) ; 7,27 (m, 1H) ; 7,83 (m, 1H).

**acide cyclohexylcarboxylique, 1-[(2-aminobenzoyl)oxy] éthyl ester (Préparation 78)**

RMN $^1H$ (300 MHz; $CDCl_3$; ppm)
1,18 - 1,88 (m, 13H) ; 2,31 (m, 1H) ; 5,74 (s, 2H) ; 6,63 (m, 2H) ; 7,09 (m, 1H) ; 7,28 (m, 1H) ; 7,83 (m, 1H)

**acide hexanoïque, [(2-aminobenzoyl)oxy] méthyl ester (Préparation 79)**

RMN $^1H$ (300 MHz; $CDCl_3$ ; ppm)
0,87(t, 3H) ; 1,29 (m, 4H) ; 1,63 (m, 2H) 2,37 (t, 2H) ; 5,7 (s, 2H) ; 5,96 (s, 2H) 6,63 (m, 2H) 7,29 (m, 1H) ; 7,88 (d, 1H).

**acide hexanoïque, 1-[(2-aminobenzoyl)oxy] éthyl ester (Préparation 80)**

RMN $^1H$ (300 MHz; $CDCl_3$ ; ppm)
0,26 (t, 3H) ; 1,30 (m, 4H) ; 1,60 (m, 5H) ; 2,35 (t, 2H) ; 5,76 (s, 2H) ; 6,63 (m, 2H) ; 7,11 (m, 1H); 7,27 (m, 1H); 7,86 (d, 1H).

**acide cyclohexylacétique, 1-[(2-aminobenzoyl)oxy] éthyl ester (Préparation 81)**

RMN $^1H$ (300 MHz; $CDCl_3$ ; ppm)
0,91 - 1,42 (m, 5H) ; 1,55 - 1,75 (m, 9H) ; 2,20 (m, 2H) ; 5,74 (s, 2H) ; 6,63 (m, 2H); 7,12 (m, 1H); 7,27 (m, 1H); 7,85 (m, 1H)

**acide cyclopentylacétique, 1-[(2-aminobenzoyl)oxy] éthyl ester (Préparation 82)**

RMN $^1H$ (300 MHz ; $CDCl_3$ ; ppm)
1,15 (m, 2H) ; 1,58 (m, 7H) ; 1,81 (m, 2H) ; 2,23 (m, 1H) ; 2,35 (m, 2H) ; 5,73 (s, 2H) ; 6,61 (d, 1H) ; 7,09 - 7,29 (m, 2H) ; 7,85 (d, 1H).

**acide 2-2-diméthylpropanoïque, [(2-aminobenzoyl) oxy] méthyl ester (Préparation 83)**

RMN $^1H$ (300 MHz; $CDCl_3$; ppm)
1,49 (s, 9H) ; 4,69 (s, 2H) ; 5,66 (s, 2H) ; 6,65 (m, 2H) ; 7,27 (m, 1H) ; 7,93 (m, 1H).

**PREPARATION 84**

**N-[3-(N,N-diméthylamino)propyl]-2-aminobenzamide**

On ajoute lentement 8,16 g (8 $10^{-2}$ mole) de N,N-diméthyl-propanediamine à 6,52 g (4 $10^{-2}$ mole) d'anhydride isatoïque puis on chauffe à 80°C pendant 1 heure. Après avoir refroidi on ajoute 150 ml d'eau et on extrait par l'acétate d'éthyle. Les phases organiques sont lavées à l'eau jusqu'à pH neutre, séchées sur sulfate de sodium, filtrées et évaporées sous pression réduite. On obtient 7,8 g (rendement : 88 %) du produit attendu.

**F = 76°C**

**PREPARATION 85**

**N,N-diéthyl-2-[N-(2-aminobenzoyl)amino]-acétamide**

A une solution de 3,27 g (2 $10^{-2}$ mole) d'anhydride isatoïque dans 20 ml de diméthylformamide, on ajoute successivement 3,33 g (2 $10^{-2}$ mole) de l'acide aminoacétique diéthylamide et 3,03 g (3 $10^{-2}$ mole) de triéthylamine. Le mélange réactionnel est alors chauffé à 70°C pendant 1 heure, puis on refroidit, on ajoute de l'eau et on extrait à l'acétate d'éthyle. Les phases organiques sont lavées à l'eau jusqu'à neutralité, séchées sur sulfate de magnésium, filtrées et concentrées sous pression réduite. On obtient 3,34 g (rendement : 67 %) du produit attendu.

**F = 62°C.**

En opérant de façon analogue à la préparation 85, on obtient le produit suivant :

**N-[2-aminobenzoyl]-L-valine, éthyl ester (Préparation 86)**

RMN $^1H$ (300 MHz; $CDCl_3$; ppm)

1,0 (t, 6H) ; 1,31 (t, 3H) ; 2,26 (m, 1H) ; 4,23 (m, 2H) ; 4,72 (m, 1H) ; 5,5 (s, 2H) ; 6,57 (d, 1H) ; 6,69 (m, 2H) ; 7,23 (t, 1H) ; 7,41 (d, 1H) ;

**PREPARATION 87**

**acide 2-amino-6-méthylbenzoïque, pentyl ester**

A une solution de 6,2 g (0,0247 mole) de 2-nitro-6-méthyl benzoate de pentyl dans 200 ml d'éthanol on additionne sous atmosphère d'azote 0,62 g de charbon palladié à 10 %. Le milieu réactionnel est placé ensuite sous atmosphère d'hydrogène et agité pendant 6 heures. Après filtration, l'éthanol est évaporé sous pression réduite. On obtient 5,22 g (rendement : 96 %) d'huile ocre.

RMN $^1H$ (300 MHz; $CDCl_3$ ; ppm)

0,92 (t, 3H) ; 1,38 (m, 4H) ; 1,73 (m, 2H) ; 2,44 (s, 3H) ; 4,32 (t, 2H) ; 5,2 (s, 2H) ; 5,54 (d, 2H) 7,07 (t, 1H).

**PREPARATON 88**

**acide 2-amino-6-chlorobenzoïque, pentyl ester**

A une suspension de 15 g (0,087 mole) d'acide 2-amino-6-chlorobenzoïque dans 250 ml de dichlorométhane on additionne 10,6 g. (0,087 mole) de 4 diméthylaminopyridine, 16,6 g (0,087 mmole) de chlorhydrate de 1-(3-diméthylaminopropyl)-3-éthyl carbodiimide et 7,65 g (0,087 mole) de n-pentanol. Le mélange réactionnel est agité à température ambiante pendant 20 heures puis lavé par 1 x 50 ml d'une solution d'acide citrique à 10 % puis par 2 x 50 ml d'eau. La phase organique est sèchée sur sulfate de magnésium, filtrée et évaporée sous pression réduite. Le résidu est purifié par flash chromatographie sur silice en éluant par un mélange cyclohexane/acétone (90/10 ; v/v). On obtient 4,64 g (rendement : 22 %) d'une huile jaune.

RMN $^1H$ (300 MHz; $CDCl_3$ ; ppm)

0,92 (t, 3H) ; 1,38 (M, 4H) ; 1,72 (q, 2H) ; 4,33 (t, 2H) ; 4,84 (s, 2H) ; 6,55 (d, 1H) ; 6,73 (d, 1H); 7,07 (t, 1H).

On a regroupé un certain nombre de produits intermédiaires dans les tableaux A, B, C et D où les symboles utilisés sont identiques à ceux des tableaux I à VII.

**exemple 1 :**

**acide 2-[[[2-butyl-1-[(4-(méthoxycarbonyl)phényl)méthyl]-1H-imidazol -5-yl]méthyl]amino]-benzoïque, méthyl ester**

8 g (22,3.$10^{-3}$ mole) du chlorhydrate de l'acide 4-[(2-butyl-5-chlorométhyl-1H-imidazol-1-yl)méthyl]-benzoïque, méthyl ester sont mis en suspension dans 80 ml de toluène anhydre. On ajoute 10,15 g (67,1.$10^{-3}$ mole) de l'ester méthylique de l'acide 2-aminobenzoïque puis 4,79 g (44,7.$10^{-3}$ mole) de 2,6-diméthylpyridine. On chauffe à reflux le mélange réactionnel pendant 8 heures puis on le verse sur de l'eau glacée. On extrait la phase aqueuse par l'acétate d'éthyle. Les phases organiques rassemblées sont lavées à l'eau jusqu'à neutralité, séchées sur sulfate de magnésium et concentrées. On obtient 11,8 g d'huile brune qui est purifiée par chromatographie en éluant par le mélange toluène/isopropanol (9/1 ; v/v). Après évaporation des éluats on obtient 9 g (rendement : 92,3 %) d'une huile orange.

RMN $^1H$ (300 MHz; $CDCl_3$ ; ppm)

0,87(t,3H) ; 1,34(m,2H) ; 1,68(m,2H) ; 2,56(t,2H) ; 3,77(s,3H) ; 3,91(s,3H) ; 4,19(d,2H) ; 5,18(s,2H) ; 6,58-6,67(m,2H) ; 6,92(d,2H) ; 7,04(s,1H) ; 7,30(t,1H) ; 7,67(t,1H) ; 7,82(d,1H); 7,91 (d,2H).

En opérant de façon analogue, on a préparé les produits des exemples 2, 76, 88, 89, 98, 219, 220, 222, 225 et les produits suivants:

**exemple 3 :**

**acide 2-[[[2-propyl-1-[(4-(méthoxycarbonyl)phényl)méthyl]-1H-imidazol-5-yl]méthyl]amino]-benzoïque, méthyl ester**

huile marron

RMN $^1H$ (300 MHz, $CDCl_3$, ppm)

0,94(t,3H) ; 1,73(m,2H) ; 2,53(t,2H) ; 3,77(s,3H) ; 3,91 (s,3H) ; 4,18(d,2H) ; 5,18(s,2H) ; 6,58-6,67(m,2H); 6,92(d,2H) ; 7,05(s,1H) ; 7,28-7,33(m,1H) ; 7,68 (t,1H) 7,82(d,1H) ; 7,90(d,2H).

**exemple 4 :**

**acide 2-[[[2-butyl-1-[(4-(phénylméthoxycarbonyl)phényl)méthyl]-1H-imidazol-5-yl]méthyl]amino]-benzoïque, 2,2-diméthyl-1,3-dioxolane-4-yl-méthyl ester**

huile

RMN $^1H$ (300 MHz; $CDCl_3$ ; ppm)

0,88(t,3H) ; 1,33(m,2H) ; 1,37(s,3H) ; 1,43(s,3H) ; 1,72(m,2H) ; 2,55(t,3H) ; 3,82(m,1H) ; 4,10(m,1H) ; 4,25(m,4H) ; 4,35(m,1H) ; 5,18(s,2H) ; 5,35(s,2H) ; 6,62(m,2H) ; 6,93(d,2H) ; 7,04(s,1H) ; 7,26-7,46(m,6H); 7,64(t,1H) ; 7,85 (d,1H) ; 7,94 (d,2H).

**exemple 5 :**

**acide 2-[[[2-butyl-1-[(4-(1,1-diméthyléthoxycarbonyl)phényl)méthyl]-1H-imidazol-5-yl]méthyl]amino]-benzoïque, phénylméthyl ester**

huile orangée

RMN $^1H$ (300 MHz; $CDCl_3$ ; ppm)

0,88(t,3H) ; 1,32(m,2H) ; 1,56(s,9H) ; 1,73(m,2H) ; 2,56(m,2H) ; 4,18(d,2H) ; 5,18(s,2H) ; 5,23(s,2H) ; 6,60(m,2H) ; 6,89(d,2H) ; 7,03(s,1H) ; 7,37(m,6H) ; 7,70(t,1H) ; 7,90(m,3H).

**exemple 6 :**

**acide 2-[[[2-butyl-4-chloro-1-[(4-(méthoxycarbonyl)phényl)méthyl]-1H-imidazol-5-yl]méthyl]amino]-3,5-dichloro-benzoïque, méthyl ester**

huile jaune

RMN $^1H$ (300 MHz; $CDCl_3$ ; ppm)

0,86(t,3H) ; 1,28(m,2H) ; 1,66(m,2H) ; 2,55(t,2H) ; 3,85(s,3H) ; 3,91(s,3H) ; 4,12(d,2H) ; 5,28(s,2H) ; 6,71

(t,1H) ; 6,98(d,2H) ; 7,40(d,1H) ; 7,78(d, 1H) ; 7,97(d,2H).

**exemple 7 :**

**acide 2-[[[2-butyl-4-chloro-1-[(4-(méthoxycarbonyl)phényl)méthyl]-1H-imidazol-5-yl]méthyl]amino]-3-méthyl-benzoïque, méthyl ester**

huile jaune pâle
RMN $^1H$ (300 MHz ; diméthylsulfoxyde ; ppm)
0,79(t,3H) ; 1,24(m,2H) ; 1,48(m,2H) ; 2,25(s,3H) ; 2,50(t,2H) ; 3,73(s,3H) ; 3,83(s,3H) ; 4,03(d,2H) ; 5,30(s,2H) ; 6,40(t,1H) ; 6,85(t,1H) ; 7,06(d,2H) ; 7,29(d,1H) ; 7,59(d,1H) ; 7,90(d,2H).

**exemple 8 :**

**acide N-[[2-butyl-4-chloro-1-[(4-(méthoxycarbonyl)phényl)méthyl]-1H-imidazol-5-yl]méthyl]-N-méthyl-2-amino-benzoïque, méthyl ester**

huile jaune
RMN $^1H$ (300 MHz; $CDCl_3$ ; ppm)
0,87(t,3H) ; 1,34(m,2H) ; 1,68(m,2H) ; 2,57(t,2H) ; 2,61(s,3H) ; 3,85(s,3H) ; 3,89(s,3H) ; 3,93(s,2H) ; 5,32(s,2H) ; 6,88-6,99(m,4H) ; 7,35(t,1H) ; 7,68(d,1H); 7,88(d,2H).

**exemple 74 :**

**acide 2-[[[2-butyl-4-chloro-1-[(4-(phénylméthoxycarbonyl)phényl)méthyl]-1H-imidazol-5-yl]méthyl]amino]-benzoïque, pentyl ester**

RMN $^1H$ (300 MHz; $CDCl_3$ ; ppm)
0,88 (m, 6H) ; 1,28 - 1,38 (m, 6H) ; 1,60 - 1,71 (m, 4H) ; 2,51 (t, 2H) ; 4,12 (t, 2H) ; 4,18 (d, 2H) ; 5,19 (s, 2H) ; 5,35 (s, 2H) ; 6,60 (t, 1H) ; 6,67 (d, 1H) ; 6,90 (d, 2H) ; 7,32 - 7,45 (m, 5H) ; 7,75 (t, 1H) ; 7,79 (d, 1H) ; 7,90 (d, 2H).

**exemple 75 :**

**acide 2-[[[2-butyl-1-[(4(méthoxycarbonyl)phényl)méthyl]-4-méthylthio-1H-imidazol-5-yl]méthyl]amino]-benzoïque, méthyl ester**

RMN $^1H$ (300 MHz; $CDCl_3$; ppm)
0,85 (t, 3H) ; 1,31 (m, 2H) ; 1,68 (m, 2H) ; 2,46 (s, 3H) ; 2,54 (t, 2H) ; 3,75 (s, 3H) ; 3,91 (s, 3H) ; 4,30 (d, 2H) ; 5,19 (s, 2H) ; 6,60 (t, 1H) ; 6,80 (d, 1H) ; 6,89 (d, 2H) ; 7,32 (m, 1H) ; 7,67 (t, 1H) ; 7,82 (m, 3H).

**exemple 77 :**

**acide 2-[[[2-butyl-1-[4((méthoxycarbonyl)phényl)méthyl]-1H-imidazol-5-yl] méthyl] amino]-benzoïque, ((dipropylamino)carbonyl)méthyl ester**

RMN $^1H$ (300 MHz; $CDCl_3$ ; ppm)
0,92 (m, 9H); 1,33 (m, 2H); 1,52 - 1,74 (m, 6H); 2,55 (t, 2H); 3,17 (t, 2H) ; 3,30 (t, 2H) ; 3,90 (s, 3H) ; 4,17 (d, 2H) ; 4,83 (s, 2H) ; 5,19 (s, 2H) ; 6,63 (m, 2H) ; 6,92 (d, 2H) ; 7 (s, 1H) ; 7,33 (t, 1H) ; 7,61 (t, 1H) ; 7,90 (d, 2H) ; 7,96 (d, 1H).

**exemple 78 :**

**acide 2-[[[2-butyl-1-[4((benzyloxycarbonyl)phényl)méthyl]-1H-imidazol-5-yl]méthyl]amino]-benzoïque, ((N,N-dipropylamino)carbonyl)méthyl ester**

RMN $^1H$ (300 MHz; $CDCl_3$; ppm)
0,84 - 0,98 (m, 9H) ; 1,35 (m, 2H) ; 15,3 - 1,71 (m, 6H) ; 2,55 (t, 2H) ; 3,15 (t, 2H) ; 3,29 (t, 2H) ; 4,16 (d, 2H) ;

4,79 (s, 2H) ; 5,19 (s, 2H) ; 5,35 (s, 2H) ; 6,60 (m, 2H) ; 6,91 (d, 2H) ; 7 (s, 1H) ; 7,16 - 7,45 (m, 6H) ; 7,59 (t, 1H) ; 7,93 - 7, 97 (m, 3H)

**exemple 79 :**

**acide 2-[[[2-butyl-1-[4((benzyloxycarbonyl)phényl)méthyl]-1H-imidazol-5-yl]méthyl]amino]-benzoïque,((N,N-diéthylamino)carbonyl)méthyl ester**

RMN $^1$H (300 MHz; $CDCl_3$; ppm)
0,87 (t, 3H) ; 1,14 (t, 3H) ; 1,23 (t, 3H) ; 1,35 (m, 2H) ; 1,68 (m, 2H) ; 2,54 (t, 2H) ; 3,25 (q, 2H) ; 3,38 (q, 2H) ; 4,16 (d, 2H) ; 4,78 (d, 2H) ; 5,19 (s, 2H) ; 5,35 (s, 2H) ; 6,61 (m, 2H) ; 6,90 (d, 2H) ; 7 (s, 1H) ; 7,16 - 7,45 (m, 6H) ; 7,58 (t, 1H) ; 7,95 (m, 3H).

**exemple 80 :**

**acide 2-[[[2-butyl-1-[4-((benzyloxycarbonyl)phényl)méthyl]-1H-imidazol-5-yl]méthyl]amino]-benzoïque, 1-((N,N-diéthylamino)carbonyl)éthyl ester**

RMN $^1$H (300 MHz; $CDCl_3$; ppm)
0,87 (t, 3H) ; 1,12 (t, 3H) ; 1,24 (t, 3H) ; 1,33(m, 2H) ; 1,47 (d,3H) ; 1,68 (m, 2H) ; 2,55 (t, 2H) ; 3,23 ) 3,52 (m, 4H) ; 4,14 (d, 2H) ; 5,17 (s, 2H) ; 5,35 (s, 2H) 5,40 (q, 1H) ; 6,60 (m, 2H) ; 6,93 (d, 2H) ; 7 (s, 1H) ; 7,16 - 7,45 (m, 6H) ; 7,57 (t, 1H) ; 7,94 (m, 3H).

**exemple 81 :**

**acide 2-[[[2-butyl-1-[4-((benzyloxycarbonyl)phényl)méthyl]-1H-imidazol-5-yl]méthyl]amino]-benzoïque,((N,N-di-(2-hydroxyéthyl)amino)carbonyl) méthyl ester**

RMN $^1$H (300 MHz; $CDCl_3$ ; ppm)
0,86 (t, 3H) ; 1,34 (m, 2H) ; 1,67 (m, 4H) ; 2,54 (t, 2H) ; 3,43 (t, 2H) ; 3,54 (t, 2H) ; 3, 81 (t, 2H) ; 3,86 (t, 2H) ; 4,15 (d, 2H) ; 4,89 (s, 2H) ; 5,16 (s, 2H) ; 5,35 (s, 2H) ; 6,60 (m, 2H) ; 6,90 (d, 2H) ; 7 (s, 1H) ; 7,26 - 7,45 (m, 7H) ; 7,93 (m, 3H).

**exemple 82 :**

**acide 2-[[[2-butyl-1-[4-((benzyloxycarbonyl)phényl)méthyl]-1H-imidazol-5-yl]méthyl]amino]-benzoïque,((N-méthyl,N-(2-hydroxyéthyl)amino)carbonyl) méthyl ester**

RMN $^1$H (300 MHz; $CDCl_3$ ; ppm)
0,77 (t, 3H) ; 1,22 (m, 2H) ; 1,47 (m, 2H) ; 2,49 (m, 2H) ; 2,81 (s, 1,5H) ; 2,95 (s, 1,5 H) ; 3,45 (m, 2H) ; 3,54 (m, 2H) ; 4,32 (d, 2H) 4,74 (s, 1H) ; 4,87 (s, 1H) ; 5,32 (s, 2H) ; 5,34 (s, 2H) ; 6,55 (m, 2H) ; 6,81 (d, 2H) ; 6,88 (s, 1H) ; 6,95 (m, 2H) ; 7,40 (m, 4H) ; 7,58 (t, 1H) ; 7,72 (d, 1H) ; 7,85 (m, 2H).

**exemple 83**

**acide 6-chloro-2-[[[2-butyl-1-[(4-(méthoxycarbonyl)phényl)méthyl]-1H-imidazol-5-yl]méthyl]amino]-benzoïque,pentyl ester**

RMN $^1$H (300 MHz; $CDCl_3$; ppm)
0,87 (m, 6H) ; 1,36 (m, 6H) ; 1,65 (m, 4H) ; 2,58 (t, 2H) ; 3,91 (s, 3H) ; 4,09 (d, 2H) ; 4,25 (t, 2H) ; 5,16 (s, 2H) ; 5,93 (t, 1H) ; 6,51 (d, 1H) ; 6,71 (d, 1H) ; 6,95 (d, 2H) ; 7,02 (s, 1H) ; 7, 11 (t, 1H) ; 7,96 (d, 2H).

**exemple 84 :**

**acide [[2-[[[2-butyl-1-[(4-(phénylméthoxycarbonyl)phényl)méthyl]-1H-imidazol-5-yl]méthyl]amino]phényl]carbonyloxy]-acétique,éthyl ester**

RMN $^1$H (300 MHz; $CDCl_3$; ppm)

0,85 (t, 3H) ; 1,29 (m, 5H) ; 1,69 (m, 2H) ; 2,17 (s, 2H) ; 2,58 (t, 2H) ; 4,22 (m, 4H) ; 4,71 (s, 2H) ; 5,17 (s, 2H) ; 5,35 (s, 2H) ; 6,61 (m, 2H) ; 6,90 (d, 2H) ; 7,03 (s, 1H) ; 7,38 (m, 4H) ; 7,46 (t, 1H) ; 7,93 (m, 3H).

**exemple 85 :**

**acide 2-éthyl-butanoïque, 1-[2-[[[2-butyl-1-[(4-(phénylméthoxycarbonyl) phényl)méthyl]-1H-imidazol-5-yl]méthyl]amino]phénylcarbonyloxy]éthyl ester**

RMN $^1H$ (300 MHz; $CDCl_3$; ppm)
0,86 (m, 9 H) ; 1,33 (m, 2H); 1,46 - 1,74 (m, 9H) ; 2,18 (m, 1H) ; 2,23 (t, 2H) ; 4,15 (d, 2H) ; 5,17 (s, 2H) ; 5,35 (s, 2H ) ; 6,65 (m, 2H) ; 7,02 (d, 2H) ; 7,05 (t, 2H) ; 7,26 - 7,44 (m, 6H) ; 7,69 (t, 1H) ; 7,80 (m, 1H) ; 7,96 (d, 2H).

**exemple 86 :**

**acide cyclopentylcarboxylique,1-[2-[[[2-butyl-1-[(4-(phénylméthoxy carbonyl)phényl)méthyl]-1H-imidazol-5-yl]méthyl]amino]phényl carbonyloxy]éthyl ester**

RMN $^1H$ (300 MHz; $CDCl_3$ ; ppm)
0,85 (t, 3H) ; 1,36 (m, 2H) ; 1,2 - 1,8 (m, 13 H) ; 2,5 (t, 2H) ; 2,67 (q, 1H) ; 4,17 (d, 2H) ; 5,17 (s, 2H) ; 5,35 (s, 2H) ; 6,61 (m, 2H) ; 6,90 (d, 2H) ; 7,02 (m, 2H); 7,16 - 7,42 (m, 6H); 7,7 (t, 1H); 7,82 (m, 1H); 7,93 (d, 2H).

**exemple 87 :**

**acide 2-[[[2-butyl-1-[(4-(méthoxycarbonyl)phényl)méthyl]-1H-imidazol-5-yl]méthyl]amino]-6-méthyl-benzoïque,pentyl ester**

RMN $^1H$ (300 MHz; $CDCl_3$; ppm)
0,87 (m, 6H) ; 1,38 (m, 6H) ; 1,64 (m, 4H) ; 2,40 (s, 3H) ; 2,54 (t, 2H) ; 3,90 (s, 3H) ; 4,12 (d, 2H) ; 4,19 (t, 2H) ; 5,18 (s, 2H) ; 6,50 (d, 2H) ; 6,68 (t, 1H) ; 6,92 (d, 2H); 7,03 (s, 1H); 7,14 (t, 1H); 7,92 (d, 2H).

**exemple 90 :**

**acide cyclopentylacétique, 1-[2-[[[2-butyl-1-[(4-(phénylméthoxycarbonyl) phényl)méthyl]-1H-imidazol-5-yl]méthyl]amino]phénylcarbonyloxy]éthyl ester**

RMN $^1H$ (300 MHz; $CDCl_3$; ppm)
0,86 (t, 3H) ; 1,15 (m, 2H) ; 1,35 (mm, 2H) ; 1,45 - 1,90 (m, 12H) ; 2,1 - 2,35 (m, 3H) ; 2,56 (t, 2H) ; 4,17 (d, 2H) ; 5,18 (s, 2H); 5,35 (s, 2H) ; 6,58 (m, 2H) ; 6,93 (d, 2H) ; 7,05 (m, 2H) ; 7,26 - 7,45 (m, 5H) ; 7,67 (t, 1H) ; 7,80 (d, 1H) ; 7,93 (d, 2H).

**exemple 91 :**

**acide cyclohexylcarboxylique,1-[2-[[[2-butyl-1-[(4-(phénylméthoxycarbonyl) phényl)méthyl]-1H-imidazol-5-yl]méthyl]amino]phénylcarbonyloxy]éthyl ester**

RMN $^1H$ (300 MHz; $CDCl_3$ ; ppm)
0,85 (t, 3H) ; 1,21 - 1,74 (m, 15H) ; 1,86 (m, 2H) ; 2,26 (m, 1H) ; 2,53 (t, 2H) ; 4,15 (d, 2H) ; 5,18 (s, 2H) 5,35 (s, 2H) ; 6,61 (m, 2H) 6,9 (d, 2H) ; 7,1 (m, 2H) ; 7,15 - 7,45 (m, 6H) ; 7,65 (t, 1H) ; 7,8(m, 1H) ; 7,93 (d, 2H).

**exemple 92 :**

**acide 2,2-diméthyl-propanoïque, 1-[2-[[[2-butyl-1-[(4-(phénylméthoxy carbonyl)phényl)méthyl]-1H-imidazol-5-yl]méthyl]amino]phénylcarbonyl oxy]éthyl ester**

RMN $^1H$ (300 MHz; $CDCl_3$; ppm)
0,87 (t, 3H); 1,19 (s, 9H); 1,36 (m, 2H); 1,54 (d, 3H); 1,67 (m, 2H) ; 2,55 (t, 2H) ; 4,17 (d, 2H) ; 5,17 (s, 2H) ; 5,35 (s, 2H); 6,70 (m, 2H) 6,90 (d, 2H) ; 7,02 (m, 2H) ; 7,26 - 7,45 (m, 7H) ; 7,7 (t, 1H) ; 7,82 (m, 1H) ; 7,96 (d,

2H).

**exemple 93 :**

**acide 2,2-diméthyl-propanoïque,[2-[[[2-butyl-1-[(4-(phénylméthoxy carbonyl)phényl)méthyl]-1H-imidazol-5-yl]méthyl]amino]phénylcarbonyl oxy]méthyl ester**

RMN $^1H$ (300 MHz; $CDCl_3$ ; ppm)
0,87 (t, 3H) ; 1,33 (m, 2H) ; 1,47 (s, 9H) ; 1,69 (m, 3H) ; 2,56 (t, 2H) ; 4,17 (d, 2H) ; 4,57 (s, 2H) ; 5,17 (s, 2H) ; 5,35 (s, 2H) ; 6,62 (m, 2H) ; 6,90 (d, 2H) ; 7,03 (s, 1H) ; 7,30 - 7,50 (m, 5 H) ; 7,53 (t, 1H) ; 7,92 (m, 3H).

**exemple 94 :**

**acide hexanoïque,[2-[[[2-butyl-1-[(4-(phénylméthoxycarbonyl)phényl) méthyl]-1H-imidazol-5-yl]méthyl]amino]phénylcarbonyloxy]méthyl ester**

RMN $^1H$ (300 MHz; $CDCl_3$; ppm)
0,85 (m, 6H) ; 1,20 - 1,8 (m, 10H); 2,34 (t, 2H) ; 2,56 (t, 2H) ; 4,17 (d, 2H) ; 5,17 (s, 2H) ; 5,35 (s, 2H) ; 5,84 (s, 2H) ; 6,59 (m, 2H) ; 6,90 (d, 2H) ; 7,03 (s, 1H) ; 7,29 - 7,45 (m, 6H) ; 7,58 (t, 1H) ; 7,84 (m, 1H) ; 7,96 (d, 2H).

**exemple 95 :**

**acide 2-[[[2-butyl-1-[(4-(phénylméthoxycarbonyl)phényl)méthyl]-1H-imidazol-5-yl]méthyl]amino]-benzoïque,2-(N,N-diéthylamino) éthyl ester**

RMN $^1H$ (300 MHz; $CDCl_3$; ppm)
0,85 (t, 3H) ; 1,05 (t, 6H) ; 1,27 (m, 2H) ; 1,65 (m, 2H) ; 2,59 (m, 6H) ; 2,78 (t, 2H) ; 4,08 - 4,26 (m, 4H) ; 5,18 (s, 2H) ; 5,35 (s, 2H) ; 6,62 (m, 2H) ; 6,91 (d, 2H) ; 7,03 (s, 1H) ; 7,26 - 7,50 (m, 6H) ; 7,71 (t, 1H) ; 7,83 (m, 1H) ; 7,93 (d, 2H).

**exemple 96 :**

**acide 2-[[[2-butyl-1-[(4-(phénylméthoxycarbonyl)phényl)méthyl]-1H-imidazol-5-yl]méthyl]amino]-benzoïque,1-méthyl-pentyl ester**

RMN $^1H$ (300 MHz; $CDCl_3$ ; ppm)
0,87 (m, 6H) ; 1,24 - 1,38 (m, 9H) ; 1,68 (m, 4H) ; 2,54 (t, 2H) ; 4,16 (d, 2H) ; 4,98 (m, 1H) ; 5,19 (s, 2H) ; 5,34 (s, 2H) ; 6,60 (m, 2H) ; 6,93 (d, 2H) ; 7,03 (s, 1H) ; 7,29 - 7,42 (m, 6 H) ; 7,78 (t, 1H) ; 7,86 (m, 1H) ; 7,94 (d, 2H).

**exemple 97 :**

**acide 2-[[[2-butyl-1-[(4-(phénylméthoxycarbonyl)phényl)méthyl]-1H-imidazol-5-yl]méthyl]amino]-benzoïque, 1-méthyl-2-oxo-butyl ester**

RMN $^1H$ (300 MHz; $CDCl_3$ ; ppm)
0,84 (m, 3H) ; 1,05 (m, 3H) ; 1,30 - 1,71 (m, 7H) ; 2,54 (m, 4H) ; 4,18 (d, 2H) ; 5,17 (m, 3H) ; 5,35 (s, 2H) ; 6,62 (m, 2H) ; 6,93 (s, 2H) ; 7,03 (s, 1H) ; 7,26 - 7,41 (m, 5H) ; 7,45 (t, 1H) ; 7,93 (m, 3H).

**exemple 99 :**

**acide 2-[[[2-butyl-1-[(4-(phénylméthoxycarbonyl)phényl)méthyl]-1H-imidazol-5-yl] méthyl] amino]-benzoïque, 2-oxo-butyl ester**

RMN $^1H$ (300 MHz; $CDCl_3$; ppm)
0,85 (t, 3H); 1,12 (t, 3H); 1,30 (m, 3H) ; 1,69 (m, 2H); 2,43 (m, 2H) ; 2,55 (m, 2H) ; 4,17 (d, 2H) ; 4,74 (s, 2H) ; 5,17 (s, 2H) ; 5,35 (s, 2H) ; 6,59 (m, 2H) ; 6,90 (d, 2H) ; 7,03 (s, 1H) ; 7,30 - 7,42 (m, 5H) ; 7,46 (t, 1H) ; 7,91 (m, 3H).

**exemple 100 :**

**acide 2-[[2-[[[2-butyl-1-[(4-(phénylméthoxycarbonyl)phényl)méthyl]-1H-imidazol-5-yl] méthyl] amino] phényl] carbonyloxy]-propanoïque, éthyl ester**

RMN $^1H$ (300 MHz; $CDCl_3$ ; ppm)
0,87 (t, 3H) ; 1,23 - 1,40 (m, 5H) ; 1,54 (d, 3H) ; 1,69 (m, 2H) ; 2,55 (t, 2H) ; 4,17 (m, 4H) ; 5,13 (m, 3H) ; 5,35 (s, 2H) ; 6,59 (m, 2H) ; 6,64 (d, 2H) ; 7,02 (s, 1H) ; 7,27 - 7,40 (m, 6H) ; 7,44 (t, 1H) ; 7,93 (m, 3H).

**exemple 101 :**

**acide [[2-[[[2-butyl-1-[(4-(phénylméthoxycarbonyl)phényl)méthyl]-1H-imidazol-5-yl] méthyl] amino] phényl] carbonyloxy]-acétique, pentyl ester**

RMN $^1H$ (300 MHz; $CDCl_3$ ; ppm)
0,87 (m, 6H) ; 1,31 (m, 6H) ; 1,62 (m, 4H) ; 2,56 (t, 2H) ; 4,16 (m, 4H) ; 4,67 (s, 2H) ; 5,15 (s, 2H) ; 5,42 (s, 2H) ; 6,63 (m, 2H) ; 6,90 (d, 2H) ; 7,03 (s, 1H) ; 7,27 - 7,46 (m, 6H) ; 7,50 (t, 1H) ; 7,95 (m, 3H).

**exemple 102 :**

**acide 2-[[[2-butyl-1-[(4-(phénylméthoxycarbonyl)phényl)méthyl]-1H-imidazol-5-yl] méthyl] amino]-benzoïque, 2-phényléthyl ester**

RMN $^1H$ (300 MHz; $CDCl_3$ ; ppm)
0,85 (t, 3H) ; 1,33 (m, 2H) ; 1,69 (1m, 2H) ; 2,55 (t, 2H) ; 2,98 (t, 2H) ; 4,16 (d, 2H) ; 4,35 (t, 2H) ; 5,17 (s, 2H) ; 5,33 (s, 2H) ; 6,60 (m, 2H) ; 6,90 (d, 2H) ; 7,03 (s, 1H) ; 7,2 - 7,45 (m, 11H) ; 7,67 (t, 1H); 7,77 (m, 1H) ; 7,93 (d, 2H).

**exemple 103 :**

**acide 2-[[[2-butyl-1-[(4-(phénylméthoxycarbonyl)phényl)méthyl]-1H-imidazol-5-yl] méthyl] amino]-benzoïque, phényl ester**

RMN $^1H$ (300 MHz; $CDCl_3$ ; ppm)
0,86 (t, 3H) ; 1,29 (m, 2H) ; 1,65 (m, 2H) ; 2,52 (t, 2H) ; 4,20 (d, 2H) ; 5,15 (s, 2H) ; 5,35 (s, 2H) ; 6,75 (m, 2H) ; 6,90 (d, 2H) ; 7,03 (s, 1H) ; 7,08 (m, 2H) ; 7,25 - 7,45 (m, 9H) ; 7,65 (t, 1H) ; 7,91 (d, 2H ) ; 8,05 (m, 1H).

**exemple 104 :**

**acide 2-[[[2-butyl-1-[(4-(phénylméthoxycarbonyl)phényl)méthyl]-1H-imidazol-5-yl] méthyl] amino]-benzoïque, 2-méthoxyéthyl ester**

RMN $^1H$ (300 MHz; $CDCl_3$ ; ppm)
0,87 (t, 3H) ; 1,31 (m, 2H) ; 1,67 (m, 2H) ; 2,56 (t, 2H) ; 3,38 (s, 3H) ; 3,64 (t, 2H) ; 4,17 (d, 2H) ; 4,30 (t, 2H) ; 5,18 (s, 2H) ; 5,35 (s, 2H) ; 6,60 (m, 2H) ; 6,94 (d, 2H) ; 7,04 (s, 1H) ; 7,25 - 7,45 (m, 6H) ; 7,64 (t, 1H) ; 7,86 (m, 1H) ; 7,93 (d, 2H).

**exemple 105 :**

**acide 2-[[[2-butyl-1-[(4-(phénylméthoxycarbonyl)phényl)méthyl]-1H-imidazol-5-yl] méthyl] amino]-benzoïque, décyl ester**

RMN $^1H$ (300 MHz; $CDCl_3$ ; ppm)
0,87 (m, 6H) ; 1,30 (m, 20H); 1,66 (m, 2H) ; 2,55 (t, 2H) ; 4,14 (m, 4H) ; 5,18 (s, 2H) ; 5,33 (s, 2H) ; 6,57 (m, 2H) ; 6,90 (d, 2H) ; 7,03 (s, 1H) ; 7,26 - 7,45 (m, 4H) ; 7,7 (t, 1H) ; 7,85 (m, 1H) ; 7,94 (d, 2H).

**exemple 106 :**

**acide 2-[[[2-butyl-1-[(4-(phénylméthoxycarbonyl)phényl)méthyl)-1H-imidazol-5-yl] méthyl] amino]-benzoïque, heptyl ester**

RMN $^1$H (300 MHz; $CDCl_3$ ; ppm)
0,86 (m, 6H) ; 1,30 (m, 10H) ; 1,66 (m, 4H) ; 2,55 (t, 2H) ; 4,14 (m, 4H) ; 5,18 (s, 2H) ; 5,35 (s, 2H) ; 6,57 (m, 2H) ; 6,90 (d, 2H) ; 7,03 (s, 1H) ; 7,28 - 7,45 (m, 6H) ; 7,27 (t, 1H) ; 7,84 (m, 1H) ; 7,93 (d, 2H).

**exemple 107 :**

**acide 2-[[[2-butyl-1-[(4-(phénylméthoxycarbonyl)phényl)méthyl]-1H-imidazol-5-yl] méthyl] amino]-benzoïque, 3-méthylbutyl ester**

RMN $^1$H (300 MHz; $CDCl_3$ ; ppm)
0,85 (t, 3H) ; 0,93 (d, 6H) ; 1,30 (m, 2H) ; 1,55 (m, 5H) ; 2,55 (t, 2H) ; 4,18 (m, 4H) ; 5,18 (s, 2H) ; 5,35 (s, 2H) ; 6,57 (m, 2H) ; 6,91 (d, 2H) ; 7,04 (s, 1H) ; 7,27 - 7,45 (m, 6H) ; 7,73 (t, 1H) ; 7,83 (m, 1H) ; 7,96 (d, 2H).

**exemple 108 :**

**acide 2-[[[2-butyl-1-[(4-(phénylméthoxycarbonyl)phényl)méthyl]-1H-imidazol-5-yl] méthyl] amino]-benzoïque, 1-méthyléthyl ester**

RMN $^1$H (300 MHz; $CDCl_3$; ppm)
0,85 (t, 3H) ; 1,29 (d, 6H) ; 1,35 (m, 2H) ; 1,66 (m, 2H) ; 2,54 (t, 2H) ; 4,17 (d, 2H) ; 5,06 (m, 1H) ; 5,18 (s, 2H) ; 5,30 (s, 2H) ; 6,59 (m, 2H) ; 6,90 (d, 2H) ; 7,03 (s, 1H) ; 7,26 - 7,45 (m, 6H) ; 7,75 (t, 1H) ; 7,85 (m, 1H) ; 7,93 (d, 2H).

**exemple 109 :**

**acide 2-[[[2-butyl-1-[(4-(phénylméthoxycarbonyl)phényl)méthyl]-1H-imidazol-5-yl] méthyl] amino]-benzoïque, cyclopropylméthyl ester**

RMN $^1$H (300 MHz; $CDCl_3$ ; ppm)
0,29 (m, 2H) , 0,55 (m, 2H) ; 0,87 (t, 3H) ; 1,1 - 1,38 (m, 3H) ; 1,68 (m, 2H) ; 2,52 (t, 2H) ; 3,97 (d, 2H) ; 4,17 (d, 2H) ; 5,18 (s, 2H) ; 5,35 (s, 2H) ; 6,60 (m, 2H) ; 6,95 (d, 2H) ; 7,03 (s, 1H) ; 7,26 - 7,45 (m, 6H) ; 7,69 (t, 1H) ; 7,90 (m, 1H) ; 7,93 (d, 2H).

**exemple 110 :**

**acide 2-[[[2-butyl-1-[(4-(phénylméthoxycarbonyl)phényl)méthyl]-1H-imidazol-5-yl] méthyl] amino]-benzoïque, 2-méthylpropyl ester**

RMN $^1$H (300 MHz; $CDCl_3$ ; ppm)
0,86 (t, 3H) ; 0,96 (d, 6H) ; 0,33 (m, 2H) ; 1,69 (m, 2H) ; 2,0 (m, 1H) ; 2,55 t, 2H) ; 3,93 (d, 2H) ; 4,17 (d, 2H) ; 5,19 (s, 2H) ; 5,34 (s, 2H) ; 6,60 (m, 2H) ; 6,91 (d, 2H) ; 7,04 (s, 1H) ; 7,28 - 7,45 (m, 6H) ; 7,72 (t, 1H) ; 7,87 (m, 1H) ; 7,93 (d, 2H).

**exemple 111 :**

**acide 2-[[[2-butyl-1-[(4-(phénylméthoxycarbonyl)phényl)méthyl]-1H-imidazol-5-yl] méthyl] amino]-benzoïque, hexadécyl ester**

RMN $^1$H (300 MHz; $CDCl_3$; ppm)
0,87 (m, 6H) , 1,20 - 1,74 (m, 32 H) ; 2,55 (t, 2H) ; 4,16 (m, 4H) ; 5,18 (s, 2H) ; 5,35 (s, 2H) ; 6,59 (m, 2H) ; 6,95 (d, 2H) ; 7,03 (s, 1H) ; 7,26 - 7,44 (m, 6H) ; 7,72 (t, 1H) ; 7,84 (m, 1H) ; 7,93 (d, 2H).

**exemple 112 :**

**acide 2-[[[2-butyl-1-[(4-(phénylméthoxycarbonyl)phényl)méthyl]-1H-imidazol-5-yl]méthyl]amino]-benzoïque, butyl ester**

RMN $^1H$ (300 MHz; $CDCl_3$ ; ppm)
0,85 (t, 3H) ; 0,95 (t, 3H) ; 1,38 (m, 4H) ; 1,67 (m, 4H) ; 2,55 (t, 2H) ; 4,17 (m, 4H) ; 6,19 (s, 2H) ; 5,35 (s, 2H) ; 6,60 (m, 2H) ; 6,91 (d, 2H) ; 7,04 (s, 1H) ; 7,27 - 7,45 (m, 6H) ; 7,72 (t, 1H) ; 7,82 (m, 1H) ; 7,95 (d, 2H).

**exemple 113 :**

**acide 2-[[[2-butyl-1-[(4-(éthoxycarbonyl)phényl)méthyl]-1H-imidazol-5-yl] méthyl] amino]-benzoïque, éthyl ester**

RMN $^1H$ (300 MHz; $CDCl_3$ ; ppm)
0,85 (t, 3H) ; 1,36 (m, 8H) ; 1,69 (m, 2H) ; 2,55 (t, 2H) ; 4,19 (m, 4H) ; 4,37 (q, 2H) ; 5,18 (s, 2H) ; 6,60 (m, 2H) ; 6,90 (d, 2H) ; 7,04 (s, 1H) ; 7,30 (m, 1H) ; 7,74 (t, 1H) ; 7,84 - 7,92 (m, 3H).

**exemple 114 :**

**acide 2-[[[2-butyl-1-[(4-(méthoxycarbonyl)phényl)méthyl]-1H-imidazol-5-yl] méthyl]amino]-benzoïque, pentyl ester**

RMN $^1H$ (300 MHz; $CDCl_3$ ; ppm)
0,85 (m, 6H) ; 1,36 (m, 6H) ; 1,74 (m, 4H) ; 2,55 (t, 2H) ; 3,90 (s, 3H) ; 4,16 (m, 4H) ; 5,19 (s, 2H) ; 6,64 (m, 2H) ; 6,94 (d, 2H) ; 7,04 (s, 1H) ; 7,30 (m, 1H) ; 7,73 (t, 1H) ; 7,83 - 7,92 (m, 3H).

**exemple 115 :**

**acide 2-[[[1-[(4-(phénylméthoxycarbonyl)phényl)méthyl]-2-propyl-1H-imidazol-5-yl] méthyl] amino]-benzoïque, pentyl ester**

RMN $^1H$ (300 MHz; $CDCl_3$ ; ppm)
0,90 (m, 6H) ; 1,40 (m, 4H) ; 1,64 (m, 4H) ; 2,53 (t, 2H) ; 4,16 (m, 4H) ; 5,18 (s, 2H) ; 5,35 (s, 2H) ; 6,59 (m, 2H) ; 6,93 (d, 2H) ; 7,04 (s, 1H) ; 7,28 - 7,45 (m, 6H) ; 7,72 (t, 1H) ; 7,85 (m, 1H) ; 7,96 (d, 2H).

**exemple 116 :**

**acide 2-[[[1-[(4-(méthoxycarbonyl)phényl)méthyl]-2-propyl-1H-imidazol-5-yl]méthyl]amino]-4-nitro-benzoïque, méthyl ester**

RMN $^1H$ (300 MHz; $CDCl_3$ ; ppm)
0,95 (t, 3H) ; 1,74 (m, 2H) ; 2,56 (t, 2H) ; 3,81 (s, 3H) ; 4,02 (s, 3H) ; 4,28 (d, 2H) ; 5,17 (s, 2H) ; 6,91 (d, 2H) ; 7,10 (s, 1H) ; 7,35 (m, 1H) ; 7,74 (m, 1H) ; 7,92 (m, 4H).

**exemple 117 :**

**acide 2-[[[2-butyl-1-[(4-((1,1-diméthyléthoxy)carbonyl)phényl)méthyl]-1H-imidazol-5-yl]méthyl]amino]-4-nitro-benzoïque, méthyl ester**

RMN $^1H$ (300 MHz; $CDCl_3$ ; ppm)
0,87 (t, 3H) ; 1,35 (m, 2H) ; 1,58 (s, 9H) ; 1,69 (m, 2H) ; 2,58 (t, 2H) ; 3,81 (s, 3H) ; 4,26 (d, 2H) ; 5,16 (s, 2H) ; 6,87 (d, 2H) ; 7,09 (s, 1H) ; 7,37 (m, 1H) ; 7,47 (m, 1H) ; 7,82 - 7,95 (m, 4H).

**exemple 118 :**

**acide hexanoïque, 1-[2-[[[2-butyl-1-[(4-phénylméthoxycarbonyl)phényl) méthyl]-1H-imidazol-5-yl]méthyl]amino]phénylcarbonyloxy]éthyl ester**

RMN $^1$H (300 MHz; $CDCl_3$; ppm)
0,87 (m, 6H) ; 1,31 (m, 6H) ; 1,52 (d, 3H) ; 1,62 (m, 4H) ; 2,30 (t, 2H) ; 2,55 (t, 2H) ; 4,16 (d, 2H) ; 5,18 (s, 2H) ; 5,35 (s, 2H) ; 6,56 (m, 2H) ; 6,90 (d, 2H) ; 7,03 (m, 2H) ; 7,27 - 7,45 (m, 6H) ; 7,66 (t, 1H) ; 7,82 (m, 1H) ; 7,93 (d, 2H).

**exemple 119 :**

**acide 2-[[[2-butyl-1-[(4-(phénylméthoxycarbonyl)phényl)méthyl]-1H-imidazol-5-yl]méthyl]amino]-benzoïque, 1,1-diméthyléthyl ester**

RMN $^1$H (300 MHz; $CDCl_3$ ; ppm)
0,85 (t, 3H) ; 1,25 (m, 2H) ; 1,44 (s, 9H) ; 1,66 (m, 2H) ; 2,55 (t, 2H) ; 4,18 (d, 2H) ; 5,19 (s, 2H) ; 5,35 (s, 2H) ; 6,55 (m, 2H) ; 6,90 (d, 2H) ; 7,04 (s, 1H) ; 7,2 - 7,45 (m, 6H) ; 7,76 (m, 2H) ; 7,96 (d, 2H).

**exemple 120 :**

**acide 2-[[[2-butyl-1-[(4-(phénylméthoxycarbonyl)phényl)méthyl]-1H-imidazol-5-yl]méthyl]amino]-benzoïque, éthyl ester**

RMN $^1$H (300 MHz ; $CDCl_3$ ; ppm)
0,85 (t, 3H) ; 1,32 (m, 5H) , 1,71 (m, 2H) ; 2,57 (t, 2H) ; 4,15 (m, 4H) ; 5,18 (s, 2H) ; 5,35 (s, 2H) ; 6,59 (m, 2H) ; 6,90 (d, 2H) ; 7,04 (s, 1H) ; 7,25 - 7,45 (m, 6H) ; 7,71 (t, 1H) ; 7,81 (m, 1H) ; 7,92 (d, 2H).

**exemple 121 :**

**acide 2-[[[2-butyl-1-[(4-(méthoxycarbonyl)phényl)méthyl]-1H-imidazol-5-yl]méthyl]amino]-benzoïque, 1-((pentylcarbonyl)oxy)éthyl ester**

RMN $^1$H (300 MHz; $CDCl_3$ ; ppm)
0,85 (t, 6H) ; 1,32 (m, 6H) ; 1,54 (d, 3H) ; 1,67 (m, 4H) ; 2,31 (t, 2H) ; 2,56 (t, 2H) ; 3,90 (s, 3H) ; 4,18 (d, 2H) ; 5,18(s, 2H) ; 6,58 (m, 2H) ; 6,94 (d, 2H) ; 7,03 (m, 2H) ; 7,35 (m, 1H) ; 7,66 (t, 1H) ; 7,82 (m, 1H) ; 7,92 (d, 2H).

**exemple 122 :**

**acide 2-[[[2-butyl-1-[(4-(phénylméthoxycarbonyl)phényl)méthyl]-1H-imidazol-5-yl]méthyl]amino]-benzoïque, pentyl ester**

RMN $^1$H (300 MHz; $CDCl_3$ ; ppm)
0,87 (m, 6H) ; 1,33 (m, 6H) ; 1,68 (m, 4H) ; 2,55 (t, 2H) ; 4,14 (m, 4H) ; 5,18 (s, 2H) ; 5,35 (s, 2H) ; 6,59 (m, 2H) ; 6,93 (d, 2H) ; 7,04 (s, 1H) ; 7,26 - 7,45 (m, 6H) ; 7,72 (t, 1H) ; 7,82 (m, 1H) ; 7,93 (d, 2H).

**exemple 123 :**

**acide cyclohexylacétique,1-[2-[[[2-butyl-1-[(4-(phénylméthoxycarbonyl) phényl)méthyl]-1H-imidazol-5-yl]méthyl]amino]phénylcarbonyloxy]éthyl ester**

RMN $^1$H (300 MHz; $CDCl_3$ ; ppm)
0,85 (t, 3H) ; 0,95 (m, 2H) ; 1,10 - 1,39 (m, 4H) ; 1,52 (d, 3H) ; 1,57 (m, 9H) ; 2,17 (m, 2H) ; 2,55 (t, 2H) ; 4,17 (d, 2H) ; 5,17 (s, 2H) ; 5,35 (s, 2H) ; 6,56 (m, 2H) ; 6,90 (d, 2H) ; 7,02 (m, 2H) ; 7,15 - 7,45 (m, 6H) 7,68 (t, 1H) ; 7,82 (m, 1H) ; 7,96 (d, 2H).

**exemple 124 :**

**acide 2-[[[2-butyl-1-[(4-(pentoxycarbonyl)phényl)méthyl]-1H-imidazol-5-yl]méthyl]amino]-benzoïque, phénylméthyl ester**

RMN $^1$H (300 MHz; $CDCl_3$ ; ppm)
0,88 (m, 6H) ; 1,37 (m, 6H) ; 1,65 - 1,75 (m, 4H) ; 2,56 (t, 2H) ; 4,20 (d, 2H) ; 4,27 (t, 2H) ; 5,17 (s, 2H) ; 5,22 (s, 2H) ; 6,60 (t, 1H) ; 6,64 (d, 1H) ; 7,0 (s, 1H) ; 7,26 - 7,39 (m, 6H) ; 7,69 (t, 1H) ; 7,88 - 7,93 (m, 3H).

**exemple 125 :**

**acide 2-[[[2-butyl-1-[(4-(méthoxycarbonyl)phényl)méthyl]-1H-imidazol-5-yl]méthyl]amino]-benzoïque, phénylméthyl ester**

RMN $^1$H (300 MHz; $CDCl_3$; ppm)
0,86 (t, 3H) ; 1,33 (m, 2H) ; 1,69 (m, 2H) ; 2,55 (t, 2H) ; 3,87 (s, 3H) ; 4,16 (d, 2H) ; 5,17 (s, 2H) ; 5,21 (s, 2H) ; 6,58 (t, 1H) ; 6,64 (d, 1H) ; 6,90 (d, 2H) ; 7,04 (s, 1H) ; 7,26 - 7,41 (m, 6H) ; 7,66 (t, 1H) ; 7,88 - 7,92 (m, 3H).

**exemple 126 :**

**acide 2-[[[2-butyl-1-[(4(éthoxycarbonyl)phényl)méthyl]-1H-imidazol-5-yl] méthyl]amino]-benzoïque, phénylméthyl ester**

RMN $^1$H (300 MHz; $CDCl_3$ ; ppm)
0,86 (t, 3H) ; 1,33 (m, 5H) ; 1,69 (m, 2H) ; 2,56 (t, 2H) ; 4,18 (d, 2H) ; 4,33 (q, 2H) ; 5,17 (s, 2H) ; 5,22 (s, 2H) ; 6,60 (t, 1H) ; 6,64 (d, 1H) ; 6,91 (d, 2H) ; 7,05 (s, 1H) ; 7,26 - 7,41 (m, 6H) ; 7,70 (t, 1H) ; 7,88 - 7,97 (m, 3H).

**exemple 127 :**

**acide 2-[[[2-butyl-1-[(4-(butoxycarbonyl)phényl)méthyl]-1H-imidazol-5-yl] méthyl]amino]-benzoïque, phénylméthyl ester**

RMN $^1$H (300 MHz; $CDCl_3$; ppm)
0,83 - 0,98 (m, 6H) ; 1,28 - 1,48 (m, 4H) ; 1,65 - 1,73 (m, 4H) ; 2,56 (t, 2H) ; 4,16 (d, 2H) ; 4,26 (t, 2H) ; 5,17 (s, 2H) ; 5,21 (s, 2H) ; 6,57 (t, 1H) ; 6,62 (d, 1H) ; 6,90 (d, 2H) ; 7,04 (s, 1H) ; 7,26 - 7,39 (m, 6H) ; 7,70 (t, 1H) ; 7,91 (m, 3H).

**exemple 128 :**

**acide 2-[[[2-butyl-1-[(4-(hexadécyloxycarbonyl)phényl)méthyl]-1H-imidazol-5-yl]méthyl]amino]-benzoïque, phénylméthyl ester**

RMN $^1$1H (300 MHz; $CDCl_3$ ; ppm)
0,88 (m, 6H) ; 1,25 - 1,41 (m, 28H) ; 1,64 - 1,76 (m, 4H) ; 2,56 (t, 2H) ; 4,18 (d, 2H) ; 4,26 (t, 2H) ; 5,17 (s, 2H) ; 5,22 (s, 2H) ; 6,58 (t, 1H) ; 6,64 (d, 1H) ; 6,90 (d, 2H) ; 7,04 (s, 1H) ; 7,26 - 7,39 (m, 6H) ; 7,70 (t, 1H) ; 7,88 - 7,93 (m, 3H).

**exemple 129 :**

**acide 2-[[[2-butyl-1-[(4-((2-méthyl-propyl)oxycarbonyl)phényl)méthyl]-1H-imidazol-5-yl]méthyl]amino]-benzoïque, phénylméthyl ester**

RMN $^1$H (300 MHz; $CDCl_3$ ; ppm)
0,85 (t, 3H) ; 0,96 (d, 6H) ; 1,29 - 1,41 (m, 2H) ; 1,65 -1,75 (m, 2H) ; 2,04 (m, 1H) ; 2,56 (t, 2H) ; 4,05 (d, 2H) ; 4,18 (d, 2H) ; 5,18 (s, 2H) ; 5,22 (s, 2H) ; 6,60 (t, 1H) ; 6,65 (d, 1H) ; 6,91 (d, 2H) ; 7,04 (s, 1H) ; 7,26 - 7,39 (m, 6H) ; 7,70 (t, 1H) ; 7,89 - 7,94 (m, 3H).

**exemple 130 :**

**acide 2-[[[2-butyl-1-[(4-(cyclopropyl-méthyloxycarbonyl)phényl)méthyl]-1H-imidazol-5-yl]méthyl]amino]-benzoïque, phénylméthyl ester**

RMN $^1$H (300 MHz; $CDCl_3$ ; ppm)
0,36 (q, 2H) ; 0,58 (q, 2H) ; 0,86 (t, 3H) ; 1,19 - 1,39 (m, 3H) ; 1,68 - 1,75 (m, 2H) ; 2,56 (t, 2H) ; 4,10 (d, 2H) ; 4,18 (d, 2H) ; 5,18 (s, 2H) ; 5,22 (s, 2H) ; 6,60 (t, 1H) ; 6,64 (d, 1H) ; 6,91 (d, 2H) ; 7,04 (s, 1H) ; 7,26 - 7,41 (m, 6H) ; 7,68 (t, 1H) ; 7,89 - 7,96 (m, 3H).

**exemple 131 :**

**acide 2-[[[2-butyl-1-[(4-((3-méthyl-butyl)oxycarbonyl)phényl)méthyl]-1H-imidazol-5-yl]méthyl]amino]-benzoïque, phénylméthyl ester**

RMN $^1$H (300 MHz; $CDCl_3$; ppm)
0,86 (t, 3H) ; 0,94 (d, 6H) ; 1,25 - 1,41 (m, 2H) ; 1,59 - 1,80 (m, 5H) ; 2,56 (t, 2H) ; 4,18 (d, 2H) ; 4,30 (t, 2H) ; 5,17 (s, 2H) ; 5,22 (s, 2H) ; 6,60 (t, 1H) ; 6,64 (d, 1H) ; 6,90 (d, 2H) ; 7,04 (s, 1H) ; 7,26 - 7,39 (m, 6H) ; 7,70 (t, 1H) ; 7,88 - 7,93 (m, 3H).

**exemple 132 :**

**acide 2-[[[2-butyl-1-[(4-cyanophényl)méthyl]-1H-imidazol-5-yl]méthyl] amino]-benzoïque, pentyl ester**

RMN $^1$H (300 MHz; $CDCl_3$ ; ppm)
0,84 - 0,95 (m, 6H) ; 1,31 - 1,42 (m, 6H) ; 1,64 - 1,75 (m, 4H) ; 2,52 (t, 2H) ; 4,15 (t, 2H) ; 4,20 (d, 2H) ; 5,19 (s, 2H) ; 6,62 (t, 1H) ; 6,66 (d, 1H) ; 6,90 (d, 2H) ; 7,07 (s, 1H) ; 7,31 (t, 1H) ; 7,47 (d, 2H) ; 7,68 (t, 1H) ; 7,83 (d, 1H).

**exemple 133 :**

**acide 2-[[[2-butyl-1-[(4-((1,1-diméthyléthoxy)carbonyl)phényl)méthyl]-1H-imidazol-5-yl]méthyl]amino]-4-nitro-benzoïque, pentyl ester**

RMN $^1$H (300 MHz; $CDCl_3$ ; ppm)
0,84 - 0,95 (m, 6H) ; 1,25 - 1,42 (m, 6H) ; 1,58 (s, 9H) ; 1,62 - 1,76(m, 4H) ; 2,57 (t, 2H) ; 4,21 (t, 2H) ; 4,23 (d, 2H) ; 5,13 (s, 2H) ; 6,88 (d, 2H) ; 7,08 (s, 1H) ; 7,38 (d, 1H) ; 7,45 (s, 1H) ; 7,85 (d, 2H) ; 7,95 (d, 2H).

**exemple 221 :**

acide 3-méthyl-2-[[[2-[[[2-butyl-1-[(4-(phénylméthoxycarbonyl)phényl)méthyl]-1H-imidazol-5-yl]méthyl]amino]phényl]carbonyl]amino]-butanoïque, éthyl ester

RMN $^1$H (300 MHz; $CDCl_3$; ppm)
0,86 (t, 3H) ; 0,97 (m, 6H) ; 1,32 (m, 5H) ; 1,70 (m, 2H) ; 2,20 (m, 1H) ; 2,53 (t, 2H) ; 4,11 (d, 2H) ; 4,23 (m, 2H) ; 4,60 (m, 1H) ; 5,19 (s, 2H) ; 5,35 (s, 2H) ; 6,51 (d, 1H) ; 6,63 (t, 2H) ; 6,96 (d, 2H) ; 7,0 (s, 1H) ; 7,26 (t, 1H) ; 7,40 (m, 6H) ; 7,52 (t, 1H) ; 7,95 (d, 2H).

**exemple 9 :**

**acide 2-[[[2-butyl-4-chloro-1-[(4-(méthoxycarbonyl)phényl)méthyl]-1H-imidazol-5-yl]méthyl]amino]-3,4,5-triméthoxy-benzoïque, méthyl ester**

A une solution de 3,55 g ($9,06.10^{-3}$ mole) de chlorhydrate de l'acide 4-[(2-butyl-4-chloro-5-chlorométhyl-1H-imidazol-1-yl)méthyl]-benzoïque, méthyl ester dans 30 ml de N-méthylpyrrolidone on additionne 4,82 g ($20.10^{-3}$ mole) de 2-amino-3,4,5-triméthoxy-benzoate de méthyle. Le mélange réactionnel est chauffé à 80°C pendant 5 heures. Après addition de 100 ml d'eau la phase aqueuse est extraite par 2 fois 60 ml d'acétate

d'éthyle. Les phases organiques sont lavées à l'eau jusqu'à pH neutre, séchées sur sulfate de magnésium, filtrées et évaporées sous pression réduite. Le résidu huileux obtenu est purifié par chromatographie en éluant avec un mélange toluène/acétate d'éthyle (85/15 ; v/v). On obtient ainsi 2,43 g (rendement : 48 %) d'huile jaune.

RMN $^1H$ (300 MHz; $CDCl_3$; ppm)
0,85(t,3H) ; 1,28(m,2H) ; 1,63(m,2H) ; 2,48(t,2H) ; 3,65(s,3H) ; 3,81(s,3H) ; 3,82(s,3H) ; 3,91(s,3H) ; 3,92(s,3H) ; 4,34(s,2H) ; 5,27(s,2H) ; 6,73(s,1H) ; 6,98(d,2H) ; 7,17(s,1H) ; 7,95(d,2H).

**exemple 10 :**

**acide 2-[[[2-butyl-4-chloro-1-[(4-(méthoxycarbonyl)phényl)méthyl]-1H-imidazol-5-yl]méthyl]amino]-benzoïque, méthyl ester**

Une suspension de 0,9 g (2,3.10$^{-3}$mole) de chlorhydrate de l'acide 4-[(2-butyl-4-chloro-5-chlorométhyl-1H-imidazol-1-yl)méthyl]-benzoïque, méthyl ester dans 4,5 ml d'anthranilate de méthyle est chauffée à 120°C pendant 20 minutes. Après addition de 15 ml d'eau et de 15 ml d'une solution saturée de bicarbonate de sodium, le mélange réactionnel est extrait par 30 ml d'acétate d'éthyle. La phase organique est lavée à l'eau jusqu'à neutralité, séchée sur sulfate de magnésium et évaporée sous pression réduite. L'huile jaune obtenue est purifiée par chromatographie sur silice en éluant avec un mélange toluène/acétate d'éthyle (90/10 ; v/v). On obtient ainsi 1,07 g (rendement : 90 %) d'une huile incolore.

RMN $^1H$ (300 MHz; $CDCl_3$; ppm)
0,86(t,3H) ; 1,34(m,2H) ; 1,66(m,2H) ; 2,52(t,3H) ; 3,76(s,3H) ; 3,91(s,3H) ; 4,21(d,2H) ; 5,30(s,2H) ; 6,62(t,1H) ; 6,72(d,1H) ; 6,93(d,2H) ; 7,31-7,39(m,1H); 7,69(m,1H); 7,81(d,1H); 7,90(d,1H).

En opérant de façon analogue à la préparation de l'exemple 10, on a obtenu le produit de l'exemple 56 et le produit suivant:

**exemple 57 :**

**acide 4-[[2-butyl-4-chloro-5-[((4-cyanophényl)amino)méthyl]-1H-imidazol-1-yl]méthyl]-benzoïque, méthyl ester**

RMN $^1H$ (300 MHz; $CDCl_3$ ; ppm)
0,87(t,3H) ; 1,35(m,2H) ; 1,67(m,2H) ; 2,55(t,2H) ; 3,91(s,3H) ; 4,18(d,2H) ; 4,45(t,1H) ; 5,21(s,2H) ; 6,68-6,76(m,2H) ; 6,95(d,2H) ; 7,33-7,40(m,2H) ; 7,94(d,2H).

**exemple 11 :**

**acide N-[[2-butyl-4-chloro-1-[(4-(méthoxycarbonyl)phényl)méthyl]-1H-imidazol-5-yl]méthyl]-indole-2-carboxylique, éthyl ester**

A une solution de 4,26 g (22,5.10$^{-3}$ mole) d'indole-2-carboxylate d'éthyle dans 50 ml de diméthylformamide anhydre, on ajoute par fractions 0,68 g (22,6.10$^{-3}$ mole) d'hydrure de sodium à 80 % dans l'huile. Après agitation à température ambiante pendant 20 minutes on ajoute 4 g (11,26.10$^{-3}$ mole) de chlorhydrate de l'acide 4-[(2-butyl-4-chloro-5-chlorométhyl-1H-imidazol-1-yl)méthyl]-benzoïque, méthyl ester. L'agitation est maintenue pendant 4,5 heures. Le mélange réactionnel est additionné de 400 ml d'eau et extrait plusieurs fois à l'acétate d'éthyle. Les phases organiques sont lavées à l'eau jusqu'à neutralité, séchées sur sulfate de magnésium et évaporées sous pression réduite. L'huile brune obtenue est purifée par chromatographie sur silice en éluant avec un mélange toluène/acétate d'éthyle (95/5 ; v/v). On obtient 2,06 g (rendement : 36 %) du produit attendu.

**F = 136°C**

En opérant de façon analogue, on a préparé le produit de l'exemple 12.

**exemple 13 :**

**acide 2-[[[4-chloro-2-propyl-1-[(4-(méthoxycarbonyl)phényl)méthyl]-1H-imidazol-5-yl]méthyl]amino]-benzoïque, méthyl ester**

2,93g (7,7.10$^{-3}$ mole) de chlorhydrate de l'acide 4-[(4-chloro-5-chlorométhyl-2-propyl-1H-imidazol-1-yl)méthyl]-benzoïque, méthyl ester sont mis en suspension dans 30 ml de toluène anhydre. On ajoute 2,6 g (17.10$^{-3}$ mole) d'anthranilate de méthyle puis on chauffe à reflux pendant 3 heures tout en agitant. Le mélange

réactionnel est alors versé dans une solution satureé de bicarbonate de sodium. On extrait à l'acétate d'éthyle. Les phases organiques sont lavées à l'eau jusqu'à neutralité, séchées sur sulfate de magnésium et concentrées. Le résidu huileux obtenu est purifié par chromatographie sur silice en éluant par le mélange toluène/acétate d'éthyle (95/5 ; v/v). On obtient ainsi 2,1 g (rendement : 59 %) de solide beige.

**F = 108°C**

En opérant de façon analogue, on a préparé les produits des exemples 14, 15, 17, 18, 21 et 22 et les produits suivants :

**exemple 16 :**

**acide 3-[[[2-butyl-4-chloro-1-[(4-(méthoxycarbonyl)phényl)méthyl]-1H-imidazol-5-yl]méthyl]amino]-naphtalène-2-carboxylique, méthyl ester**

huile jaune

RMN $^1H$ (300 MHz; $CDCl_3$ ; ppm)

0,86(t,3H) ; 1,33(m,2H) ; 1,67(m,2H) ; 2,51(t,3H) ; 3,83(s,3H) ; 3,92(s,3H) ; 4,30(d,2H) ; 5,29(s,1H) ; 6,90(m,3H) ; 7,20(m,1H) ; 7,39(m,2H) ; 7,58(d,1H) ; 7,66(d,1H) ; 7,84(d,2H) ; 8,41 (s,1H).

**exemple 19 :**

**acide 2-[[[2-butyl-4-iodo-1-[(4-(méthoxycarbonyl)phényl)méthyl]-1H-imidazol-5-yl]méthyl]amino]-benzoïque, méthyl ester**

huile jaune

RMN $^1H$ (300 MHz; $CDCl_3$ ; ppm)

0,85(t,3H) ; 1,28(m,2H) ; 1,68(1m,2H) ; 2,55(t,2H) ; 3,77(s,3H) ; 3,91(s,3H) ; 4,18(d,2H) ; 5,23(s,2H) ; 6,64(m,2H) ; 6,89(d,2H) ; 7,33(t,1H) ; 7,66(t,1H) ; 7,80(d,1H); 7,88(d,2H).

**exemple 20 :**

**acide 2-[[[2-butyl-4-trifluorométhyl-1-[(4-(méthoxycarbonyl)phényl) méthyl]-1H-imidazol-5-yl]méthyl]amino]-benzoïque, méthyl ester**

huile incolore

RMN $^1H$ (300 MHz; $CDCl_3$; ppm)

0,87(t,3H) ; 1,34(m,2H) ; 1,71(m,2H) ; 2,59(t,2H) ; 3,78(s,3H) ; 3,91(s,3H) ; 4,32(d,2H) ; 5,22(s,2H) ; 6,64(m,2H) ; 6,92(d,2H) ; 7,31(m,1H) ; 7,64(t,1H) ; 7,83(d,1H) ; 7,92(d,2H),

**exemple 215 :**

**acide 4-[[2-butyl-5-[((2-(((3-(diméthylamino)propyl)amino)carbonyl)phényl) amino)méthyl]-1H-imidazol-1-yl]méthyl]-benzoïque, méthyl ester**

RMN $^1H$ (300 MHz; $CDCl_3$; ppm)

0,86 (t,3H) ; 1,32 (m, 2H) ; 1,70 (m, 4H) ; 2,27 (s, 6H) ; 2,48 (m, 4H) ; 3,44 (m, 2H) ; 3,88 (s, 3H) ; 4,12 (d, 2H) ; 5,21 (s, 2H) ; 6,59 (t, 1H) ; 6,64 (d, 1H) ; 6,94 (d, 2H) ; 7,0 (s, 1H) ; 7,23 (t, 2H) ; 7,91 (d, 2H) ; 7,99 (t, 1H) ; 8,27 (t, 1H).

**exemple 23 :**

**acide 2-[[[2-butyl-4-chloro-1-[(4-carboxyphényl)méthyl]-1H-imidazol-5-yl] méthyl]amino]-benzoïque, méthyl ester**

A une solution de 9 g ($19,1.10^{-3}$ mole) d'acide 2-[[[2-butyl-4-chloro-1-[(4-(méthoxycarbonyl)phényl)méthyl]-1H-imidazol-5-yl]méthyl]amino]-benzoïque, méthyl ester dans 80 ml de méthanol on additionne 0,8 g ($20.10^{-3}$ mole) d'hydroxyde de sodium et 10 ml d'eau. Le mélange réactionnel est chauffé à 50°C pendant 3,5 heures. Le méthanol est évaporé sous pression réduite et le résidu est étendu de 150 ml d'eau. La phase aqueuse est lavée par 3 fois 50 ml d'acétate d'éthyle puis acidifiée par de l'acide chlorhydrique 1N jusqu'à pH

= 5 et extraite par 2 fois 50 ml d'acétate d'éthyle. Les phases organiques sont lavées à l'eau jusqu'à neutralité, séchées sur sulfate de magnésium, filtrées et évaporées sous pression réduite. Le résidu solide est purifié par chromatographie sur silice en éluant à l'aide d'un mélange dichlorométhane/méthanol (95/5 ; v/v). On obtient 5,1 g (rendement : 58 %) de solide blanc.

**F = 181°C**

En opérant de façon analogue, on a obtenu les produits des exemples 24 ,223, et 224.

**exemple 25 :**

**acide 2-[[[2-butyl-1-[(4-carboxyphényl)méthyl)-1H-imidazol-5-yl]méthyl] amino]-benzoïque, phénylméthyl ester**

Une solution de 2,6 g (4,7.$10^{-3}$ mole) de l'acide 2-[[[2-butyl-1-[(4-(1,1-diméthyléthoxycarbonyl)phényl)méthyl]-1H-imidazol-5-yl]méthyl]amino]-benzoïque, phénylméthyl ester dans 10 ml d'acide trifluoroacétique est agitée à 0°C pendant 3 heures. L'acide trifluoroacétique est évaporé sous pression réduite. Après addition de 60 ml d'eau au résidu et de soude jusqu'à pH = 6, on extrait par 2 fois 30 ml d'acétate d'éthyle. La phase organique est lavée par 2 fois 10 ml d'eau, séchée sur sulfate de magnésium, filtrée et évaporée sous pression réduite. On obtient 2,4 g (rendement : 100 %) d'une mousse jaune.

**F = 90°C**

En opérant de façon analogue, on a préparé les produits des exemples 195 à 197.

**exemple 26 :**

**acide 2-[[[2-butyl-1-[(4-carboxyphényl)méthyl]-1H-imidazol-5-yl]méthyl] amino]-benzoïque, 2,2-diméthyl-1,3-dioxolane-4-yl-méthyl ester**

A une solution de 3,9 g (6,38.$10^{-3}$ mole) d'acide 2-[[[2-butyl-1-[((4-phénylméthoxycarbonyl)phényl)méthyl]-1H-imidazol-5-yl]méthyl]amino]-benzoïque, 2,2-diméthyl-1,3-dioxolane-4-yl-méthyl ester dans 150 ml de méthanol on additionne sous atmosphère d'azote, 0,39 g de charbon palladié à 10 %. Le milieu réactionnel est placé ensuite sous atmosphère d'hydrogène et agité pendant 2,5 heures. Après filtration, le méthanol est évaporé sous pression réduite. Le résidu obtenu est purifié par chromatographie sur silice en éluant à l'aide d'un mélange dichlorométhane/méthanol (90/10 ; v/v). On obtient 2,3 g (rendement : 69 %) d'une mousse blanche.

**F = 92°C**

En opérant de façon analogue, on a préparé les produits des exemples 27, 134 à 179.

**exemple 28 :**

**acide 2-[[[2-butyl-1-[(4-carboxyphényl)méthyl]-1H-imidazol-5-yl]méthyl] amino]-benzoïque, 2,3-dihydroxy-propyl ester**

Une suspension de 2 g (3,83.$10^{-3}$ mole) d'acide 2-[[[2-butyl-1-[(4-carboxyphényl)méthyl]-1H-imidazol-5-yl]méthyl]amino]-benzoïque, 2,2-diméthyl-1,3-dioxolane-4-yl-méthyl ester dans 100 ml d'acide chlorhydrique 1N est agitée à température ambiante pendant 2 heures. Le mélange réactionnel est amené à pH = 7 à l'aide d'une solution d'hydroxyde de sodium 5N puis extrait par 2 fois 50 ml de butanol. La phase organique est lavée à l'eau et évaporée sous pression réduite. La mousse blanche obtenue est purifiée par chromatographie sur silice en éluant à l'aide d'un mélange chlorure de méthylène/méthanol (90/10 ; v/v). On obtient 7,3 g (rendement : 71 %) de poudre blanche.

**F = 123°C**

En opérant de manière analogue, on a préparé les produits des exemples 29, 30, 31,71 et 218.

**exemple 32 :**

**acide 2-[[[2-butyl-1-[(4-carboxyphényl)méthyl]-1H-imidazol-5-yl]méthyl] amino]-benzoïque**

5,1 g (11,7.$10^{-3}$ mole) de l'ester méthylique de l'acide 2-[[[2-butyl-1-[((4-méthoxycarbonyl)phényl)méthyl]-1H-imidazol-5-yl]méthyl]amino]-benzoïque sont dissous dans 50 ml de méthanol. On ajoute 17,6 ml (35,2.$10^{-3}$ mole) de soude 2N et porte à reflux pendant 4 heures puis le méthanol est évaporé et le résidu est solubilisé

dans l'eau glacée. Le diacide est précipité par addition d'acide chlorhydrique 1N jusqu'à obtention d'un pH égal à 4. Le solide obtenu est filtré, lavé à l'eau jusqu'à neutralité et séché sur anhydride phosphorique pour donner 3,75 g d'un solide jaune pâle. Ce produit brut est lavé par du méthanol chaud ce qui conduit à 3,5 g (rendement : 73,5 %) de solide blanc.

**F = 234°C**

En opérant de façon analogue, on a préparé les produits des exemples 33 à 52, 55, 66, 67, 198 à 212, 226 et 228.

**exemple 53 :**

**acide 2-[[[2-butyl-1-[(4-carboxyphényl)méthyl]-1H-imidazol-5-yl]méthyl] amino]-benzoïque, di-sel de potassium**

On mélange 203 mg ($0,5.10^{-3}$ mole) de l'acide 2-[[[2-butyl-1-[(4-carboxyphényl)méthyl]-1H-imidazol-5-yl]méthyl]amino]-benzoïque à 10 ml de potasse 0,1 N ($10^{-3}$ mole) et 20 ml d'eau. On agite jusqu'à obtention d'une solution limpide et on lyophilise. On obtient 240 mg (rendement : 100 %) d'un solide blanc.

**F = 206°C**

**exemple 54 :**

**acide N-[[2-butyl-4-chloro-1-[(4-(méthoxycarbonyl)phényl)méthyl]-1H-imidazol-5-yl]méthyl]-N-(méthylcarbonyl)-2-amino-benzoïque, méthyl ester**

A une solution de 2,5 g ($5,31.10^{-3}$ mole) d'acide 2-[[[2-butyl-4-chloro-1-[(4-(méthoxycarbonyl)phényl)méthyl]-1H-imidazol-5-yl]méthyl]amino]-benzoïque, méthyl ester dans 25 ml de pyridine on ajoute 12,5 ml d'anhydride acétique et on chauffe à 60°C pendant 1,5 heure. La solution est versée dans une solution froide d'acide chlorhydrique 1N. On extrait à l'acétate d'éthyle, lave les phases organiques par une solution d'acide chlorhydrique 1N puis par la saumure jusqu'à obtention d'un pH = 4. Après séchage sur sulfate de magnésium et concentration, on obtient 3,3 g d'huile jaune pâle qui est cristallisée dans 100 ml d'éther éthylique pour donner 1,85 g (rendement : 73 %) de cristaux blancs.

**F = 142°C**

**exemple 58 :**

**acide 2-[[[2-butyl-4-chloro-1-[(4-((triphénylméthyl)-1H-tétrazol-5-yl) phényl)méthyl]-1H-imidazol-5-yl]méthyl]amino]-benzoïque, méthyl ester**

4,3 g ($9,84.10^{-3}$ mole) d'acide 2-[[[2-butyl-4-chloro-1-[(4-cyanophényl)méthyl]-1H-imidazol-5-yl]méthyl]amino]-benzoïque, méthyl ester sont mis en suspension dans 80 ml de toluène anhydre. On ajoute 830 mg ($12,7.10^{-3}$ mole) d'azoture de sodium et 2,94 g ($14,7.10^{-3}$ mole) de chlorure de triméthylétain puis on porte à reflux pendant 48 heures. Après avoir refroidi à température ambiante on ajoute 1,19 g ($11,8.10^{-3}$ mole) de triéthylammine et 4,11 g ($14,7.10^{-3}$ mole) de chlorure de triphénylméthyle. On agite à la même température pendant 4 heures puis on ajoute de l'eau et extrait à l'acétate d'éthyle. Le résidu obtenu après lavage, séchage et évaporation est purifié par chromatographie en éluant par un mélange toluène/acétate d'éthyle (9/1 ; v/v). On obtient 5,6 g (rendement : 79 %) d'huile incolore.

RMN $^1$H (300 MHz; $CDCl_3$; ppm)

0,87(t,3H) ; 1,37(m,2H) ; 1,68(m,2H) ; 2,55(t,2H) ; 3,66(s,3H) ; 4,20(d,2H) ; 5,18(s,2H) ; 6,53(t,1H) ; 6,68(d,1H) ; 6,91(d,2H) ; 7,14-7,40(m,15H) ; 7,69-7,72(m,2H) ; 7,95(d,2H).

En opérant de façon analogue, on a préparé le produit de l'exemple 59 et le produit suivant :

**exemple 217 :**

**acide 2-[[[2-butyl-1-[(4-((triphénylméthyl)-1H-tétrazol-5-yl)phényl)méthyl]-1H-imidazol-5-yl]méthyl]amino]-benzoïque, pentyl ester**

RMN $^1$H (300 MHz; $CDCl_3$ ; ppm)

0,88 (m, 6H) ; 1,23 - 1,39 (m, 8H) ; 1,65 - 1,71 (m, 2H) ; 2,58 (t, 2H) ; 4,10 (d, 2H) ; 4,16 (t, 2H) ; 5,18 (s, 2H) ; 6,58 (t, 1H) ; 6,63 (d, 1H) ; 6,94 (d, 2H) ;7,02 (s, 1H) ; 7,13 - 7,38 (m, 16H) ; 7,75 (t, 1H) ; 7,82 (d, 1H) ; 8,01

(d, 2H).

**exemple 60 :**

**acide 2-[[[2-butyl-1-[(4-((((2-méthylphényl)sulfonyl)amino)carbonyl) phényl)méthyl]-1H-imidazol-5-yl]méthyl]amino]-benzoïque, méthyl ester**

A une suspension de 1,47 g ($3{,}49.10^{-3}$ mole) d'acide 2-[[[2-butyl-1-[(4-carboxyphényl)méthyl]-1H-imidazol-5-yl]méthyl]amino]-benzoïque, méthyl ester dans 50 ml de dichlorométhane on additionne 0,6 g ($3{,}49.10^{-3}$ mole) d'ortho-toluènesulfonamide, 0,67 g ($3{,}49.10^{-3}$ mole) de chlorhydrate de 1-(3-diméthylaminopropyl)-3-éthylcarbodiimide et 0,43 g ($3{,}49.10^{-3}$ mole) de diméthylaminopyridine. Après 20 heures sous agitation à température ambiante le solvant est évaporé sous pression réduite. Le résidu obtenu est purifié par chromatographie sur silice en éluant à l'aide d'un mélange toluène/isopropanol (80/20 ; v/v). On obtient ainsi 1,6 g (rendement : 80 %) de solide blanc.

**F = 135°C**

En opérant de façon analogue, on a préparé les produits des exemples 61, 64, 187 à 194, 227 et les produits suivants :

**exemple 62 :**

**acide 2-[[[2-butyl-1-[(4-((2,2-diméthyl-1,3-dioxolane-4-yl-méthoxy)carbonyl) phényl)méthyl]-1H-imidazol-5-yl]méthyl]amino]-benzoïque, phénylméthyl ester**

huile jaunâtre

RMN $^1H$ (300 MHz; $CDCl_3$ ; ppm)

0,88(t,3H) ; 1,34(m,2H) ; 1,37(s,3H) ; 1,44(s,3H) ; 1,70(m,2H) ; 2,56(t,2H) ; 3,83(m,1H) ; 4,10(m,1H) ; 4,19(d,2H) ; 4,32(m,2H) ; 4,41(m,1H) ; 5,18(s,2H) ; 5,22(s,2H) ; 6,64(m,2H) ; 6,91(d,2H) ; 7,05(s,1H) ; 7,28-7,41(m,6H) ; 7,7(t, 1H) ; 7,92(m,3H).

**exemple 63 :**

**acide 2-[[[2-butyl-1-[(4-((2-(morpholin-1-yl)éthoxy)carbonyl)phényl), méthyl]-1H-imidazol-5-yl]méthyl]amino]-benzoïque, 2-(morpholin-1-yl)éthyl ester**

huile incolore

RMN $^1H$ (300 MHz; $CDCl_3$ ; ppm)

0,87(t,3H) ; 1,34(m,2H) ; 1,69(m,2H) ; 2,56(m,10H) ; 2,73(m,4H) ; 3,71(m,8H) ; 4,19(d,2H) ; 4,32(t,2H) ; 4,44(t,2H) ; 5,19(s,2H); 6,64(q,2H) ; 6,95(d,2H) ; 7,04(s,1H) ; 7,31 (m,1H) ; 7,70(t,1H) ; 7,82(d, 1H) ; 7,92(d,2H).

**exemple 65 :**

**acide 2-[[[2-butyl-1-[(4-(2,2-diméthyl-1,3-dioxolane-4-yl-méthoxycarbonyl) phényl)méthyl]-1H-imidazol-5-yl]méthyl]amino]-benzoïque, 2,2-diméthyl-1,3-dioxolane-4-yl-méthyl ester**

RMN $^1H$ (300 MHz; $CDCl_3$ ; ppm)

0,88(t,3H) ; 1,29(m,2H) ; 1,38(s,6H) ; 1,41(s,6H) ; 1,69(m,2H) ; 2,56(t,2H) ; 3,85(m,2H) ; 4,10-4,25(m,6H); 4,35-4,47(m,4H) ; 5,19(s,2H) ; 8,61(m,2H) ; 6,95(d,2H); 7,04(s, 1H) ; 7,34(m,1H) ; 7;65(t,1H) ; 7,86(d,1H)) ; 7,95(d,2H).

exemple 68 :

**acide 2-[[[2-butyl-1-[(4-((((diéthylamino)carbonyl)méthoxy)carbonyl) phényl)méthyl]-1H-imidazol-5-yl]méthyl]amino]-benzoïque, ((diéthylamino)carbonyl)méthyl ester**

A une suspension de 2 g ($4{,}9.10^{-3}$ mole) d'acide 2-[[[2-butyl-1-[(4-carboxyphényl)méthyl]-1H-imidazol-5-yl]méthyl]amino]-benzoïque dans 5 ml de diméthylformamide on ajoute 1,09 g ($10{,}8.10^{-3}$ mole) de triéthylamine, 147 mg ($10^{-3}$ mole) d'iodure de sodium et 1,46 g ($9{,}8.10^{-3}$ mole) de N,N-diéthyl chloroacétamide. Le mélange est chauffé à 90°C pendant 2 heures. Après refroidissement on ajoute de l'eau et on extrait à l'acétate d'éthyle. Les phases organiques lavées à l'eau sont séchées sur sulfate de magnésium et concentrées. On purifie le produit brut obtenu par chromatographie sur silice en éluant par le mélange toluène/alcool isopropylique (9/1 ; v/v). Après évaporation, on obtient 1,1 g (rendement : 35 %) du produit attendu.

**F = 55°C**

En opérant de façon analogue, on a préparé les produits des exemples 180 à 183 et les produits suivants :

**exemple 69 :**

**acide 2-[[[2-butyl-1-[(4-(((((1,1-diméthyléthyl)carbonyl)oxy)méthoxy) carbonyl)phényl)méthyl]-1H-imidazol-5-yl]méthyl]amino]-benzoïque, (((1,1-diméthyléthyl)carbonyl)oxy)méthyl ester**

huile jaune

RMN $^1H$ (300 MHz; $CDCl_3$ ; ppm)

0,88(t,3H) ; 1,21(s,9H) ; 1,22(s,9H) ; 1,37(m,2H) ; 1,70(m,2H) ; 2,56(t,2H) ; 4,19(d,2H) ; 5,19(s,2H) ; 5,88(s,2H) ; 5,98(s,2H) ; 6,64(m,2H) ; 6,95(d,2H) ; 7,05(s,1H); 7,35(t,1H) ; 7,60(t,1H) ; 7,86(d,1H) ; 7,95(d,2H).

**exemple 70 :**

**acide 2-[[[2-butyl-1-[(4-((((4-méthylpipérazin-1-yl)carbonyl)methoxy) carbonyl)phényl)méthyl]-1H-imidazol-5-yl]méthyl]amino]-benzoïque, ((4-méthylpipérazin-1-yl)carbonyl)méthyl ester**

RMN $^1H$ (300 MHz; $CDCl_3$ ; ppm)

0,87(t,3H) ; 1,26(m,2H) ; 1,70(m,2H) ; 2,33(s,6H) ; 2,43(m,8H) ; 2,54(t,2H) ; 3,46(m,4H) ; 3,63(m,4H) ; 4,29(d,2H) ; 4,86(s,2H) ; 4,95(s,2H); 5,18(s,2H) ; 6,89(d,2H) ; 7,11(s,1H) ; 7,39(d,1H) ; 7,51(s,1H); 7,82(t,1H) ; 7,96(d,2H) ; 8,06(d,1H).

**exemple 184 :**

**acide butanoïque,[2-[[[2-butyl-1-[(4-(phénylméthoxycarbonyl)phényl) méthyl]-1H-imidazol-5-yl]méthyl]amino]phénylcarbonyloxy]méthyl ester**

RMN $^1H$ (300 MHz; $CDCl_3$ ; ppm)

0,87 (m, 6H) ; 1,31 (m, 2H) ; 1,67 (m, 4H) ; 2,33 (t, 2H) ; 2,59 (t, 2H) ; 4,19 (d, 2H) ; 5,18 (s, 2H) ; 5,35 (s, 2H) ; 5,88 (s, 2H) ; 6,59 (m, 2H) ; 6,90 (d, 2H) ; 7,04 (s, 1H) ; 7,30 - 7,50 (m, 6H) ; 7,59 (t, 1H) ; 7,82 - 7,96 (m, 3H).

**exemple 185 :**

**acide 2-[[[2-butyl-1-[(4-((((propylcarbonyl)oxy)méthoxy)carbonyl)phényl) méthyl]-1H-imidazol-5-yl]méthyl]amino]-benzoïque, ((propylcarbonyl) oxy)méthyl ester**

RMN $^1H$ (300 MHz; $CDCl_3$ ; ppm)

0,93 (m, 9H) ; 1,34 (m, 2H) ; 1,63 (m, 6H) ; 2,36 (m, 4H) ; 2,56 (t, 2H) ; 4,19 (d, 2H) 5,18 (s, 2H) ; 5,88 (s, 2H) ; 5,96 (s, 2H) 6, 61 (m, 2H) ; 6,92 (d, 2H) ; 7,04 (s, 1H) ; 7,35 (m, 1H) ; 7,58 (t, 1H) ; 7,86 (m, 1H) ; 7,95 (d, 2H).

**exemple 186 :**

**acide 2-[[[2-[[[2-butyl-1-[(4-(méthoxycarbonyl)phényl)méthyl]]-1H-imidazol-5-yl]méthyl]amino]phényl]carbonyloxy]-acétique, éthyl ester**

RMN $^1H$ (300 MHz; $CDCl_3$ ; ppm)

0,85 (t, 3H) ; 1,29 (t, 3H) ; 1,36 (m, 2H) ; 1,69 (m, 2H) ; 2,56 (t, 2H) ; 3,91 (s, 3H) ; 4,17 (d, 2H) ; 4,26 (m, 2H) ; 4,71 (s, 2H) ; 5,17 (s, 2H) ; 6,65 (m, 2H) ; 6,94 (d, 2H) ; 7,04 (s, 1H) ; 7,33 (m, 1H) ; 7,53 (t, 1H) , 7,95 (m, 3H).

**exemple 72 :**

**tri-chlorhydrate de l'acide 2-[[[2-butyl-1-[(4-((((4-méthylpipérazin-1-yl)carbonyl)méthoxy)carbonyl)phényl)méthyl]-1H-imidazol-5-yl]méthyl] amino]-4-nitro-benzoïque, ((4-méthylpipérazin-1-yl)carbonyl)méthyl ester**

1,4 g ( $1,9.10^{-3}$ mole) d'acide 2-[[[2-butyl-1-[(4-((((4-méthylpipérazin-1-yl)carbonyl)méthoxy)carbonyl)phényl)méthyl]-1H-imidazol-5-yl]méthyl]amino]-4-nitro-benzoïque, ((4-méthylpipérazin-1-yl)carbonyl)méthyl ester sont dissous dans un mélange de 25 ml d'acétate d'éthyle et de 10 ml de chlorure de méthylène. On ajoute un excès d'éther éthylique saturé par du chlorure d'hydrogène gazeux. Une gomme jaune précipite. Après décantation, on lave par l'éther éthylique et on sèche. On obtient 1,4 g (rendement : 87 %) d'une poudre jaune.

**F = 194°C**

En opérant de façon analogue, on a préparé le produit de l'exemple 213.

**exemple 73 :**

**tri-oxalate de l'acide 2-[[[2-butyl-1-[(4-((2-(morpholin-1-yl)éthoxy) carbonyl)phényl)méthyl]-1H-imidazol-5-yl]méthyl]amino]-benzoïque, 2-(morpholin-1-yl)éthyl ester**

A une solution de 0,71 g ($1,12.10^{-3}$ mole) de l'acide 2-[[[2-butyl-1-[(4-((2-(morpholin-1-yl)éthoxy)carbonyl)phényl)méthyl]-1H-imidazol-5-yl]méthyl]amino]-benzoïque,2-(morpholin-1-yl)éthyl ester dans 15 ml d'acétate d'éthyle, on additionne à température ambiante, 0,302 g ($3,36.10^{-3}$ mole) d'acide oxalique dissous dans un mélange de 1 ml de méthanol et 5 ml d'acétate d'éthyle. Le mélange réactionnel est agité pendant 1 heure et le précipité formé est filtré et séché sous vide. Le solide obtenu est dissous dans 20 ml d'eau et lyophilisé. On obtient 0,7 g (rendement : 69 %) de mousse jaunâtre.

**F = 102°C**

**exemple 214 :**

**acide 2-[[[2-butyl-1-[(4-(pentoxycarbonyl)phényl)méthyl]-1H-imidazol-5-yl]méthyl]amino]-benzoïque, pentyl ester**

A une suspension de 1,07 g (0,0026 mole) de l'acide 2-[[[2-butyl-1-[(4-carboxyphényl)méthyl]-1H-imidazol-5-yl]méthyl]amino]-benzoïque dans 25 ml de dichlorométhane on additionne 6.50 mg (0,052 mole) de 4-diméthylaminopyridine, 1 g (0,0.52 mole) de chlorhydrate de 1-(3-diméthylaminopropyl)-3-éthylcarbodiimide et 458 mg (0,052 mole) de n-pentanol. Le mélange réactionnel est agité à température ambiante pendant 24 h puis concentré sous pression réduite. Le résidu est purifié par flash chromatographie sur silice en éluant par un mélange méthylcyclohexane / acétone (85/15 ; v/v). On obtient 1,2 g d'huile jaunâtre (rendement : 83 %)

RMN $^1H$ (300 MHz; $CDCl_3$ ; ppm)

0,92 (m, 9H) ; 1,37 (m, 10H) ; 1,67 (m , 6H) ; 2,55 (t, 2H) ; 4,18 (m, 4H) ; 4,29 (t, 2H) ; 5,19 (s, 2H) ; 6,61 (q, 2H) ; 6,93 (d, 2H) ; 7,04 (s, 1H) ; 7,30 (m, 1H) ; 7,83(t, 1H) ; 7,73 - 7,93(m, 3H).

**exemple 216 :**

**Fumarate de l'acide 4-[[2-butyl-5-[((2-(((3-(diméthylamino)propyl)amino) carbonyl)phényl)amino)méthyl]-1H-imidazol-1-yl]méthyl]-benzoïque méthyl ester**

On dissout 0,96 g (1,9 $10^{-3}$ mole) de l'acide 4-[[2-butyl-5-[((2-(((3-(diméthylamino)propyl)amino)carbonyl)phényl)amino)méthyl]-1H-imidazol-1-yl]méthyl]-benzoïque, méthyl ester dans 30 ml d'acétate d'éthyle. On chauffe à 50°C et ajoute une solution de 0,214 g (1,85 10-3 mole) d'acide fumarique dans 4 ml de méthanol. Après avoir refroidi à 15°C pendant 1 heure on filtre le précipité obtenu. Après séchage, on obtient 1 g (rendement : 87 %) du produit attendu.

**F = 164°C**

On a regroupé dans les tableaux I à VII suivants un certain nombre de composés selon l'invention. Dans ces tableaux les symboles utilisés ont les significations suivantes :

Et = $-C_2H_5$

n-Pr = $-CH_2-CH_2-CH_3$

i-Pr = $-CH(CH_3)_2$
c-Pr = cyclopropyl
n-Bu = $-CH_2-CH_2-CH_2-CH_3$
s-Bu = $-CH(CH_3)-CH_2-CH_3$
i-Bu = $-CH_2-CH(CH_3)_2$
t-Bu = $-C(CH_3)_3$
n-Pent = $-(CH_2)_4-CH_3$
i-Pent = $-CH_2-CH_2-CH(CH_3)_2$
c-Pent = cyclopentyl
n-Hex = $-(CH_2)_5-CH_3$
c-Hex = cyclohexyl
n-Hep = $-(CH_2)_6-CH_3$
n-Dec = $-(CH_2)_9-CH_3$
n-Cet = $-(CH_2)_{15}-CH_3$
Mcs = $-CH_2-CH_2-O-CH_3$
Deae = $-CH_2-CH_2-N(C_2H_5)_2$
Gl = $-CH_2-CH(OH)-CH_2OH$

Ig = 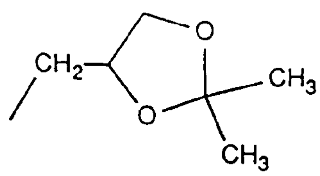

Ph = phényl
Bn = benzyl

Eph = 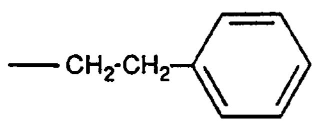

TT = 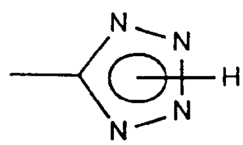

TTT = 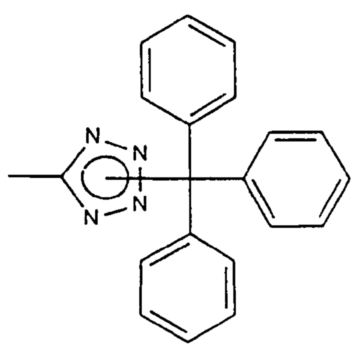

MOE = 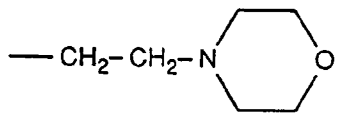

NAE = 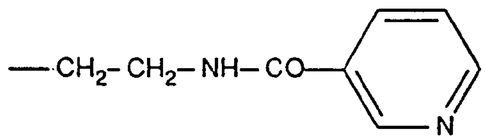

Pz = 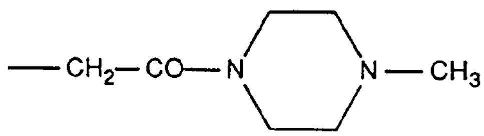

PhSA = 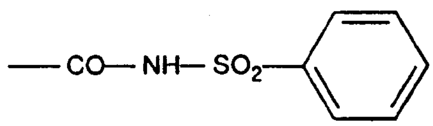

TSA = 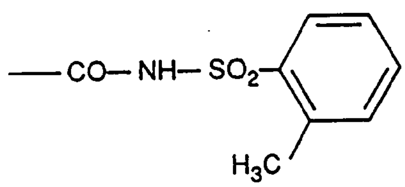

OCSA = 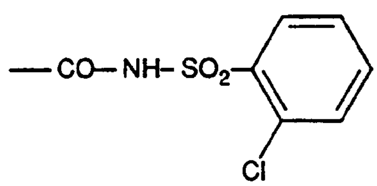

MCSA = 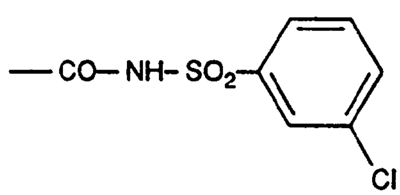

PCSA = 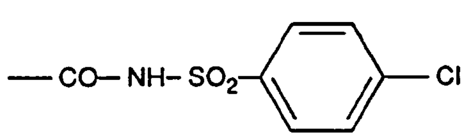

PASA = 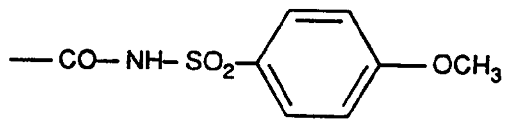

OASA = 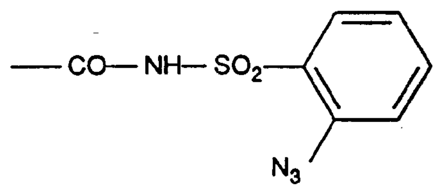

MESA = $-CO-NH-SO_2-CH_3$
AAE = $-CH_2-CO-N(CH_2-CH_3)_2$
AAP = $-CH_2-CO-N(CH_2-CH_2-CH_3)_2$
AAHE = $-CH_2-CO-N(CH_2-CH_2OH)_2$
AAMHE = $-CH_2-CO-N(CH_3)(CH_2-CH_2OH)$
APE = $-CH(CH_3)-CO-N(CH_2-CH_3)_2$
W = $-CH_2-CO_2-$
X = $-CH(CH_3)-CO_2-$
Y = $-CH_2-O-CO-$
Z = $-CH(CH_3)-O-CO-$
Gly = $-NH-CH_2-CO-$
L-Val = $-NH-CH[CH(CH_3)_2]-CO-$ (L)

TABLEAU A

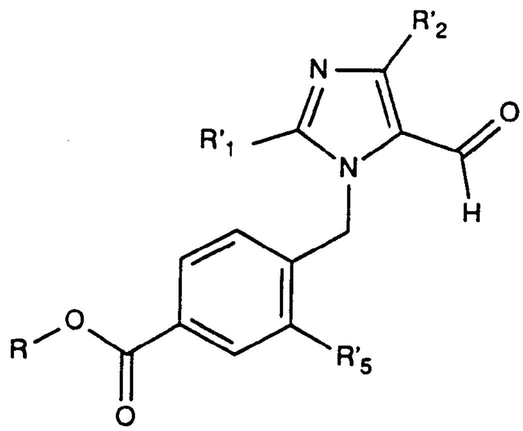

| Prép | R'$_1$ | R'$_2$ | R'$_5$ | R | F(°C) |
|---|---|---|---|---|---|
| 2 | n-Pr | Cl | H | $CH_3$ | 89 |
| 3 | n-Pr | H | H | $CH_3$ | 72 |
| 4 | n-Bu | $CF_3$ | H | $CH_3$ | 59 |
| 5 | n-Bu | H | H | H | 148 |
| 6 | n-Bu | H | H | Bn | - |
| 9 | n-Bu | Cl | H | t-Bu | 54 |
| 10 | n-Bu | Cl | Cl | $CH_3$ | 112 |
| 11 | n-Bu | I | H | $CH_3$ | 82 |
| 12 | n-Bu | H | H | t-Bu | - |
| 31 | n-Bu | Cl | H | Et | 60 |
| 32 | n-Bu | H | H | Et | - |
| 33 | n-Bu | Cl | H | H | 126 |
| 34 | n-Bu | H | H | n-Cet | 56 |
| 35 | n-Bu | H | H | n-Pent | - |
| 36 | n-Bu | H | H | n-Bu | - |
| 37 | n-Bu | H | H | i-Bu | - |
| 38 | n-Bu | H | H | $CH_2$-c-Pr | - |
| 39 | n-Bu | H | H | i-Pent | - |
| 40 | n-Bu | Cl | H | Bn | - |
| 62 | n-Bu | S-$CH_3$ | H | CH3 | 72-74 |

TABLEAU B

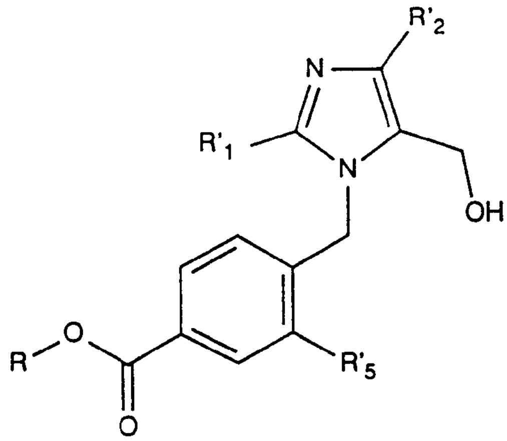

| Prép | R'$_1$ | R'$_2$ | R'$_5$ | R | F(°C) |
|---|---|---|---|---|---|
| 7 | n-Bu | H | H | $CH_3$ | 144 |
| 13 | n-Bu | $CF_3$ | H | $CH_3$ | 113 |
| 14 | n-Bu | Cl | Cl | $CH_3$ | 108 |
| 15 | n-Pr | Cl | H | $CH_3$ | 120 |
| 16 | n-Bu | H | H | t-Bu | 163 |
| 17 | n-Bu | H | H | Bn | 111 |
| 18 | n-Bu | I | H | $CH_3$ | 150 |
| 19 | n-Bu | Cl | H | t-Bu | 174 |
| 20 | n-Bu | Cl | H | $CH_3$ | 94 |
| 21 | n-Pr | H | H | $CH_3$ | 135 |
| 41 | n-Bu | S-$CH_3$ | H | $CH_3$ | 154 |
| 42 | n-Bu | H | H | n-Pent | 137 |
| 43 | n-Bu | H | H | Et | 140 |
| 44 | n-Bu | H | H | n-Bu | 140 |
| 45 | n-Bu | H | H | n-Cet | 75 |
| 46 | n-Bu | H | H | i-Bu | 144 |
| 47 | n-Bu | H | H | $CH_2$-c-Pr | 100 |
| 48 | n-Bu | H | H | i-Pent | 170 |
| 49 | n-Bu | Cl | H | Bn | 127 |

## TABLEAU C

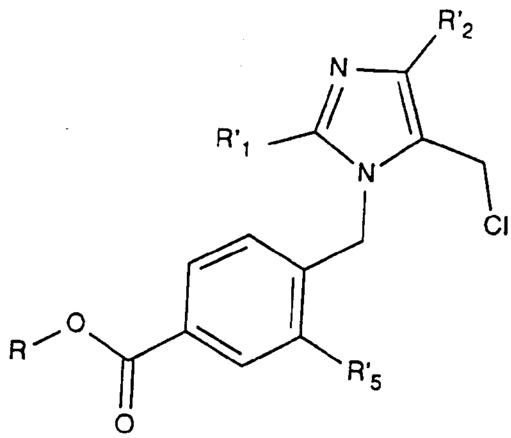

| Prép | R'1 | R'2 | R'5 | R | F(°C) |
|---|---|---|---|---|---|
| 8* | n-Bu | H | H | $CH_3$ | 158 |
| 22 | n-Pr | Cl | H | $CH_3$ | 128 |
| 23 | n-Bu | H | H | Bn | 160 |
| 24 | n-Bu | H | H | t-Bu | 150-191 |
| 25 | n-Bu | Cl | Cl | $CH_3$ | 70 |
| 26 | n-Bu | I | H | $CH_3$ | 140 |
| 27* | n-Bu | Cl | H | t-Bu | 133 |
| 28* | n-Bu | Cl | H | $CH_3$ | 120 |
| 29* | n-Pr | H | H | $CH_3$ | 172 |
| 30 | n-Bu | $CF_3$ | H | $CH_3$ | - |
| 51* | n-Bu | $S-CH_3$ | H | $CH_3$ | 115 |
| 53* | n-Bu | H | H | n-Pent | 130 |
| 54* | n-Bu | H | H | Et | 130 |
| 55* | n-Bu | H | H | n-Bu | 130 |
| 56* | n-Bu | H | H | n-Cet | 135 |
| 57* | n-Bu | H | H | i-Bu | 148 |
| 58* | n-Bu | H | H | $CH_2$-c-Pr | 150 |
| 59* | n-Bu | H | H | i-Pent | 135 |
| 61 | n-Bu | Cl | H | Bn | - |

Note : * chlorhydrates

TABLEAU D

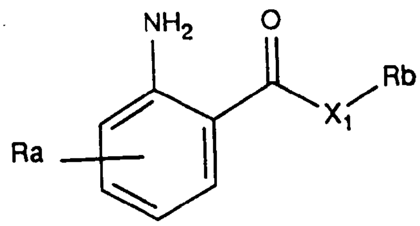

| Prép | $R_a$ | $X_1$ | $R_b$ | F(°C) |
|---|---|---|---|---|
| 64 | H | O | AAP | 64 |
| 65 | H | O | $CH(CH_3)(CO)Et$ | - |
| 66 | H | O | W-Et | - |
| 67 | H | O | W-n-Pent | - |
| 68 | H | O | $CH_2$-c-Pr | - |
| 69 | H | O | $CH_2$-CO-Et | 53 |
| 70 | H | O | $CH(CH_3)$-n-Bu | - |
| 71 | H | O | Deae | - |
| 72 | H | O | APE | 134 |
| 73 | H | O | AAHE | 107 |
| 74 | H | O | AAMHE | 103 |
| 75 | H | O | Z-t-Bu | 80 |
| 76 | H | O | $Z-CH(Et)_2$ | - |
| 77 | H | O | Z-c-Pent | - |
| 78 | H | O | Z-c-Hex | - |
| 79 | H | O | Y-n-Pent | - |
| 80 | H | O | Z-n-Pent | - |
| 81 | H | O | Z-$CH_2$-c-Hex | - |
| 82 | H | O | Z-$CH_2$-c-Pent | - |
| 83 | H | O | Y-t-Bu | - |
| 84 | H | NH | $(CH_2)_3-N(CH_3)_2$ | 76 |
| 85 | H | NH | AAE | 62 |
| 86 | H | NH | CH(iPr)-$CO_2$Et | - |
| 87 | 6-$CH_3$ | O | n-Pent | - |
| 88 | 6-Cl | O | n-Pent | - |

TABLEAU I

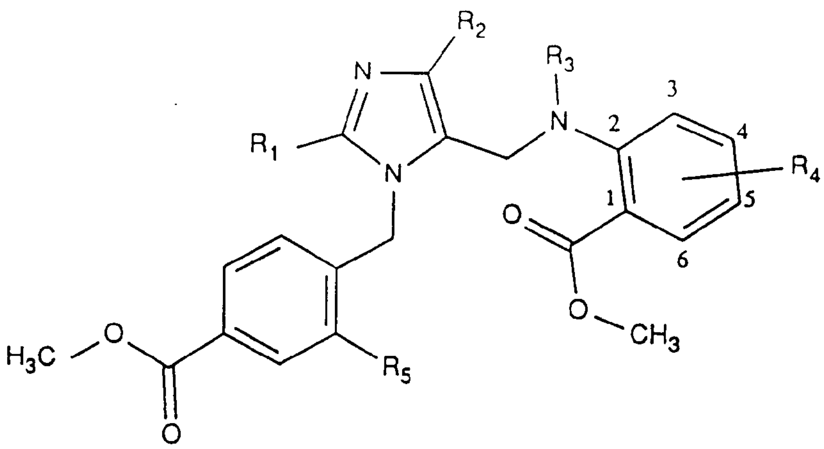

| Ex | $R_1$ | $R_2$ | $R_3$ | $R_4$ | $R_5$ | F(°C) |
|---|---|---|---|---|---|---|
| 1 | n-Bu | H | H | H | H | - |
| 2 | n-Bu | H | H | 4-$NO_2$ | H | 144 |
| 3 | n-Pr | H | H | H | H | - |
| 6 | n-Bu | Cl | H | 3,5-diCl | H | - |
| 7 | n-Bu | Cl | H | 3-$CH_3$ | H | - |
| 8 | n-Bu | Cl | $CH_3$ | H | H | - |
| 9 | n-Bu | Cl | H | 3,4,5-tri $OCH_3$ | H | - |
| 10 | n-Bu | Cl | H | H | H | - |
| 13 | n-Pr | Cl | H | H | H | 108 |
| 14 | n-Bu | Cl | H | 5-Cl | H | 90 |
| 15 | n-Bu | Cl | H | 4-Cl | H | 116 |
| 17 | n-Bu | Cl | H | 4-$NO_2$ | H | 136 |
| 18 | n-Bu | Cl | H | 5-$CH_3$ | H | * |
| 19 | n-Bu | I | H | H | H | - |
| 20 | n-Bu | $CF_3$ | H | H | H | - |
| 21 | n-Bu | Cl | H | H | Cl | 140 |
| 54 | n-Bu | Cl | $COCH_3$ | H | H | 142 |
| 75 | n-Bu | $SCH_3$ | H | H | H | - |
| 116 | n-Pr | H | H | 4-$NO_2$ | H | - |
| 225 | n-Bu | H | H | 4-$N_3$ | H | 132 |

Note : * double point de fusion : 87°C puis 97°C

TABLEAU II

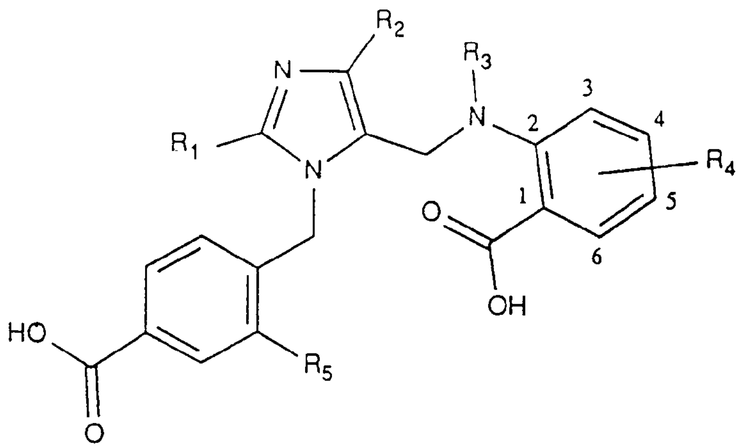

| Ex | $R_1$ | $R_2$ | $R_3$ | $R_4$ | $R_5$ | F(°C) |
|---|---|---|---|---|---|---|
| 32 | n-Bu | H | H | H | H | 234 |
| 35 | n-Bu | H | H | 4-$NO_2$ | H | 250 |
| 36 | n-Pr | H | H | H | H | 249 |
| 37 | n-Bu | Cl | H | 3-$CH_3$ | H | 115 |
| 38 | n-Bu | Cl | $CH_3$ | H | H | 147 |
| 39 | n-Bu | Cl | H | 3,5-diCl | H | 220 |
| 40 | n-Bu | Cl | H | 3,4,5-tri $OCH_3$ | H | 212 |
| 41 | n-Bu | Cl | H | H | Cl | 244 |
| 42 | n-Pr | Cl | H | H | H | 246 |
| 43 | n-Bu | $CF_3$ | H | H | H | 262 |
| 44 | n-Bu | I | H | H | H | 225 |
| 45 | n-Bu | Cl | H | 5-$CH_3$ | H | 232 |
| 46 | n-Bu | Cl | H | 4-$NO_2$ | H | 260 |
| 48 | n-Bu | Cl | H | 4-Cl | H | 247 |
| 49 | n-Bu | Cl | H | 5-Cl | H | 248 |
| 50 | n-Bu | Cl | H | H | H | 235 |
| 55 | n-Bu | Cl | $COCH_3$ | H | H | 230 |
| 198 | n-Bu | $SCH_3$ | H | H | H | 207 |
| 199 | n-Bu | H | H | 6-$CH_3$ | H | 225 |
| 200 | n-Bu | H | H | 6-Cl | H | 259 |
| 212 | n-Pr | H | H | 4-$NO_2$ | H | 281 |
| 226 | n-Bu | H | H | 4-$N_3$ | H | 215(dec) |

TABLEAU III

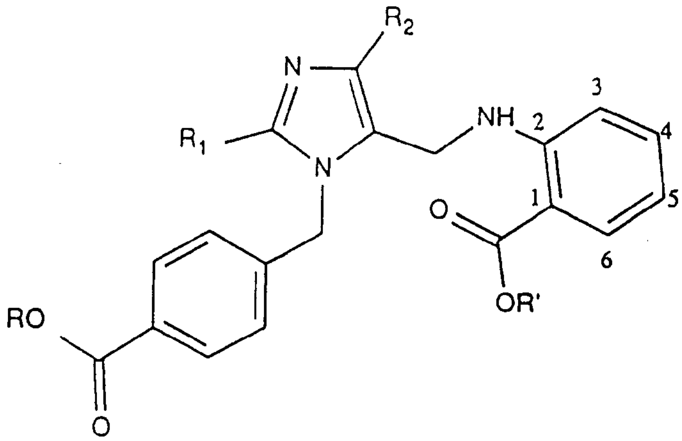

| Ex | $R_1$ | $R_2$ | R | R' | F(°C) |
|---|---|---|---|---|---|
| 4 | n-Bu | H | Bn | Ig | - |
| 5 | n-Bu | H | t-Bu | Bn | - |
| 22 | n-Bu | Cl | t-Bu | $CH_3$ | 102 |
| 23 | n-Bu | Cl | H | $CH_3$ | 181 |
| 24 | n-Bu | H | H | $CH_3$ | 85 |
| 25 | n-Bu | H | H | Bn | 90 |
| 26 | n-Bu | H | H | Ig | 92 |
| 27 | n-Bu | H | Ig | H | 202 |
| 28 | n-Bu | H | H | Gl | 123 |
| 29 | n-Bu | H | Gl | H | 134 |
| 53 | n-Bu | H | K | K | 206 |
| 62 | n-Bu | H | Ig | Bn | - |
| 63 | n-Bu | H | MOE | MOE | - |
| 64 | n-Bu | H | NAE | NAE | 84 |
| 65 | n-Bu | H | Ig | Ig | - |
| 68 | n-Bu | H | AAE | AAE | 55 |
| 69 | n-Bu | H | Y-t-Bu | Y-t-Bu | - |
| 70 | n-Bu | H | Pz | Pz | - |
| 71 | n-Bu | H | Gl | Gl | 60 |
| 73** | n-Bu | H | MOE | MOE | 102 |

TABLEAU III (suite 1)

| Ex | $R_1$ | $R_2$ | R | R' | F(°C) |
|---|---|---|---|---|---|
| 74 | n-Bu | Cl | Bn | n-Pent | - |
| 76 | n-Bu | H | $CH_3$ | AAE | 81 |
| 77 | n-Bu | H | $CH_3$ | AAP | - |
| 78 | n-Bu | H | Bn | AAP | - |
| 79 | n-Bu | H | Bn | AAE | - |
| 80 | n-Bu | H | Bn | APE | - |
| 81 | n-Bu | H | Bn | AAHE | - |
| 82 | n-Bu | H | Bn | AAMHE | - |
| 84 | n-Bu | H | Bn | W-Et | - |
| 85 | n-Bu | H | Bn | Z-CH-$Et_2$ | - |
| 86 | n-Bu | H | Bn | Z-c-Pent | - |
| 90 | n-Bu | H | Bn | Z-$CH_2$-c-Pent | - |
| 91 | n-Bu | H | Bn | Z-c-Hex | - |
| 92 | n-Bu | H | Bn | Z-t-Bu | - |
| 93 | n-Bu | H | Bn | Y-t-Bu | - |
| 94 | n-Bu | H | Bn | Y-n-Pent | - |
| 95 | n-Bu | H | Bn | Deae | - |
| 96 | n-Bu | H | Bn | $CH(CH_3)$-n-Bu | - |
| 97 | n-Bu | H | Bn | $CH(CH_3)$-CO-Et | - |
| 98 | n-Bu | H | $CH_3$ | $CH_2$-CO-Et | 100 |
| 99 | n-Bu | H | Bn | $CH_2$-CO-Et | - |
| 100 | n-Bu | H | Bn | X-Et | - |
| 101 | n-Bu | H | Bn | W-n-Pent | - |
| 102 | n-Bu | H | Bn | EPh | - |
| 103 | n-Bu | H | Bn | Ph | - |
| 104 | n-Bu | H | Bn | Mcs | - |
| 105 | n-Bu | H | Bn | n-Dec | - |
| 106 | n-Bu | H | Bn | n-Hep | - |
| 107 | n-Bu | H | Bn | i-Pent | - |
| 108 | n-Bu | H | Bn | i-Pr | - |
| 109 | n-Bu | H | Bn | $CH_2$-c-Pr | - |
| 110 | n-Bu | H | Bn | i-Bu | - |
| 111 | n-Bu | H | Bn | n-Cet | - |
| 112 | n-Bu | H | Bn | n-Bu | - |
| 113 | n-Bu | H | Et | Et | - |
| 114 | n-Bu | H | $CH_3$ | n-Pent | - |
| 115 | n-Pr | H | Bn | n-Pent | - |
| 118 | n-Bu | H | Bn | Z-n-Pent | - |
| 119 | n-Bu | H | Bn | t-Bu | - |

## TABLEAU III (suite 2)

| Ex | $R_1$ | $R_2$ | R | R' | F(°C) |
|---|---|---|---|---|---|
| 120 | n-Bu | H | Bn | Et | - |
| 121 | n-Bu | H | $CH_3$ | Z-n-Pent | - |
| 122 | n-Bu | H | Bn | n-Pent | - |
| 123 | n-Bu | H | Bn | Z-$CH_2$-c-Hex | - |
| 124 | n-Bu | H | n-Pent | Bn | - |
| 125 | n-Bu | H | $CH_3$ | Bn | - |
| 126 | n-Bu | H | Et | Bn | - |
| 127 | n-Bu | H | n-Bu | Bn | - |
| 128 | n-Bu | H | n-Cet | Bn | - |
| 129 | n-Bu | H | i-Bu | Bn | - |
| 130 | n-Bu | H | -$CH_2$-c-Pr | Bn | - |
| 131 | n-Bu | H | i-Pent | Bn | - |
| 134 | n-Bu | H | H | AAP | 140 |
| 135 | n-Bu | H | H | AAE | 168 |
| 136 | n-Bu | H | H | APE | 135 |
| 137 | n-Bu | H | H | AAHE | 108 |
| 138 | n-Bu | H | H | AAMHE | 110 |
| 139 | n-Bu | H | H | Z-CH-$Et_2$ | 170 |
| 140 | n-Bu | H | H | Z-c-Pent | 170 |
| 141 | n-Bu | H | H | W-Et | 155 |
| 144 | n-Bu | H | H | Z-$CH_2$-c-Pent | 154 |
| 145 | n-Bu | H | H | Z-c-Hex | 60 |
| 146 | n-Bu | H | H | Z-t-Bu | 90 |
| 147 | n-Bu | H | H | Y-t-Bu | 160 |
| 148 | n-Bu | H | H | Y-n-Pent | 140 |
| 149 | n-Bu | H | H | Y-n-Pr | 162 |
| 150 | n-Bu | H | H | Deae | 68 |
| 151 | n-Bu | H | H | $CH(CH_3)$-n-Bu | 74 |
| 152 | n-Bu | H | H | $CH(CH_3)$-CO-Et | 80 |
| 153 | n-Bu | H | H | X-Et | 164 |
| 154 | n-Bu | H | H | W-n-Pent | 157 |
| 155 | n-Bu | H | H | $CH_2$-CO-Et | 144 |
| 156 | n-Bu | H | H | EPh | 128 |
| 157 | n-Bu | H | H | Ph | 231 |
| 158 | n-Bu | H | H | Mcs | 78 |
| 159 | n-Bu | H | H | n-Dec | 50 |

## TABLEAU III (Fin)

| Ex | $R_1$ | $R_2$ | R | R' | F(°C) |
|---|---|---|---|---|---|
| 160 | n-Bu | H | H | n-Hep | 96 |
| 161 | n-Bu | H | H | i-Pent | 164 |
| 162 | n-Bu | H | H | i-Pr | 172 |
| 163 | n-Bu | H | H | $CH_2$-c-Pr | 171 |
| 164 | n-Bu | H | H | i-Bu | 163 |
| 165 | n-Bu | H | H | n-Cet | 82 |
| 166 | n-Bu | H | H | n-Bu | 151 |
| 167 | n-Pr | H | H | n-Pent | 177 |
| 168 | n-Bu | H | H | Z-n-Pent | 143 |
| 169 | n-Bu | H | H | Et | 173 |
| 170 | n-Bu | H | H | n-Pent | 161 |
| 171 | n-Bu | H | H | Z-$CH_2$-c-Hex | 114 |
| 172 | n-Bu | H | n-Pent | H | 202 |
| 173 | n-Bu | H | $CH_3$ | H | 188 |
| 174 | n-Bu | H | Et | H | 201 |
| 175 | n-Bu | H | n-Bu | H | 194 |
| 176 | n-Bu | H | n-Cet | H | 152 |
| 177 | n-Bu | H | i-Bu | H | 190 |
| 178 | n-Bu | H | $CH_2$-c-Pr | H | 198 |
| 179 | n-Bu | H | i-Pent | H | 197 |
| 181 | n-Bu | H | AAP | AAP | 97 |
| 182* | n-Bu | H | Pz | Pz | 250-260 |
| 184 | n-Bu | H | Bn | Y-n-Pr | - |
| 185 | n-Bu | H | Y-n-Pr | Y-n-Pr | - |
| 186 | n-Bu | H | $CH_3$ | W-Et | - |
| 196 | n-Bu | H | Bn | H | 175 |
| 213*** | n-Bu | H | H | Deae | 206 |
| 214 | n-Bu | H | n-Pent | n-Pent | - |
| 224 | n-Bu | Cl | H | n-Pent | 145 |

Notes : * : 3 HCl
** : 3 $HO_2C$-$CO_2H$
*** : 2HCl

## TABLEAU IV

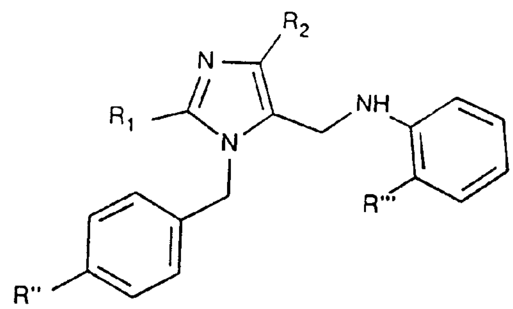

| Ex | $R_1$ | $R_2$ | R" | R"' | F(°C) |
|---|---|---|---|---|---|
| 30 | n-Bu | Cl | TT | $CO_2CH_3$ | 185 |
| 31 | n-Bu | Cl | $CO_2CH_3$ | TT | 182 |
| 51 | n-Bu | Cl | TT | $CO_2H$ | 158 |
| 52 | n-Bu | Cl | $CO_2H$ | TT | 200 |
| 56 | n-Bu | Cl | CN | $CO_2CH_3$ | 126 |
| 57 | n-Bu | Cl | $CO_2CH_3$ | CN | - |
| 58 | n-Bu | Cl | TTT | $CO_2CH_3$ | - |
| 59 | n-Bu | Cl | $CO_2CH_3$ | TTT | 130 |
| 60 | n-Bu | H | TSA | $CO_2CH_3$ | 135 |
| 61 | n-Bu | Cl | TSA | $CO_2CH_3$ | 244 |
| 66 | n-Bu | Cl | TSA | $CO_2H$ | 234 |
| 67 | n-Bu | H | TSA | $CO_2H$ | 190 |
| 88 | n-Bu | H | $CO_2CH_3$ | CONH-AAE | 42 |
| 89 | n-Bu | H | $CO_2CH_3$ | $CONHOCH_3$ | 146 |
| 132 | n-Bu | H | 4-CN | $CO_2$-n-Pent | - |
| 142 | n-Bu | H | $CO_2H$ | CO-L-Val-OEt | 110 |
| 143 | n-Bu | H | $CO_2H$ | CO-Gly-OEt | 140 |
| 187 | n-Bu | H | TSA | $CO_2$-Y-n-Pr | 228 |
| 188 | n-Bu | H | OCSA | $CO_2CH_3$ | 125 |
| 189 | n-Bu | H | MCSA | $CO_2CH_3$ | 145 |
| 190 | n-Bu | H | PCSA | $CO_2CH_3$ | 220 |
| 191 | n-Bu | H | PhSA | $CO_2CH_3$ | 120 |
| 192 | n-Bu | H | PASA | $CO_2CH_3$ | 228 |
| 193 | n-Bu | H | MESA | $CO_2CH_3$ | 120 |
| 194 | n-Bu | H | OCSA | $CO_2$-n-Pent | 259 |

TABLEAU IV (Fin)

| Ex | $R_1$ | $R_2$ | R'' | R''' | F(°C) |
|---|---|---|---|---|---|
| 201 | n-Bu | H | MCSA | $CO_2H$ | 235 |
| 202 | n-Bu | H | PCSA | $CO_2H$ | 215 |
| 203 | n-Bu | H | PhSA | $CO_2H$ | 203 |
| 204 | n-Bu | H | PASA | $CO_2H$ | 193 |
| 205 | n-Bu | H | MESA | $CO_2H$ | 238 |
| 206 | n-Bu | H | $CO_2H$ | $CONH(CH_2)_3N(CH_3)_2$ | 111 |
| 207 | n-Bu | H | $CO_2H$ | CONH-AAE | 92 |
| 208 | n-Bu | H | $CO_2H$ | $CONH-O-CH_3$ | 211 |
| 209* | n-Bu | H | $CO_2H$ | $CONH_2$ | 196 |
| 210 | n-Bu | H | $CO_2H$ | CONH(n-Bu) | 183 |
| 211 | n-Bu | H | OCSA | $CO_2H$ | 198 |
| 215 | n-Bu | H | $CO_2CH_3$ | $CONH(CH_2)_3N(CH_3)_2$ | - |
| 216** | n-Bu | H | $CO_2CH_3$ | $CONH(CH_2)_3N(CH_3)_2$ | 164 |
| 217 | n-Bu | H | TTT | $CO_2$-n-Pent | - |
| 218* | n-Bu | H | TT | $CO_2$-n-Pent | 204 |
| 219 | n-Bu | H | $CO_2CH_3$ | $CONH_2$ | 186 |
| 220 | n-Bu | H | $CO_2CH_3$ | CONH(n-Bu) | 125 |
| 221 | n-Bu | H | $CO_2$-Bn | CO-L-Val-OEt | - |
| 222 | n-Bu | H | $CO_2$-Bn | CO-Gly-OEt | 107 |
| 227 | n-Bu | H | OASA | $CO_2CH_3$ | 150(dec) |
| 228 | n-Bu | H | OASA | $CO_2H$ | 150(dec) |

Note :* HCl** fumarate

TABLEAU V

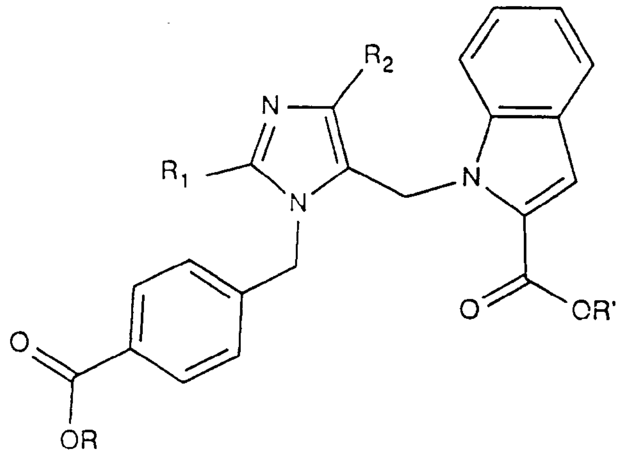

| Prép | $R_1$ | $R_2$ | R | R' | F(°C) |
|---|---|---|---|---|---|
| 11 | n-Bu | Cl | $CH_3$ | $CH_2CH_3$ | 136 |
| 12 | n-Bu | H | $CH_3$ | $CH_2CH_3$ | 86 |
| 33 | n-Bu | H | H | H | 280 |
| 34 | n-Bu | Cl | H | H | 268 |

TABLEAU VI

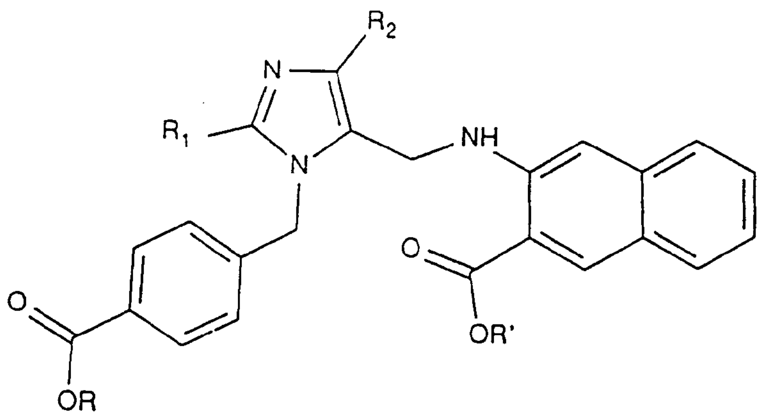

| Prép | $R_1$ | $R_2$ | R | R' | F(°C) |
|---|---|---|---|---|---|
| 16 | n-Bu | Cl | $CH_3$ | $CH_3$ | - |
| 47 | n-Bu | Cl | H | H | 221 |

TABLEAU VII

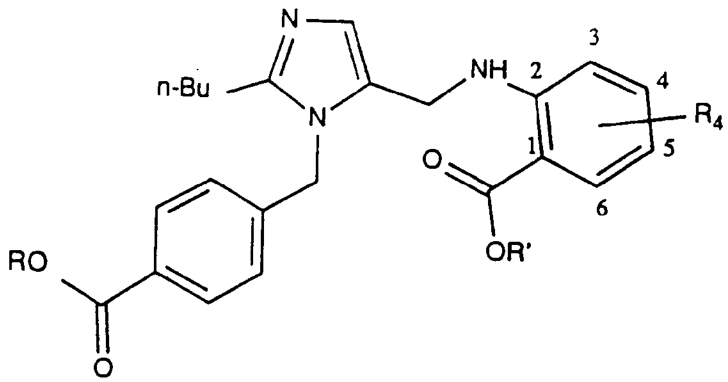

| Ex | R | R' | $R_4$ | F(°C) |
|---|---|---|---|---|
| 72* | Pz | Pz | 4-$NO_2$ | 194 |
| 83 | $CH_3$ | n-Pent | 6-Cl | - |
| 87 | $CH_3$ | n-Pent | 6-$CH_3$ | - |
| 117 | t-Bu | $CH_3$ | 4-$NO_2$ | - |
| 133 | t-Bu | n-Pent | 4-$NO_2$ | - |
| 180 | AAE | AAE | 4-$NO_2$ | 158 |
| 183 | Y-t-Bu | Y-t-Bu | 4-$NO_2$ | 83 |
| 195 | H | $CH_3$ | 4-$NO_2$ | 234 |
| 197 | H | n-Pent | 4-$NO_2$ | 161 |
| 223 | H | n-Pent | 6-$CH_3$ | 153 |

**Note : * 3 HCl**

Les produits selon l'invention sont des inhibiteurs des effets de l'angiotensine II.

L'activité des composés selon l'invention comme antagonistes du récepteur vasculaire de l'angiotensine II a été évaluée par leur efficacité à antagoniser la réponse contractile induite par l'angiotensine II dans des anneaux isolés d'aorte de lapin. Les anneaux sont suspendus dans un bain de Krebs-Henseleit maintenu à 37°C et aéré par un mélange $O_2/CO_2$ (95/5 , v/v) puis étirés à une tension de repos de 2 g. Après une heure de repos, on provoque une contraction par l'angiotensine II ($3.10^{-9}$ M) en présence du produit à tester préincubé pendant 15 minutes. La concentration (exprimée en nanomole) de produit à tester produisant une inhibition de 50 % de la réponse contractile ($IC_{50}$) est calculée à partir de la courbe concentration-réponse . Les résultats obtenus avec un certain nombre de composés selon l'invention sont regroupés dans le tableau VIII.

Les produits selon l'invention sont utiles en thérapeutique dans le traitement ou la prévention de l'hypertension artérielle, du glaucome , des désordres circulatoires, des resténoses consécutives aux angioplasties, des développements de lésions athéromateuses ou fibrinoprolifératives, des néphropathies et rétinopathies d'origine diabétique, de l'infarctus, de l'angor et pour l'amélioration de la fonction cognitive.

Selon l'invention on préconise une composition thérapeutique caractérisée en ce qu'elle renferme au moins un composé de formule I ou l'un de ses sels d'addition en quantité thérapeutiquement efficace en association avec un excipient physiologiquement acceptable.

On préconise également l'utilisation des composés de formule I ou l'un de leurs sels d'addition, en tant qu'agents antagonistes de l'angiotensine II, pour l'obtention d'un médicament préventif ou curatif de l'hypertension artérielle, des désordres circulatoires et du glaucome.

TABLEAU VIII

| Ex | $IC_{50}$ (x$10^{-9}$ M) | Ex | $IC_{50}$ (x$10^{-9}$ M) |
|---|---|---|---|
| 26 | 100 | 156 | 82 |
| 28 | 80 | 157 | 54 |
| 32 | 3,6 | 158 | 75 |
| 33 | 7,1 | 161 | 64 |
| 34 | 8,4 | 163 | 50 |
| 35 | 1,2 | 164 | 40 |
| 36 | 7 | 166 | 15 |
| 39 | 106,2 | 168 | 67 |
| 40 | 57,6 | 170 | 80 |
| 41 | 5,1 | 174 | 80 |
| 42 | 5,1 | 178 | 67 |
| 43 | 6,3 | 180 | 30 |
| 44 | 5,3 | 181 | 80 |
| 45 | 6,7 | 183 | 60 |
| 46 | 10,5 | 187 | 7 |
| 47 | 71,3 | 195 | 82 |
| 48 | 5,3 | 197 | 55 |
| 49 | 13,2 | 198 | 4,6 |
| 50 | 5,7 | 201 | 10 |
| 51 | 10,8 | 202 | 14,7 |
| 52 | 40,2 | 203 | 4,4 |
| 55 | 69,1 | 204 | 9,5 |
| 66 | 8,8 | 205 | 5,5 |
| 67 | 1,5 | 209 | 70 |
| 68 | 30 | 210 | 84 |
| 76 | 90 | 211 | 0,8 |
| 134 | 35 | 212 | 46 |
| 141 | 10 | 226 | 6 |
| 145 | 40 | 228 | 2 |
| 148 | 12 | | |
| 149 | 5 | | |
| 155 | 8,7 | | |

**Revendications**

1. Composé phényl-amino-méthyl-imidazol caractérisé en ce qu'il est choisi parmi l'ensemble constitué par : (i) les phényl-amino-méthyl-imidazoles de formule :

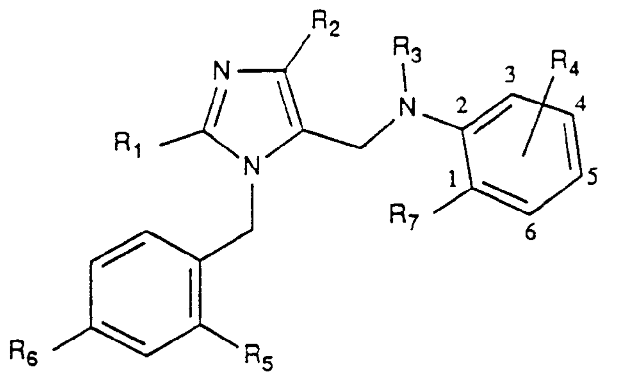

(I)

dans laquelle :

- $R_1$ représente un groupe alkyle en $C_1$-$C_4$,
- $R_2$ représente l'atome d'hydrogène, un halogène, un groupe alkylthio en $C_1$-$C_4$ ou un groupe perfluoroalkyle en $C_1$-$C_3$,
- $R_3$ représente l'atome d'hydrogène, un groupe alkyle en $C_1$-$C_4$ ou un groupe $COR_8$ où $R_8$ représente un groupe alkyle en $C_1$-$C_4$,
- $R_4$ est en position 3, 4, 5 ou 6 et représente l'atome d'hydrogène, un ou plusieurs groupes alkyles en $C_1$-$C_4$, alkoxy en $C_1$-$C_4$, azido, nitro ou un ou plusieurs halogènes, ou forme un groupe amino-2-naphtyle avec le groupe amino-phényle auquel il est lié,
- $R_5$ représente un atome d'hydrogène ou un halogène,
- $R_6$ et $R_7$, identiques ou différents, représentent chacun un groupe tétrazol-5-yle ou un groupe $COR_9$ où $R_9$ représente :
  - un groupe hydroxy,
  - un groupe alkoxy en $C_1$-$C_{16}$,
  - un groupe cyclopropylméthoxy,
  - un groupe phénoxy,
  - un groupe benzyloxy,
  - un groupe 2-phényléthoxy,
  - un groupe glycéryle,
  - un groupe isopropylidène glycéryle,
  - un groupe 2-méthoxyéthoxy,
  - un groupe 2-oxo-butyl oxy,
  - un groupe 1-méthyl-2-oxo-butyl oxy,
  - un groupe 2-(N,N-diéthylamino)-éthoxy,
  - un groupe morpholino-éthoxy,
  - un groupe N-(éthyloxy)nicotinamide,
  - un groupe $-O-CHR_{15}-O(CO)-R_{12}$ dans lequel $R_{15}$ représente l'atome d'hydrogène ou un groupe alkyle en $C_1$-$C_3$ et $R_{12}$ représente un groupe alkyle en $C_1$-$C_7$, un groupe cyclopentyle, un groupe cyclohexyle, un groupe cyclopentylméthyle ou un groupe cyclohexylméthyle,
  - un groupe oxyacétate de formule $-O-CHR_{17}-CO_2-R_{16}$, dans lequel $R_{16}$ et $R_{17}$ représentent chacun indépendamment l'atome d'hydrogène ou un groupe alkyle en $C_1$-$C_5$,
  - un groupe oxyacétamide de formule $-O-CH_2-CO-NR_{10}R_{11}$, dans lequel $R_{10}$ et $R_{11}$, identiques ou différents représentent chacun un groupe alkyle en $C_1$-$C_4$, un groupe hydroxyéthyle ou forment avec l'atome d'azote auquel ils sont liés un groupe 4-méthylpipérazine-1-yle,
  - un groupe amino de formule $-NR_{18}R_{19}$ dans lequel $R_{18}$ et $R_{19}$ représentent chacun, indépendamment, l'atome d'hydrogène, un groupe alkyle en $C_1$-$C_4$, un groupe méthoxy, un groupe 2-(N,N-diméthylamino) propyle ou,$NR_{18}R_{19}$ représente un reste acide aminé de structure glycine ou valine, dont la fonction acide est éventuellement protégée sous forme d'ester ou d'amide,
- $R_6$ pouvant également représenter :

  - un groupe $COR_{13}$ où $R_{13}$ représente un groupe méthylsulfonylamino de formule $-NH-SO_2-CH_3$ ou un groupe arylsulfonylamino de formule :

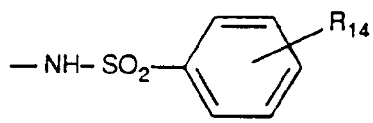

dans laquelle $R_{14}$ représente l'atome d'hydrogène, un halogène, un groupe azido, un groupe alkyle en $C_1$-$C_4$ ou un groupe méthoxy et peut se trouver en position ortho, méta ou para.

- $R_3$ et $R_7$, considérés ensemble,pouvant former, avec l'atome d'azote et le groupe phényle auxquels ils sont respectivement liés, un hétérocycle azoté ortho-condensé carboxy-2-indol-1-yle ou éthoxycarbonyl -2-indol-1-yle; et,

(ii) les sels d'addition avec les acides minéraux et organiques ou avec les bases minérales et organiques des composés de formule I.

2. Composé selon la revendication 1 caractérisé en ce que, dans la formule I, $R_6$ ou $R_7$ représente un groupe COOH.

3. Composé selon la revendication 2 caractérisé en ce que les groupes carboxyliques $R_6$ et $R_7$ sont salifiés par une base organique ou minérale.

4. Composé de formule I selon la revendication 1 caractérisé en ce qu'il est salifié par un acide organique ou minéral.

5. Composé selon la revendication 1 caractérisé en ce que, dans la formule I, $R_6$ ou $R_7$ représente un groupe méthylsulfonylaminocarbonyl ou un groupe arylsulfonylaminocarbonyl.

6. Composition thérapeutique caractérisée en ce qu'elle renferme au moins un composé de formule I ou l'un de ses sels d'addition en quantité thérapeutiquement efficace, en association avec un excipient physiologiquement acceptable.

7. Utilisation d'un composé selon la revendication 1, en tant qu'agent antagoniste de l'angiotensine II, pour l'obtention d'un médicament préventif ou curatif de l'hypertension artérielle, des désordres circulatoires ou du glaucome.

8. Composé intermédiaire, utile dans la synthèse de composés de formule I selon la revendication 1, caractérisé en ce qu'il s'agit d'un produit 1-phénylméthyl-imidazol-5-carboxaldéhyde de formule :

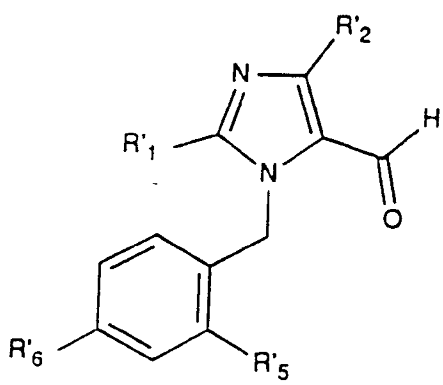

dans laquelle :

(i) $R'_1$ représente un groupe n-propyle, $R'_2$ représente un atome d'hydrogène ou un halogène, $R'_5$ représente l'atome d'hydrogène et $R'_6$ représente un groupe cyano ou un groupe $COR'_9$ où $R'_9$ représente un groupe alkoxy en $C_1$-$C_{16}$ ou un groupe benzyloxy, ou

(ii) $R'_1$ représente un groupe n-butyle, $R'_2$ et $R'_5$ représentent l'atome d'hydrogène et $R'_6$ représente un groupe $COR'_9$ dans lequel $R'_9$ représente un groupe t-butoxy ou benzyloxy.

9. Composé intermédiaire, utile dans la synthèse de composé de formule I selon la revendication 1, caractérisé en ce qu'il s'agit d'un produit 1-phénylméthyl-5-hydroxyméthyl-imidazole de formule :

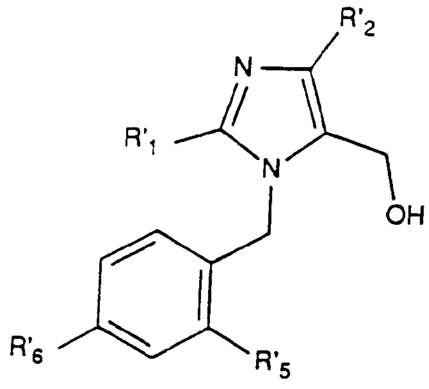

dans laquelle :
R'$_1$ représente un groupe alkyle en $C_1$-$C_4$, R'$_2$ représente l'atome d'hydrogène ou un halogène, R'$_5$ représente l'atome d'hydrogène et R'$_6$ représente un groupe COR'$_9$ où R'$_9$ représente un groupe alkoxy en $C_1$-$C_{16}$ ou un groupe benzyloxy.

**10.** Composé intermédiaire utile dans la synthèse de composé de formule I selon la revendication 1, caractérisé en ce qu'il s'agit d'un produit 1-phénylméthyl-5-halogénométhyl-imidazole de formule :

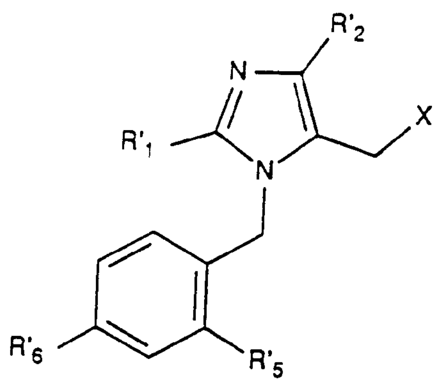

dans laquelle
R'$_1$ représente un groupe n-butyle, R'$_2$ et R'$_5$ représentent l'atome d'hydrogène, R'$_6$ représente un groupe COR'$_9$ dans lequel R'$_9$ représente un groupe t-butoxy ou benzyloxy et X représente un halogène.

**11.** Procédé de préparation d'un composé selon la revendication 1 caractérisé en ce qu'il comprend les étapes consistant à :
(a)soumettre à une substitution nucléophile un composé de formule :

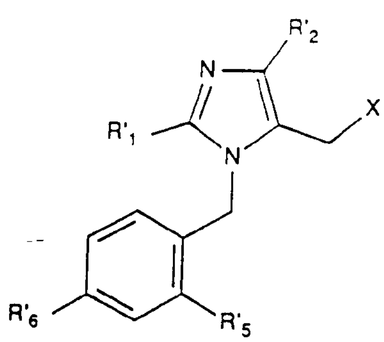

(II')

dans laquelle :

- $R'_1$ représente un groupe alkyle en $C_1$-$C_4$,
- $R'_2$ représente l'atome d'hydrogène, un halogène, un groupe alkylthio en $C_1$-$C_4$ ou un groupe perfluoroalkyle en $C_1$-$C_3$,
- $R'_5$ représente un atome d'hydrogène ou un halogène,
- $R'_6$ représente un groupe cyano, un groupe $COR'_9$ où $R'_9$ représente un groupe alkoxy en $C_1$-$C_{16}$, un groupe benzyloxy ou un groupe isopropylidène glycéryle,
- X représente un halogène notamment l'atome de chlore, ou un groupe paratoluènesulfonyle,

par réaction avec un composé de formule :

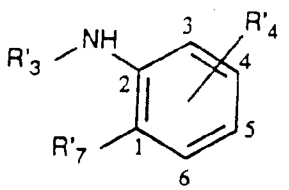

(III')

dans laquelle :

- $R'_3$ représente l'atome d'hydrogène ou un groupe alkyle en $C_1$-$C_4$,
- $R'_4$ est en position 3, 4, 5 ou 6 et représente l'atome d'hydrogène, un ou plusieurs groupes alkyles en $C_1$-$C_4$, alkoxy en $C_1$-$C_4$, azido, nitro ou un ou plusieurs halogènes ou forme un groupe amino-2 naphtyle avec le groupe amino-phényle auquel il est lié,
- $R'_7$ représente un groupe cyano ou un groupe $COR'_9$ où $R'_9$ représente:
  - un groupe alkoxy en $C_1$-$C_{16}$, un groupe benzyloxy, un groupe isopropylidène glycéryle, un groupe phénoxy, un groupe 2-phényléthoxy, un groupe 2-méthoxyéthoxy, un groupe 2-oxobutyl oxy,un groupe 1-méthyl-2-oxo-butyl oxy, un groupe 2-(N,N-diéthylamino)-éthoxy, ou
  - un groupe $-O-CHR_{15}-O(CO)-R_{12}$ dans lequel $R_{15}$ représente l'atome d'hydrogène ou un groupe alkyle en $C_1$-$C_3$ et $R_{12}$ représente un groupe alkyle en $C_1$-$C_7$, un groupe cyclopentyle, un groupe cyclohexyle, un groupe cyclopentylméthyle ou un groupe cyclohexylméthyle,
  - un groupe oxyacétate de formule $-O-CHR_{17}-CO_2-R_{16}$, dans lequel $R_{16}$ et $R_{17}$ représentent chacun indépendamment l'atome d'hydrogène ou un groupe en $C_1$-$C_5$,
  - un groupe oxyacétamide de formule $-O-CH_2-CO-NR_{10}R_{11}$, dans lequel $R_{10}$ et $R_{11}$, identiques ou différents représentent chacun un groupe alkyle en $C_1$-$C_4$ ou un groupe hydroxyéthyle,
  - un groupe amino de formule $-NR_{18}R_{19}$ dans lequel $R_{18}$et $R_{19}$ représentent chacun indépendamment l'atome d'hydrogène, un groupe alkyle en $C_1$-$C_4$, un groupe méthoxy, un groupe 2-(N,N-diméthylamino) propyle ou $NR_{18}R_{19}$ représente un reste acide aminé de structure glycine ou valine, dont la fonction acide est éventuellement protégée sous forme d'ester où d'amide,
- $R'_3$ et $R'_7$, considérés ensemble,pouvant former, avec l'atome d'azote et le groupe phényle auxquels ils sont respectivement liés, un hétérocycle azoté ortho-condensé carboxy-2-indol-1-yle ou éthoxycarbonyle-2-indol-1 -yle,

en milieu anhydre en présence ou non d'un solvant polaire ou non polaire et aprotique, comme par exemple le toluène, les xylènes, le tétrahydroturanne, le diméthylformamide, les hydrocarbures chlorés, les éthers, le dioxanne, la N-méthylpyrrolidin-2-one, la N,N'-diméthylpropylèneurée ou le diméthylsulfoxyde et en présence ou non d'une base forte comme par exemple la triéthylamine, la 2,6-lutidine, les hydrures de sodium ou potassium, l'hexaméthyldisilylamidure de potassium ou de lithium ou le diisopropylamidure de lithium, à raison de 1 mole de composé II' pour 1 à 20 moles de composé III', à une température comprise entre la température ambiante (15-25°C) et environ 200°C, pendant 0,1 à 12 heures, pour obtenir un composé de formule :

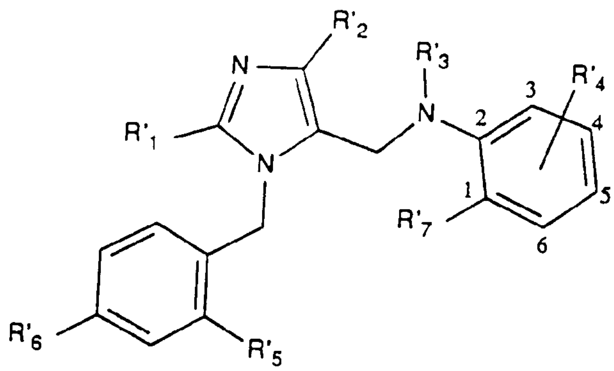

(I')

dans laquelle R'$_1$, R'$_2$, R'$_3$, R'$_4$, R'$_5$, R'$_6$ et R'$_7$ ont les significations indiquées ci-dessus;

(b) si nécessaire, soumettre le composé de formule I' ainsi obtenu aux traitements suivants :

(i) saponification d'un composé de formule I' dans laquelle l'un au moins des groupes R'$_6$ et R'$_7$ représente un groupe COR'$_9$ où R'$_9$ représente un groupe alkoxy en $C_1$-$C_{16}$, selon les méthodes connues de l'homme de l'art, notamment en présence d'une base forte comme par exemple une solution aqueuse à hydroxyde de sodium ou de potassium, dans le diméthoxyéthane ou un alcool comme par exemple le méthanol, pour obtenir un composé de formule I dans laquelle R$_6$ et R$_7$ représentent un groupe COOH ou R$_6$, représente un groupe COOH et R$_7$ représente un groupe COR$_9$ où R$_9$ représente un groupe alkoxy en $C_1$-$C_{16}$;

(ii)estérification du composé ainsi obtenu au stade (i) selon les méthodes connues de l'homme de l'art, notamment par réaction avec un alcool approprié ou par réaction avec un dérivé halogéné approprié, pour obtenir un composé de formule I dans laquelle R$_6$ et R$_7$ représentent un groupe COR$_9$ où R$_9$ a les significations des groupes R'$_9$ indiqués ci-dessus;

(iii) acylation du méthylsulfonamide ou d'un arylsulfonamide de formule :

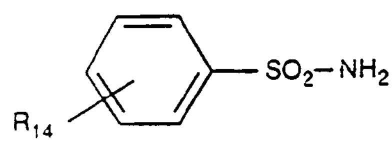

dans laquelle R$_{14}$ représente l'atome d'hydrogène, un halogène, un groupe azido, un groupe alkyle en $C_1$-$C_4$ ou un groupe méthoxy, par un mono-acide obtenu au stade (i) selon les méthodes connues de l'homme de l'art, notamment en présence d'un réactif de couplage comme par exemple le chlorhydrate de 1-(3-diméthylaminopropyl)-3-éthylcarbodiimide ou la N,N-dicyclohexylcarbodiimide, pour obtenir un composé de formule I dans laquelle R$_6$ représente un groupe COR$_{13}$ où R$_{13}$ représente un groupe méthylsulfonylamino de formule -NH-$SO_2$- $CH_3$, ou un groupe arylsulfonylamino de formule :

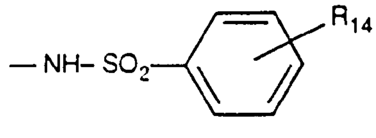

dans laquelle R$_{14}$ a la signification indiquée ci-dessus, R$_7$ représente un groupe COR$_9$ où R$_9$ représente un groupe alkoxy en $C_1$-$C_{16}$ et R$_1$, R$_2$, R$_3$, R$_4$, R$_6$ ont les significations indiquées ci-dessus pour respectivement R'$_1$, R'$_2$, R'$_3$, R'$_4$ et R'$_5$;

(iv) acylation d'un composé de formule I' dans laquelle R'$_3$ représente l'atome d'hydrogène et R'$_1$, R'$_2$, R'$_4$, R'$_5$ R'$_6$ et R'$_7$ ont les significations indiquées ci-dessus, selon les méthodes connues de l'homme de l'art, notamment par réaction avec un anhydrique d'acide, comme par exemple l'anhydrique acétique, pour obtenir un composé de formule I dans laquelle R$_3$ représente un groupe COR$_6$ où R$_8$ représente un groupe alkyle en $C_1$-$C_4$, et R$_1$, R$_2$, R$_4$, R$_5$, R$_6$ et R$_7$ ont les significations indiquées

ci-dessus pour respectivement $R'_1$, $R'_2$, $R'_4$, $R'_5$, $R'_6$ et $R'_7$;

(v) si nécessaire, déprotection d'un composé de formule I' dans laquelle au moins l'un des groupes $R'_6$ et $R'_7$ représente un groupe $COR'_9$ où $R'_9$ représente un groupe alkoxy en $C_1$-$C_4$, un groupe benzyloxy ou un groupe isopropylidène glycéryle, selon les méthodes connues de l'homme de l'art, notamment par traitement en milieu acide ou par hydrogénation catalytique, pour obtenir un composé de formule I dans laquelle l'un au moins des groupes $R_6$ ou $R_7$ représente un groupe COOH ou CO-glycéryle et l'autre groupe représente un groupe $COR_9$ où $R_9$ a la signification indiquée ci-dessus pour $R'_9$;

(vi) conversion d'un composé de formule I' dans laquelle $R'_6$ ou $R'_7$ représente un groupe cyano, en composé de formule I dans laquelle $R_6$ ou $R_7$ représente un groupe tétrazol-5-yle selon les méthodes connues de l'hommme de l'art, notamment par cycloaddition 1,3-dipolaire d'azotures de trialkylétain ou triarylétain.

Office européen des brevets

# RAPPORT DE RECHERCHE EUROPEENNE

Numero de la demande

EP 93 40 0833
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | WO-A-9 202 510 (SMITHKLINE BEECHAM CORP.) 20 Février 1992<br>RN : 141771-40-6 : Benzoic acid, 4-[[2-butyl-5-(chloromethyl)-1H-imidazol-1-yl]methyl]-, methyl ester, monohydrochloride<br>RN : 133040-03-6 : Benzoic acid, 4-[(2-butyl-5-formyl-1H-imidazol-1-yl)methyl]- , methyl ester<br>RN : 133040-02-5 : Benzoic acid, 4-[(2-butyl-4-chloro-5-formyl-1H-imidazol-1-yl)methyl]-, methyl ester<br>--- | 8,10 | C07D233/54<br>C07D233/64<br>C07D233/68<br>C07D233/84<br>C07D401/12<br>C07D401/14<br>C07D403/06<br>C07D403/10<br>C07D405/12<br>C07D405/14<br>A61K31/415 |
| X | JOURNAL OF MEDICINAL CHEMISTRY.<br>vol. 35, no. 5, 6 Mars 1992, WASHINGTON US pages 877 - 885<br>Thomas A P; Allott C P; Gibson K H; Major J S; Masek B B; Oldham A A; Ratcliffe A H; Roberts D A; Russell S T; Thomason D A 'New nonpeptide angiotensin II receptor antagonists. 1. Synthesis, biological properties and structure-activity relationships of 2-alkylbenzimidazole derivatives'<br>RN : 138459-26-4 : Benzoic acid, 4-[[2-butyl-4-chloro-5-(hydroxymethyl)-1H-imidazol-1-yl]methyl]-3-fluoro-, methyl ester<br>--- | 9 | **DOMAINES TECHNIQUES RECHERCHES (Int. Cl.5)**<br>C07D |
| D,X | EP-A-0 403 159 (SMITHKLINE BEECHAM CORP.) 19 Décembre 1990<br>RN : 133486-58-5 : Benzonitrile, 4-[(2-butyl-5-formyl-1H-imidazol-1-yl)methyl]-<br>RN : 133486-34-7 : Benzoic acid, 4-[(2-butyl-5-formyl-1H-imidazol-1-yl)methyl]-3-chloro-, ethyl ester<br>RN : 133040-03-6 : Benzoic acid, 4-[(2-butyl-5-formyl-1H-imidazol-1-yl)methyl]- , methyl ester<br>---<br>-/-- | 8 | |

Le présent rapport a été établi pour toutes les revendications

| Lieu de la recherche | Date d'achèvement de la recherche | Examinateur |
|---|---|---|
| LA HAYE | 21 JUIN 1993 | Bernd Kissler |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 93 40 0833
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | EP-A-0 403 158 (SMITHKLINE BEECHAM CORP.) 19 Décembre 1990<br>RN : 133486-34-7 : Benzoic acid, 4-[(2-butyl-5-formyl-1H-imidazol-1-yl)methyl]-3-chloro-, ethyl ester<br>--- | 8 | |
| X | EP-A-0 425 211 (SMITHKLINE BEECHAM CORP.) 2 Mai 1991<br>RN : 133040-03-6 : Benzoic acid, 4-[(2-butyl-5-formyl-1H-imidazol-1-yl)methyl]- , methyl ester<br>--- | 8 | |
| X | JOURNAL OF MEDICINAL CHEMISTRY.<br>vol. 34, no. 4, 1991, WASHINGTON US<br>pages 1514 - 1517<br>Weinstock J; Keenan R M; Samanen J; Hempel J; Finkelstein J A; Franz R G; Gaitanopoulos D E; Girard G R; Gleason J G; et al.<br>'1-(Carboxybenzyl)imidazole-5-acrylic acids: potent and selective angiotensin II receptor antagonists'<br>RN : 133040-03-6 : Benzoic acid, 4-[(2-butyl-5-formyl-1H-imidazol-1-yl)methyl]- , methyl ester<br>RN : 133040-02-5 : Benzoic acid, 4-[(2-butyl-4-chloro-5-formyl-1H-imidazol-1-yl)methyl]-, methyl ester<br>---<br>-/-- | 8 | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 21 JUIN 1993 | Bernd Kissler |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 93 40 0833
Page 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | JOURNAL OF MEDICINAL CHEMISTRY. vol. 33, no. 5, 1990, WASHINGTON US pages 1312 - 1329 Duncia J V; Chiu A T; Carini D J; Gregory G B; Johnson A L; Price W A; Wells G J; Wong P C; Calabrese J C; Timmermans P B M W M 'The discovery of potent nonpeptide angiotensin II receptor antagonists: a new class of potent antihypertensives' RN : 114799-90-5 : Benzonitrile, 4-[[2-butyl-4-chloro-5-(hydroxymethyl)-1H-imidazol-1-yl]methyl]- RN : 114798-52-6 : Benzoic acid, 4-[[2-butyl-4-chloro-5-(hydroxymethyl)-1H-imidazol-1-yl]methyl]- RN : 114771-97-0 : Benzonitrile, 4-[[2-butyl-4-chloro-5-(chloromethyl)-1H-imidazol-1-yl]methyl]- --- | 9 | |
| X | JOURNAL OF MEDICINAL CHEMISTRY. vol. 33, no. 5, 1990, WASHINGTON US pages 1330 - 1336 Carini D J; Duncia J V; Johnson A L; Chiu A T; Price W A; Wong P C; Timmermans P B M W 'Part VI. Nonpeptide angiotensin II receptor antagonists: N-[(benzyloxy)benzyl]imidazoles and related compounds as potent antihypertensives' RN : 114799-90-5 : Benzonitrile, 4-[[2-butyl-4-chloro-5-(hydroxymethyl)-1H-imidazol-1-yl]methyl]- --- -/-- | 9 | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 21 JUIN 1993 | Bernd Kissler |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0402)

Office européen des brevets

# RAPPORT DE RECHERCHE EUROPEENNE

Numero de la demande

EP 93 40 0833
Page 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 324 377 (DU PONT DE NEMOURS) 19 Juillet 1989<br>RN : 114799-90-5 : Benzonitrile, 4-[[2-butyl-4-chloro-5-(hydroxymethyl)-1H-imidazol-1-yl]methyl]-<br>RN : 114773-53-4 : Benzoic acid, 4-[[2-butyl-4-chloro-5-(hydroxymethyl)-1H-imidazol-1-yl]methyl]-, monosodium salt<br>RN : 114798-52-6 : Benzoic acid, 4-[[2-butyl-4-chloro-5-(hydroxymethyl)-1H-imidazol-1-yl]methyl]-<br>--- | 9 | |
| X | EP-A-0 253 310 (DU PONT DE NEMOURS) 20 Janvier 1988<br>RN : 114799-90-5 : Benzonitrile, 4-[[2-butyl-4-chloro-5-(hydroxymethyl)-1H-imidazol-1-yl]methyl]-<br>RN : 114798-52-6 : Benzoic acid, 4-[[2-butyl-4-chloro-5-(hydroxymethyl)-1H-imidazol-1-yl]methyl]-<br>RN : 114773-53-4 : Benzoic acid, 4-[[2-butyl-4-chloro-5-(hydroxymethyl)-1H-imidazol-1-yl]methyl]-, monosodium salt<br>RN : 114771-97-0 : Benzonitrile, 4-[[2-butyl-4-chloro-5-(chloromethyl)-1H-imidazol-1-yl]methyl]-<br>--- | 9,10 | DOMAINES TECHNIQUES RECHERCHES (Int. Cl.5) |
| D,A | EP-A-0 427 463 (SMITHKLINE BEECHAM CORP.) 15 Mai 1991<br>* le document en entier *<br>--- | 1-11 | |
| A | EP-A-0 081 324 (FARMOS GROUP LTD.) 15 Juin 1983<br>* page 3, ligne 18 - ligne 22 *<br>* revendication 1 *<br>----- | 1-11 | |

Le présent rapport a été établi pour toutes les revendications

| Lieu de la recherche | Date d'achèvement de la recherche | Examinateur |
|---|---|---|
| LA HAYE | 21 JUIN 1993 | Bernd Kissler |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)